# EUROPEAN PATENT SPECIFICATION

(11) **EP 0 823 906 B1**
(45) Date of publication and mention of the grant of the patent: **09.07.2003**
(21) Application number: 96915342.8
(22) Date of filing: 01.05.1996
(51) Int. Cl.: C07D 401/12, C07D 405/14, C07D 409/14, C07D 401/14, C07D 453/02, C07D 487/04, A61K 31/445

(54) **PIPERAZINO DERIVATIVES AS NEUROKININ ANTAGONISTS**
PIPERAZINO-DERIVATIVE ALS NEUROKININANTAGONISTEN
DERIVES PIPERAZINO EN TANT QU'ANTAGONISTES DES NEUROKININES

(30) Priority: 02.05.1995 US 432739; 31.08.1995 US 3084 P
(43) Date of publication of application: 18.02.1998
(73) Proprietor: SCHERING CORPORATION, Kenilworth New Jersey 07033 (US)
(72) Inventor: SHUE, Ho-Jane, Pine Brook, NJ 07058 (US); SHIH, Neng-Yang, North Caldwell, NJ 07006 (US); BLYTHIN, David, J., North Caldwell, NJ 07006 (US); CHEN, Xiao, Edison, NJ 08820 (US); TOM, Wing, C., Cedar Grove, NJ 07009 (US); PIWINSKI, John, J., Lebanon, NJ 08833 (US); MCCORMICK, Kevin, D., Edison, NJ (US)
(74) Representative: Ritter, Stephen David
(86) International application number: US9605660
(87) International publication number: WO96034864

(56) References cited:
- EP-A- 0 655 442
- WO-A-92/20661
- WO-A-94/13646
- WO-A-96/10568
- GB-A- 2 230 262

## Description

### BACKGROUND OF THE INVENTION

The present invention relates to a genus of compounds useful as antagonists of neurokinin receptors. In particular, these can be neurokinin-1 receptor (NK₁) antagonists. Some can also be neurokinin-1 receptor (NK₁)antagonists and neurokinin-2 receptor (NK₂) antagonists, that is, NK₁ / NK₂ dual receptor antagonists. Some can also be neurokinin-2 receptor (NK₂) antagonists. Some can also be neurokinin-3 receptor (NK₃) antagonists.

Neurokinin receptors are found in the nervous system and the circulatory system and peripheral tissues of mammals, and therefore are involved in a variety of biological processes. Neurokinin receptor antagonists are consequently expected to be useful in the treatment or prevention of various mammalian disease states, for example asthma, cough, bronchospasm, inflammatory diseases such as arthritis, migraine, nociception, CNS diseases such as anxiety, and various gastrointestinal disorders such as Crohn's disease.

In particular, NK₁ receptors have been reported to be involved in microvascular leakage and mucus secretion, and NK₂ receptors have been associated with smooth muscle contraction, making NK₁ and NK₂ receptor antagonists especially useful in the treatment and prevention of asthma.

GB 2,230,262 discloses 3-substituted N,N-di(trimethoxybenzoyl)piperazines which are said to be useful as PAF receptor antagonists. This publication makes no reference to neurokinin antagonist activity.

WO 92/20661 and WO 94/13646 disclose N,N-diacylpiperazines as *inter alia* neurokinin antagonists. The compounds disclosed in these publications can contain *inter alia* a nitrogen-containing moiety adjacent to the carbonyl groups of the diacylpiperazine moiety, wherein the nitrogen atom must be directly attached to the carbonyl groups.

The invention relates to compounds of the formula: 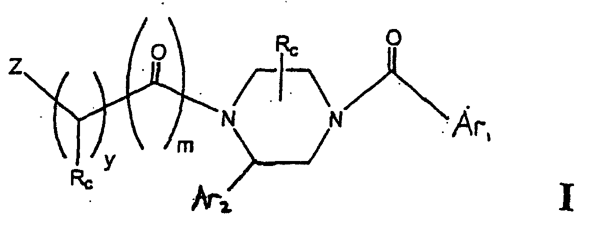
n is 0 to 2,
m is 1, and y is 1 to 3; or m is 2, and y is 0;
   and with the further proviso that no more than one R_{c} is other than H in the 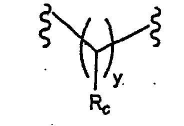 moiety;
   each R_{c} is independently H, C₁-C₆ alkyl, -(CH₂)ₙ₁-R4 where n₁ is 1 to 6; R₄ is -ORₐ, 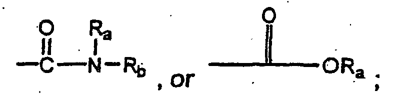
each Rₐ and R_{b} is independently selected from the group consisting of H, C₁-C₆ alkyl, phenyl, phenyl substituted by R¹, R², and/or R³; benzyl, benzyl substituted by R¹, R², and/or R³; allyl; or when Rₐ and R_{b}
   are attached to the same nitrogen, then Rₐ and R_{b} together with the nitrogen to which they are attached, form a 4 to 7 member ring;
wherein each R₁ and R₂ is independently H, C₁-C₆ alkyl, CF₃, C₂F₅, Cl, Br, I, F, NO₂, ORₐ, CN, NRₐR_{b}, 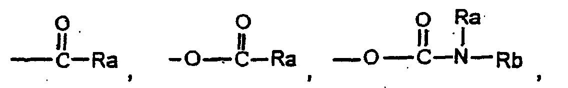 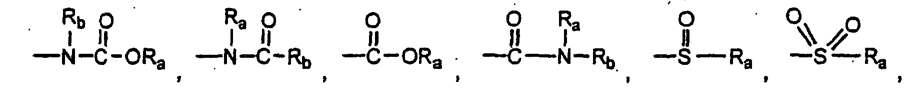 -SRₐ, and 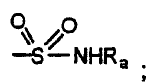 and where Rₐ is not H in 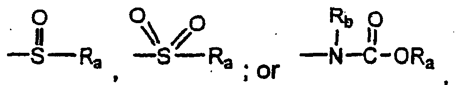 or when R₁ and R₂ are on adjacent carbons on a ring, they can form 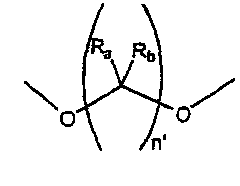 wherein n' is 1 or 2; and each R₃ is independently H, C₁-C₆ alkyl, CF₃, C₂F₅, 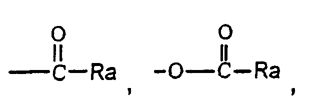 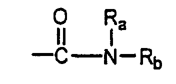 Cl, Br, I, or F, ORₐ, OCF₃, or phenyl;
Ar₁ is R¹,R²,R3-substituted heteroaryl, wherein heteroaryl is selected from pyridyl, benzothienyl, benzofuranyl, benzothiazolyl, indolyl or quinolyl, 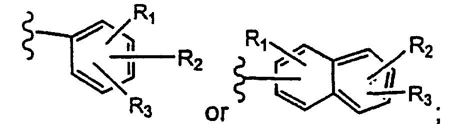
Ar₂ is 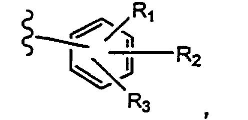
Z is 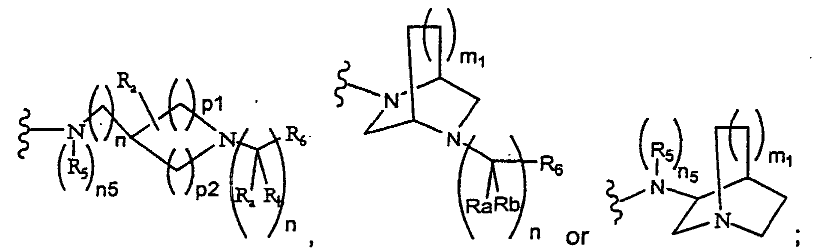
each R₅ is independently selected from the group consisting of H, 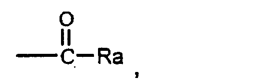 C₁-C₆ alkyl; with the proviso that when R₅ is C₁-C₆ alkyl, two R₅ can be attached to the nitrogen to form a quaternary salt; n₅ is 1, or n₅ is 2 with the proviso that each R₅ is independently C₁-C₆ alkyl; and m is 0 or 1;
p₁ and p₂ are each independently 1 to 4 with the proviso that p₁ and p₂ added together are 2 to 6;
R₆ is thienyl, furanyl, pyridyl, tetrazolyl, imidazolyl, 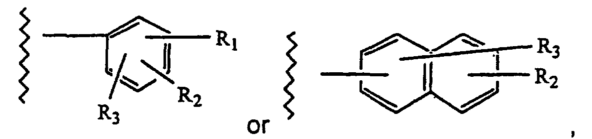
or a pharmaceutically acceptable salt thereof.

All of the variables in the above formulas such as Z, R₁, R₂, and R₃, have the same meaning throughout the specification unless otherwise specified.

Also preferred are compounds of formula I wherein m is 1, and y is 1.

Also preferred are compounds of formula I wherein Z is

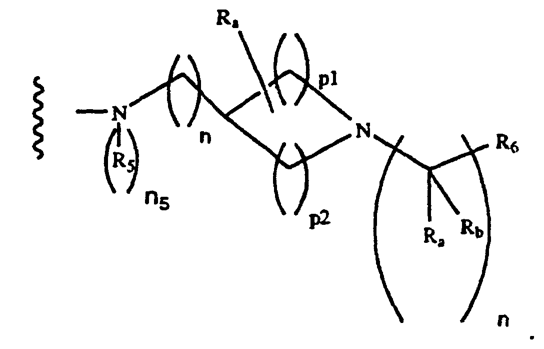

Also preferred are compounds of formula I wherein Z is

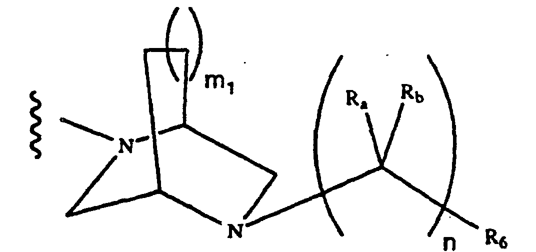

Also preferred are compounds of formula I wherein Z is

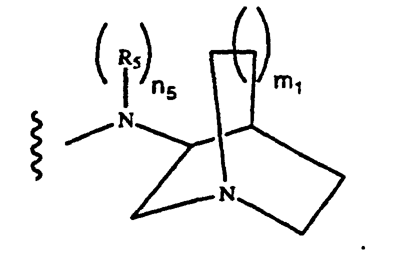

Also preferred are compounds of formula I wherein Z is

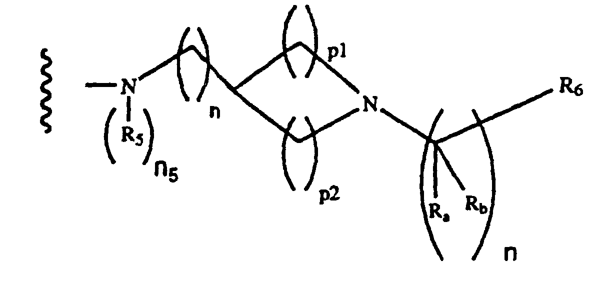

Also preferred are compounds of formula I wherein Z is

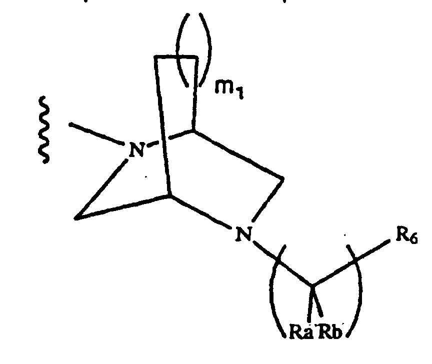

Also preferred are compounds of formula I wherein Z is

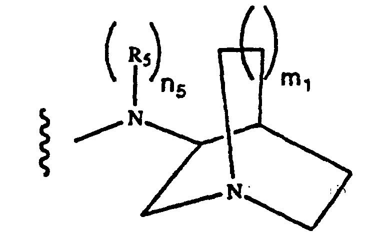

Also preferred are compounds of formula I
wherein R₆ is

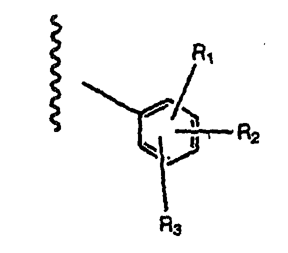

Also preferred are compounds of formula I wherein

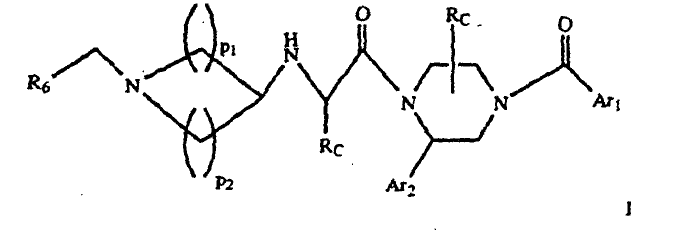

Also preferred are compounds of formula I'
where in R_{c} is H, p₁ and p₂ are 2, Ar₁ and Ar₂ are both

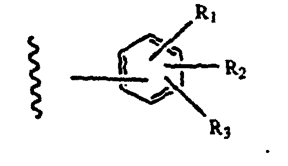

Also preferred are compounds of formula l' wherein R₆ is

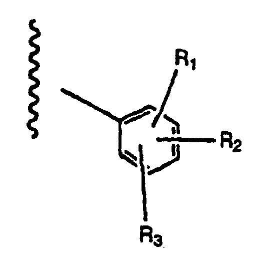

Also preferred are compounds of formula I wherein
Z is 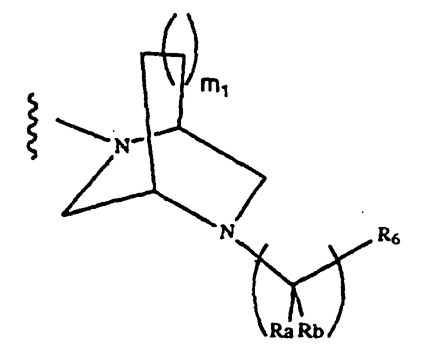 and R₆ is

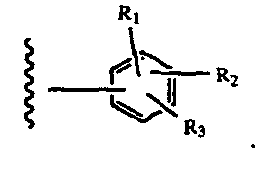

Exemplary compounds of the invention are compounds of the formulas: 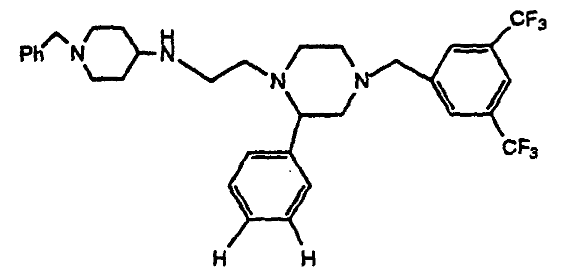 and 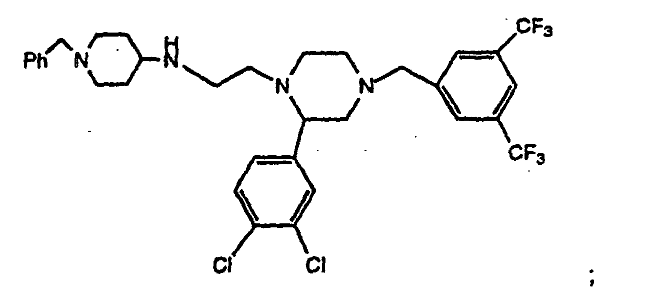 or a compound selected from the group consisting of 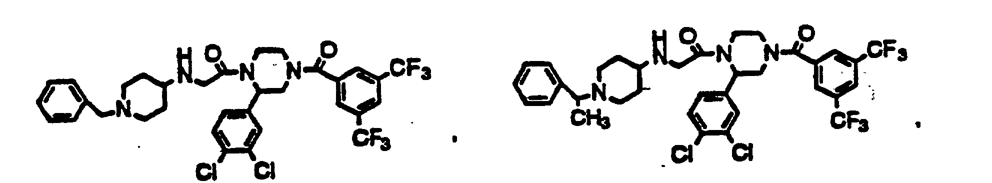 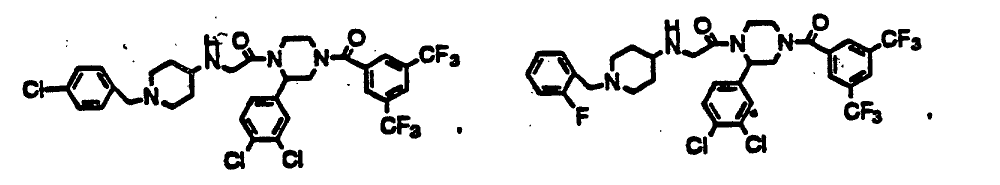 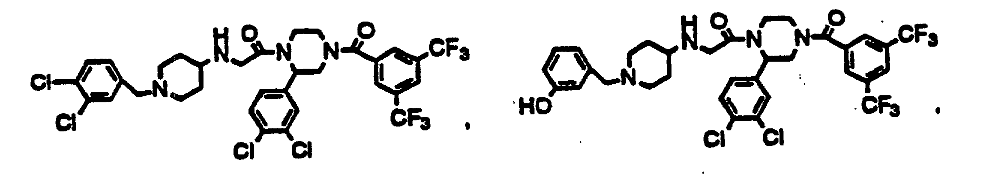 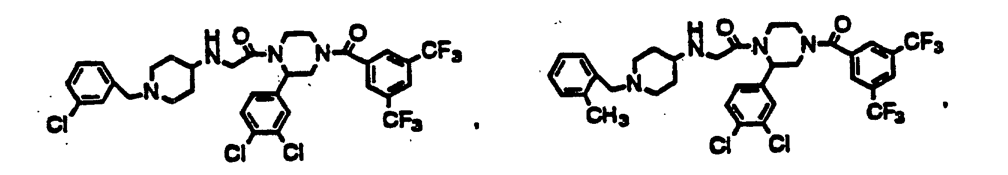 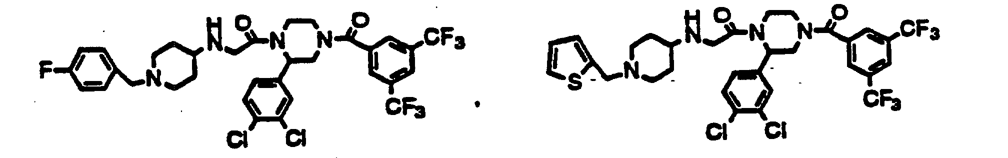 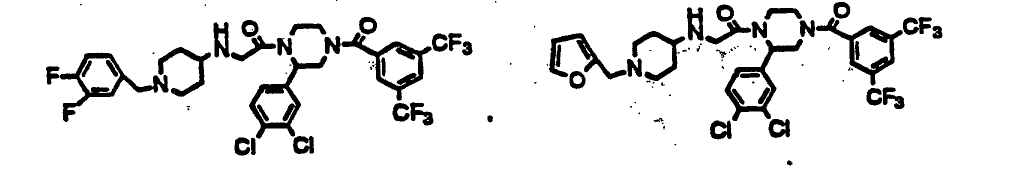 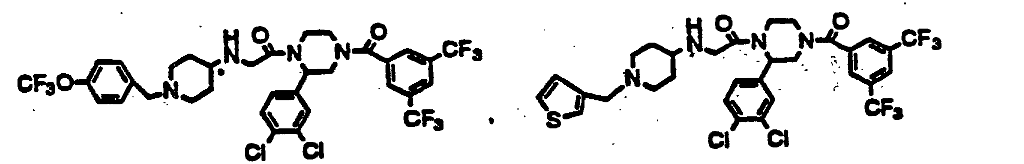 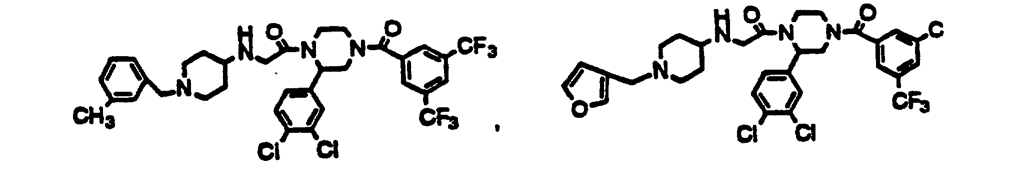 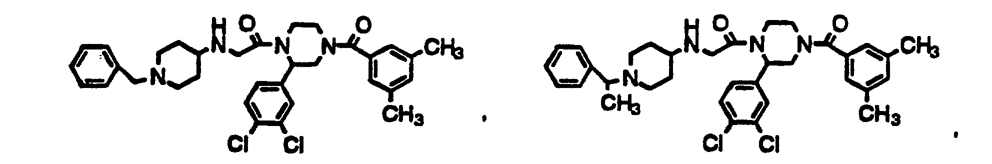 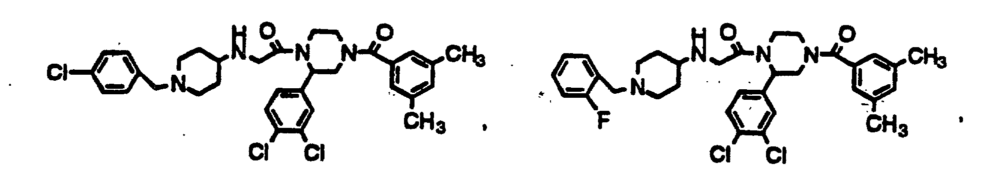 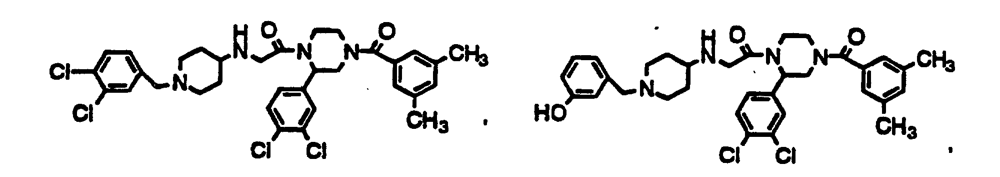 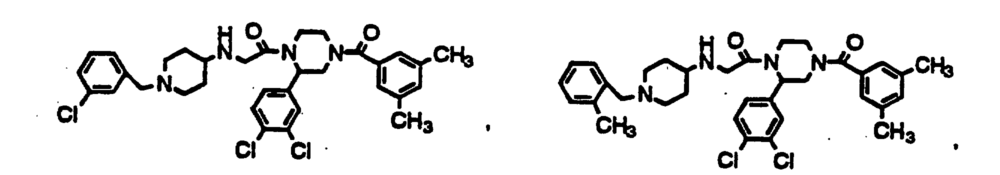 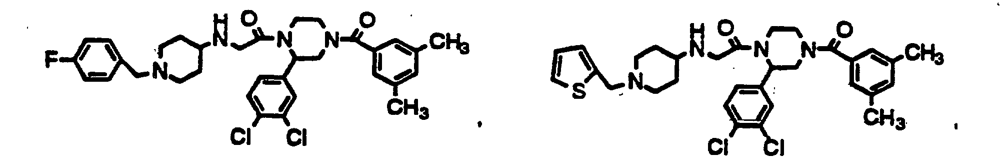 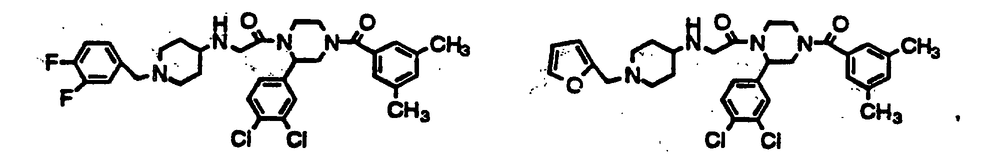 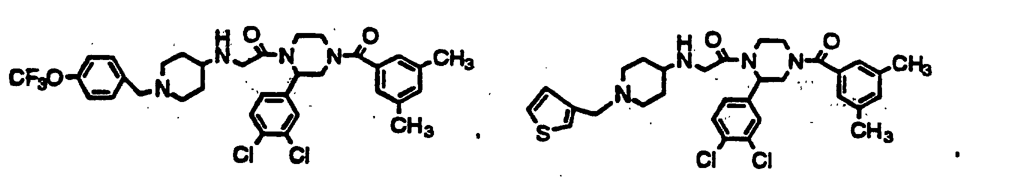 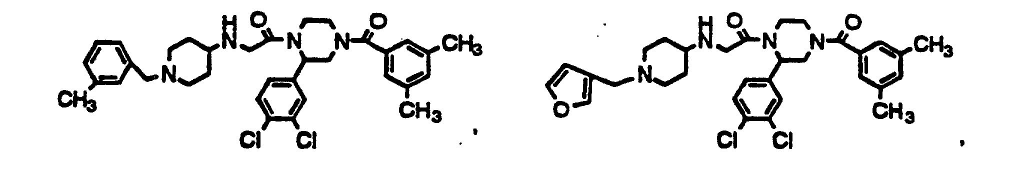 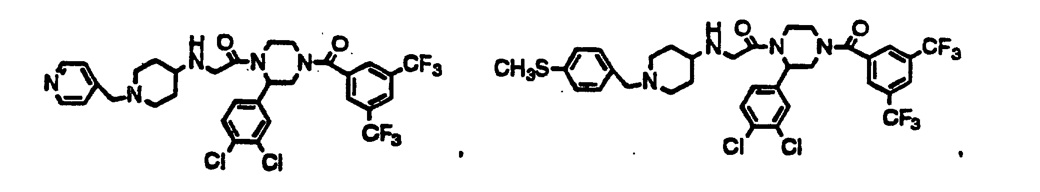 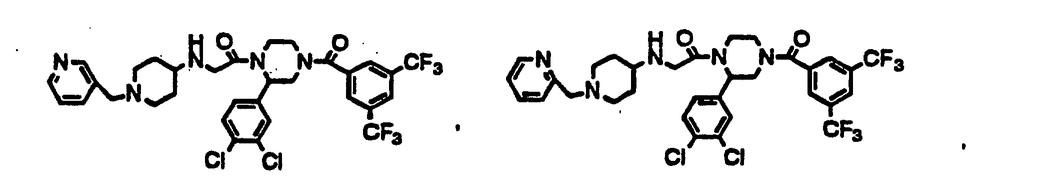 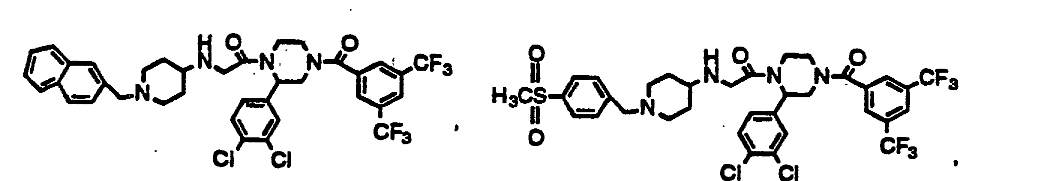 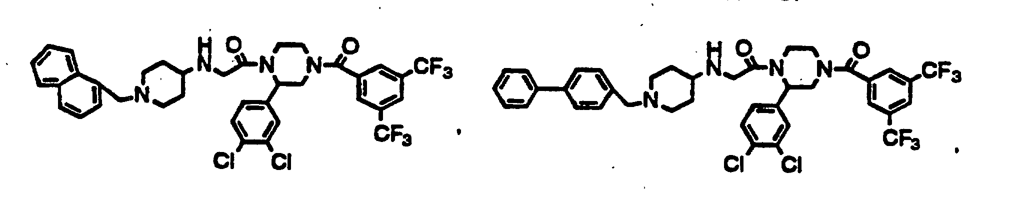 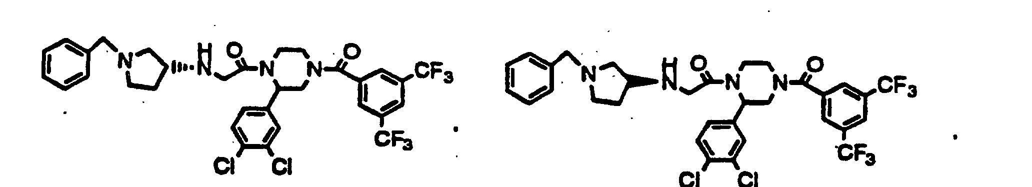 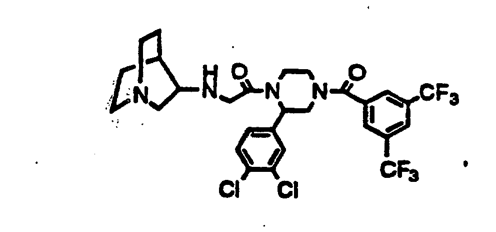 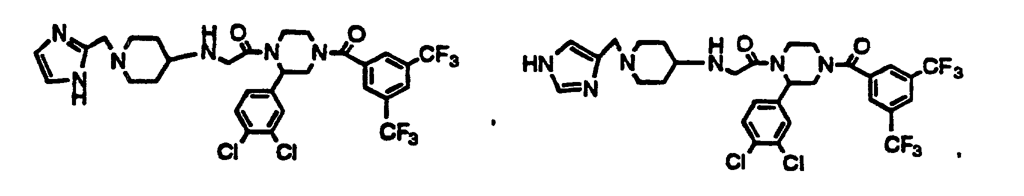 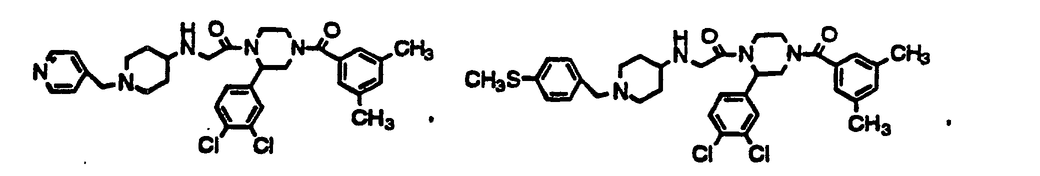 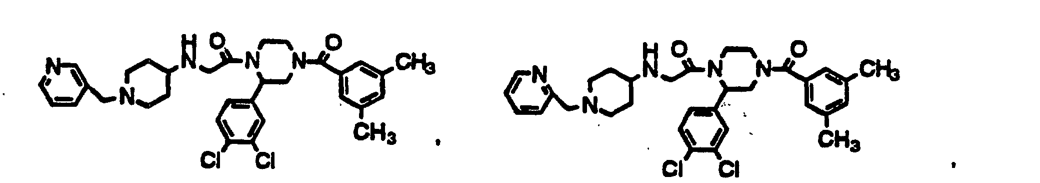 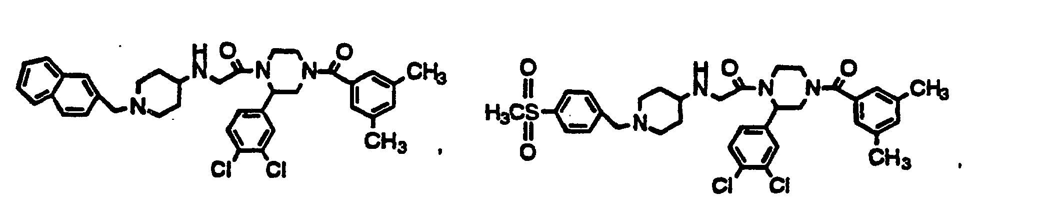 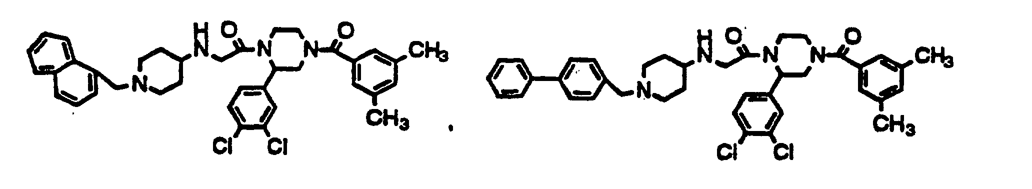 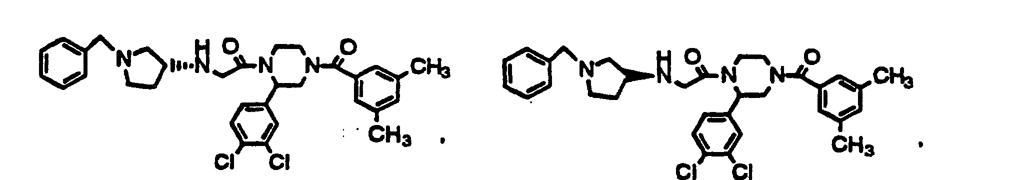 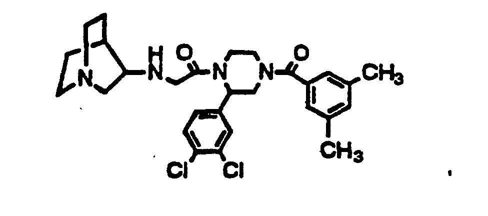 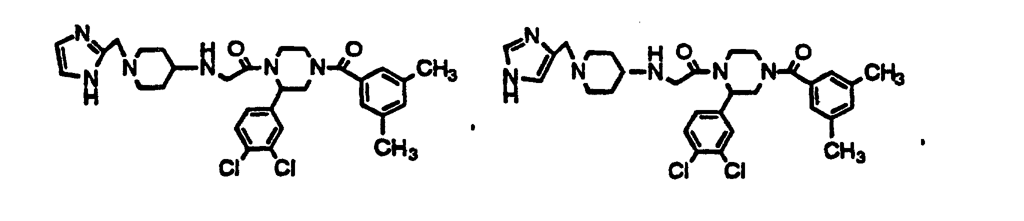 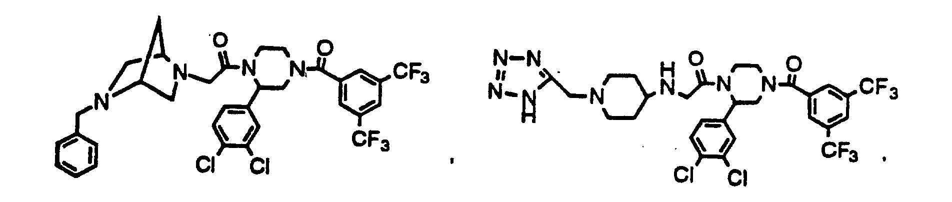 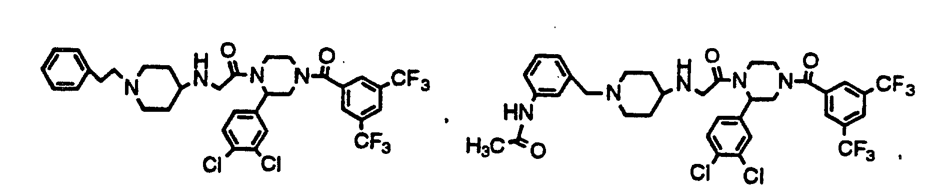 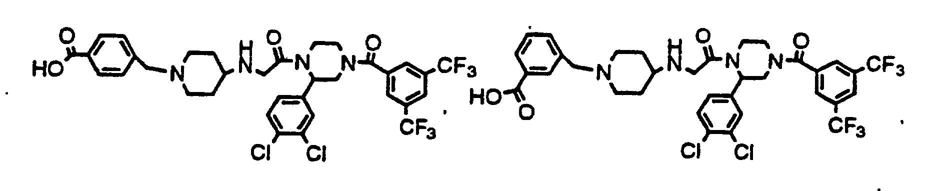 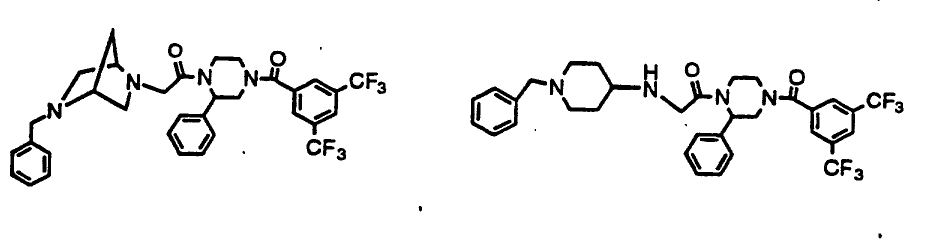 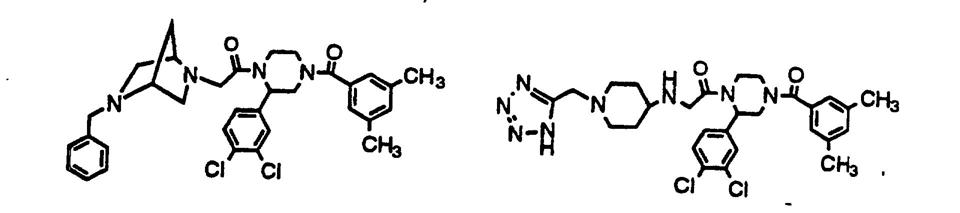 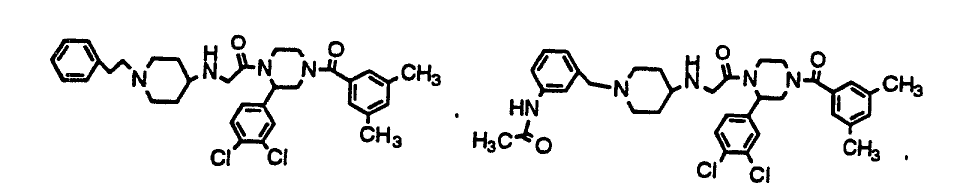 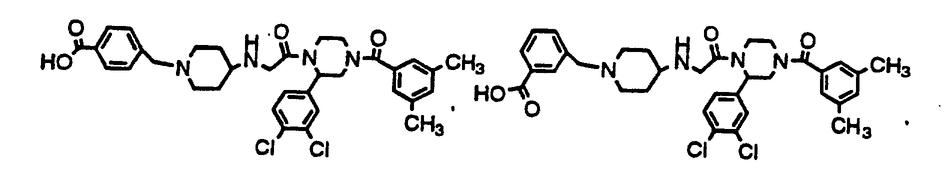 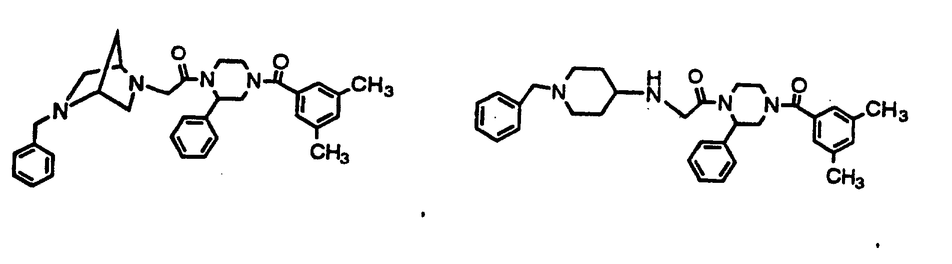 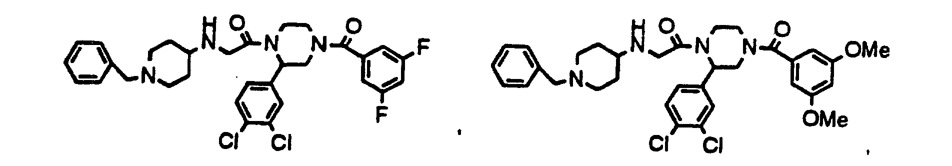 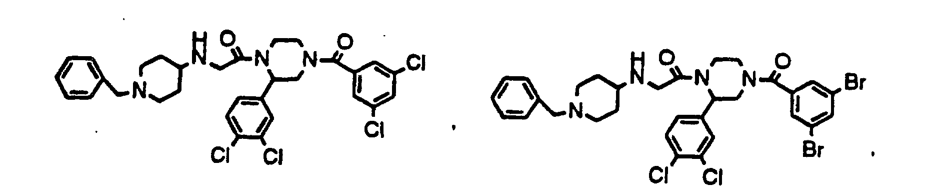 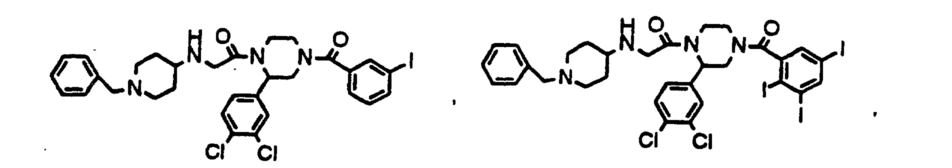 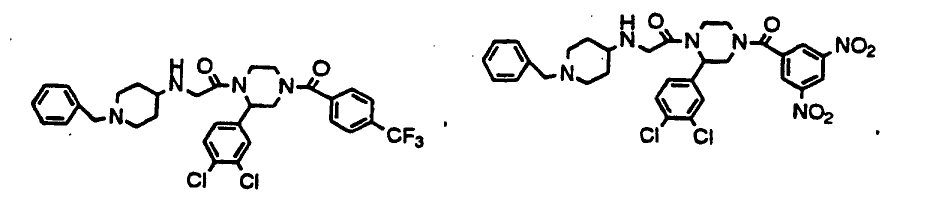 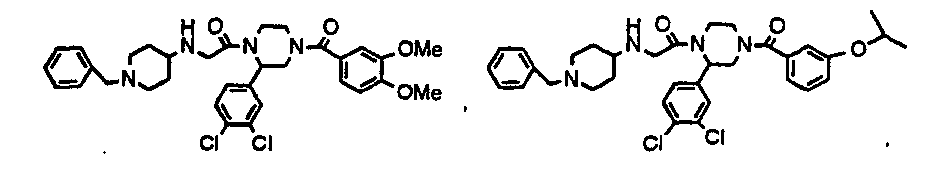 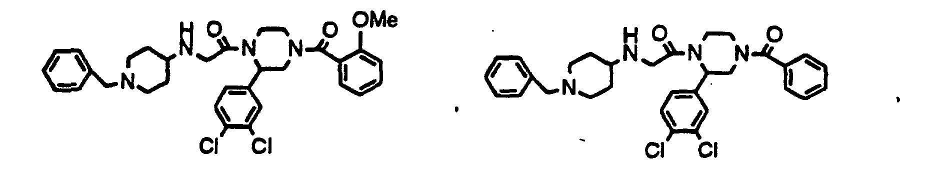 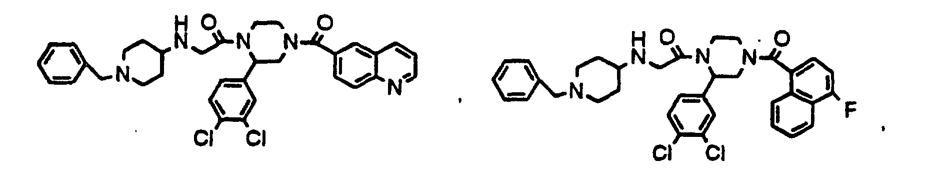 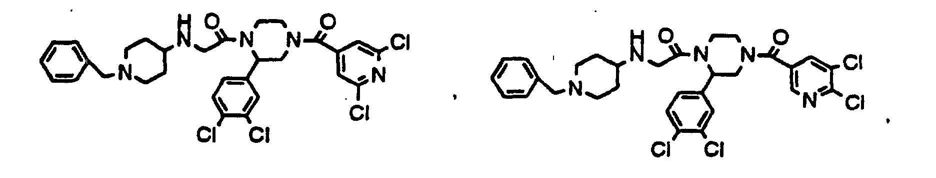 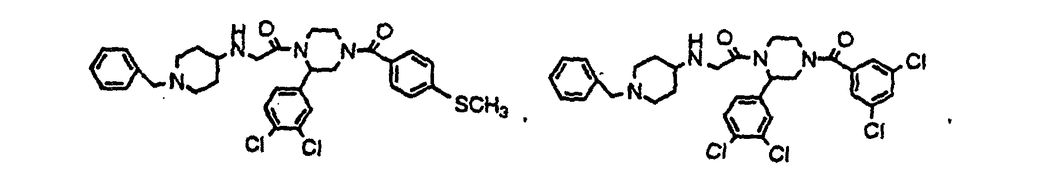 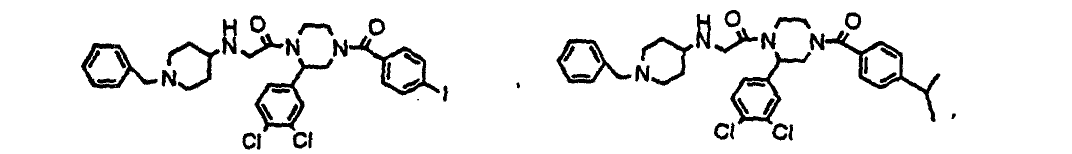 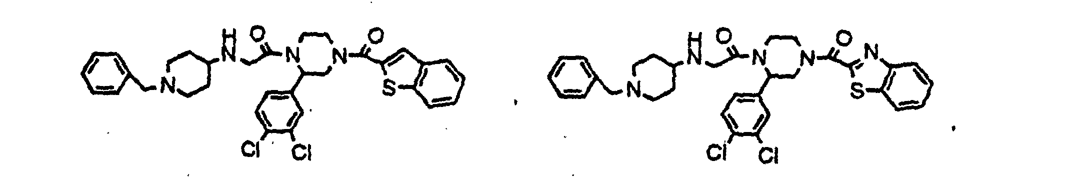 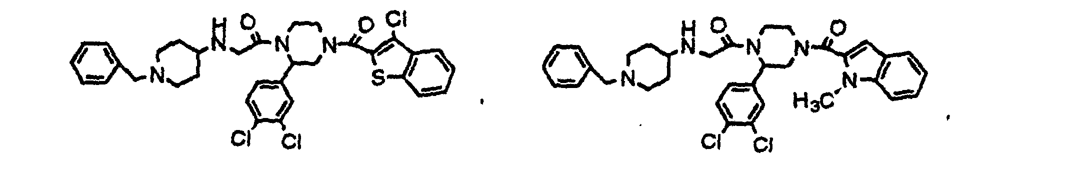 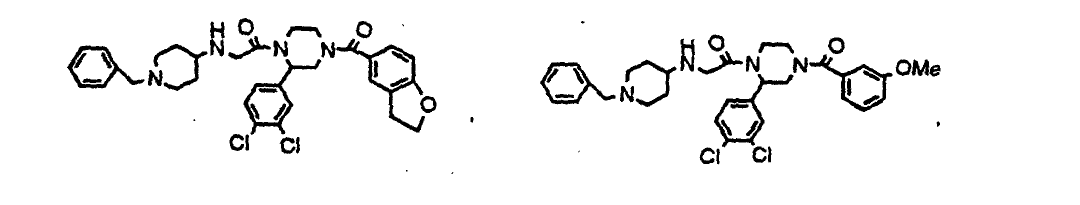 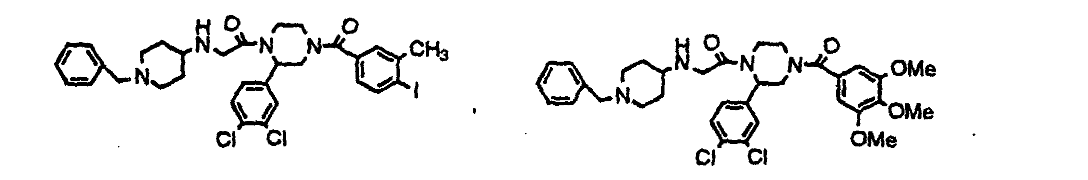 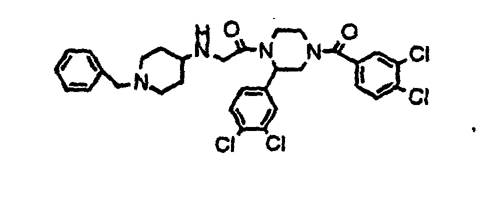 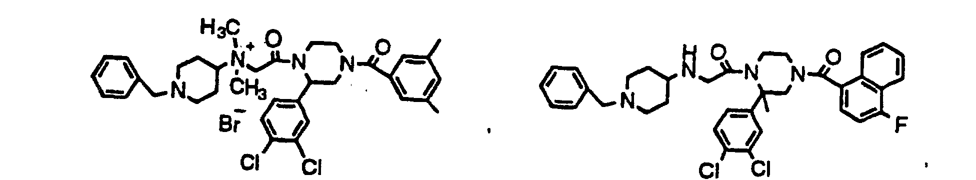 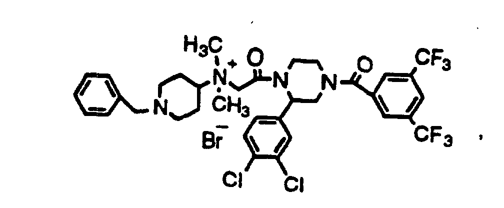 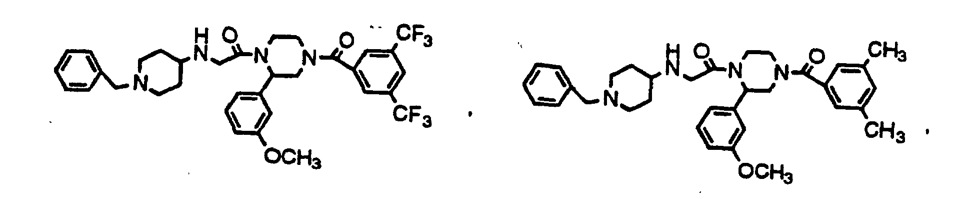 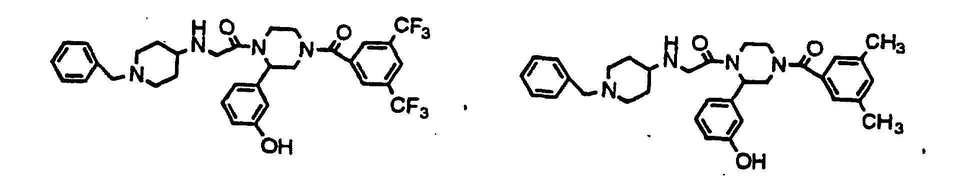 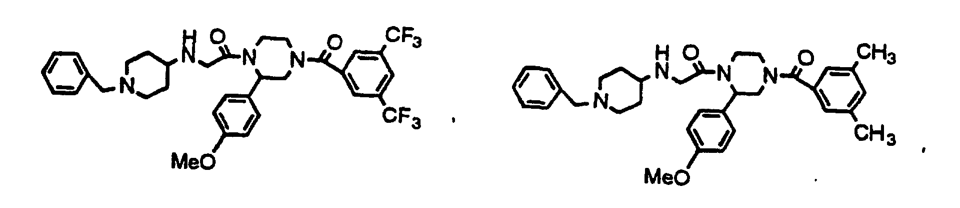 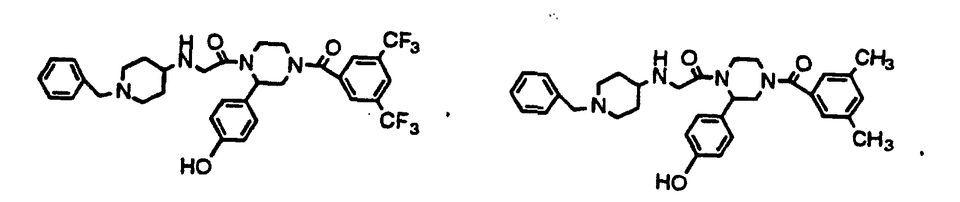 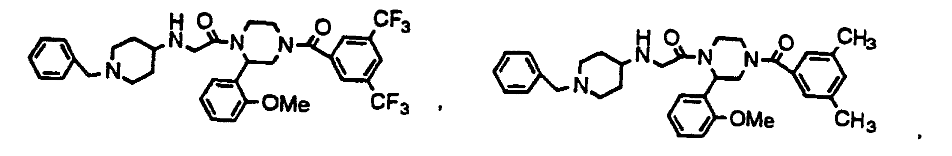 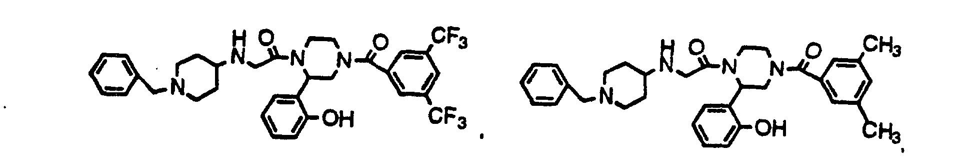 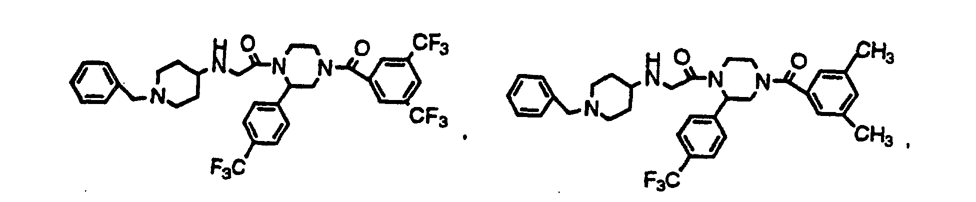 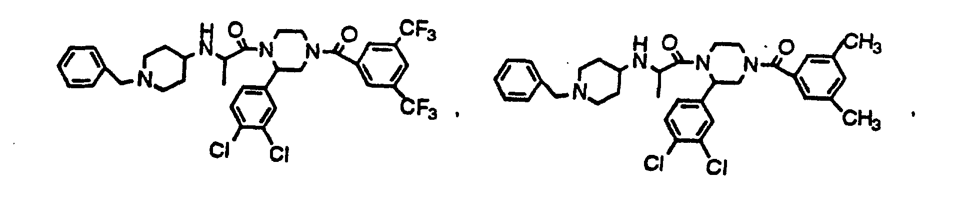 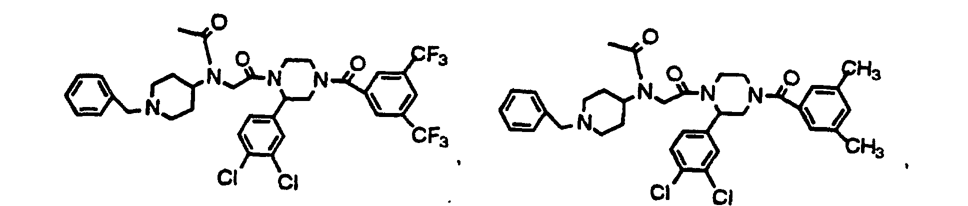 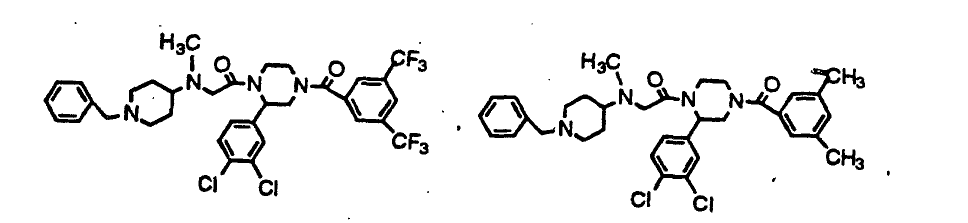 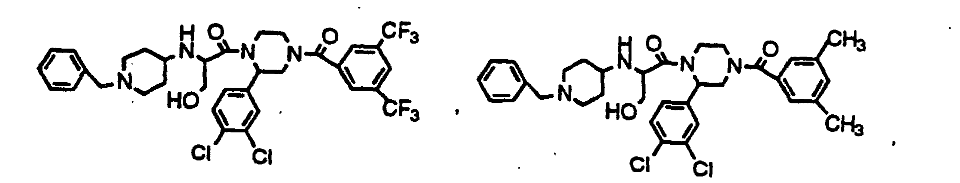 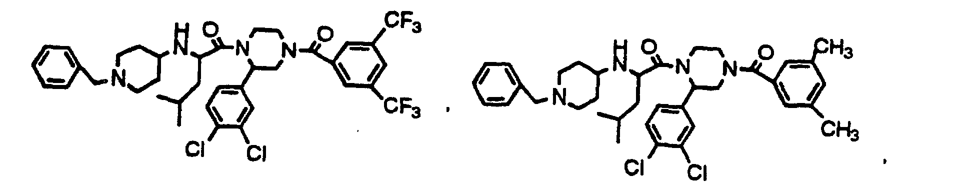 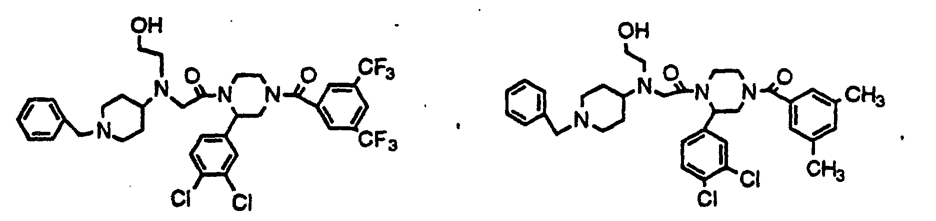 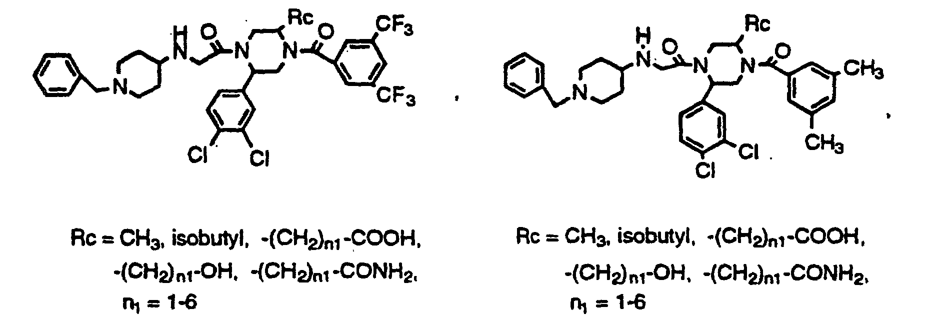 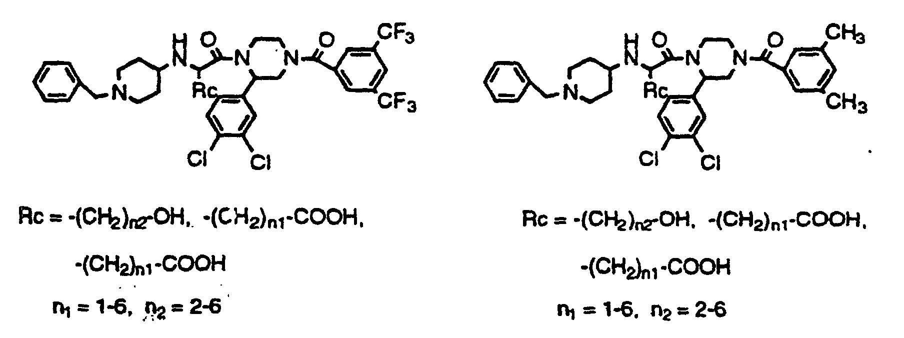 or a pharmaceutically acceptable salt thereof.

The invention also relates to a pharmaceutical composition comprising a thereapeutically effective amount of a compound of formula I in combination with a pharmaceutically acceptable carrier.

The invention also relates to the use of a compound of Formula I for the manufacture of a medicament for inducing neurokinin antagonism.

The invention further relates to the use of a compound of Formula I for the manufacture of a medicament for the treatment of chronic airway diseases; (such as asthma and allergies); inflammatory diseases (such as inflammatory bowel disease, psoriasis, fibrositis, osteoarthritis, and rheumatoid arthritis; migraine; central nervous system disorders (such as depression, psychosis, dementia, and Alzheimer's disease); Down's syndrome, neuropathy; multiple sclerosis; opthalmic disorders, conjunctivitis; auto immune disorders' graft rejection; systemic lupus erythematosus; Gl disorders (such as Crohn's disease and ulcerative colitis; disorders of bladder function; circulatory disorders such as angina; Raynaud's disease; coughing and pain. In particular, the compounds of Formula I are suitable for use in the manufacture of a medicament for treating asthma.

### Detailed Description of the Invention

As used herein the term alkyl means a straight or branched, saturated hydrocarbon chain having from 1 to 6 carbon atoms. The number of carbon atoms may be designated. For example, "C₁-C₆ alkyl" represents a straight or branched, saturated hydrocarbon having from 1 to 6 carbon atoms.

The term alkenyl means means a straight or branched, saturated alkenyl having from 2 to 6 carbon atoms. The number of carbon atoms may be designated. For example, "C₂-C₆ alkenyl" represents a straight or branched alkenyl having from 1 to 6 carbon atoms.

The term alkynyl means a straight or branched alkynyl having from 2 to 6 carbon atoms. The number of carbon atoms may be designated. For example, "C₂-C₆ alkynyl" represents a straight or branched chain alkynyl having from 2 to 6 carbon atoms.

As used herein, a heavy dark line ( 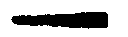 ) denotes a chemical bond coming above the plane of the page. A dashed line ( 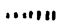 ) denotes a chemical bond coming below the plane of the page.

As used herein, 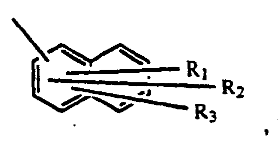 for example, means that R₁, R₂, and R₃ can be in either of the rings of the above naphthyl moiety.

Asymmetric centers exist in compounds of formula I of the invention. Accordingly, compounds of formula I include stereoisomers.

All such isomeric forms and mixtures thereof are within the scope of the present invention. Unless otherwise indicated, the methods of preparation disclosed herein may result in product distributions which include all possible structural isomers, although it is understood that physiological response may vary according to stereochemical structure. The isomers may be separated by conventional means such as fractional crystallization, preparative plate or column chromatography on silica, alumina, or reversed phase supports or HPLC (high performance liquid chromatography).

Enantiomers may be separated, where appropriate, by derivatization or salt formation with an optically pure reagent, followed by separation by one of the aforementioned methods. Alternatively, enantiomers may be separated by chromatography on a chiral support.

The compounds of formula I can exist in unsolvated as well as solvated forms, including hydrated forms, e.g. the hemihydrate. In general, the solvated forms, with pharmaceutically acceptable solvents such as water, ethanol, and the like are equivalent to the unsolvated forms for the purposes of the invention.

Those compounds of formula 1 which contain a basic group such as -CH₂NH₂, form pharmaceutically acceptable salts. The preferred pharmaceutically acceptable salts are nontoxic acid addition salts formed by adding to a suitable compound of the invention about a stoichiometric amount of a mineral acid, such as HCI, HBr, H₂SO₄ or H₃PO₄ or of an organic acid such as acetic, propionic, valeric, oleic, palmitic, stearic, lauric, benzoic, lactic, para-toluenesulfonic, methanesulfonic, citric, maleic, fumaric, succinic and the like, respectively.

### General Methods of Preparation

The compounds of this invention may be prepared by one of the following general methods. As used herein RT means room temperature. Unless otherwise indicated, variables in the structural formulas below are as defined above. Starting materials and reagents used in the methods and examples below, are known or may be prepared according to known methods.

As used herein the term "substituted phenyl" means 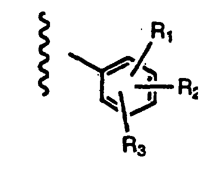 wherein R₁, R₂, and R₃ are as described herein.
"substituted " means substituted by R₁, R₂, and/or R₃ as described herein.
"Aryl" means phenyl, naphthyl, indenyl, tetrahydronaphthyl, indanyl, anthracenyl or fluorenyl.
"Halogeno" refers to fluoro, chloro, bromo or iodo atoms.
"Heterocycloalkyl" refers to 4- to 6-membered rings comprising 1 to 3 heteroatoms independently selected from the group consisting of -O-, -S- and -N(R⁶)-, with the remaining ring members being carbon. Examples of heterocycloalkyl rings are tetrahydrofuranyl, pyrrolidinyl, piperidinyl, morpholinyl, thiomorpholinyl and piperazinyl.
"Heteroaryl" refers to 5-to 10-membered single or benzofused aromatic rings comprising 1 to 3 heteroatoms independently selected from the group consisting of -O-, -S- and -N=. Examples of single-ring heteroaryl groups are pyridyl, isoxazolyl, oxadiazolyl, furanyl, pyrrolyl, thienyl, imidazolyl, pyrazolyl, tetrazolyl, thiazolyl, thiadiazolyl, pyrazinyl, pyrimidinyl, pyridazinyl and triazolyl. Examples of benzofused heteroaryl groups are quinolinyl, thianaphthenyl and benzofurazanyl. N-oxides of nitrogen-containing heteroaryl groups are also included. All positional isomers are contemplated, e.g., 1-pyridyl, 2-pyridyl, 3-pyridyl and 4-pyridyl.

Where R² and R³ substituents form a ring and additional heteroatoms are present, the rings do not include adjacent oxygen and/or sulfur atoms or three adjacent heteroatoms. Typical rings so formed are morpholinyl, piperazinyl and piperidinyl.

As used herein, the term "BOC" means t-butoxycarbonyl.

As used herein, the term "Ph" means phenyl.

As used herein, the term "RT" means room temperature.

As used herein, the term "parallel synthesis" means the preparation of individual chemical compounds as one of a batch of, for instance, 20, 30, or even 100 identical reactions on usually a single substrate but using a different reagent in each vessel. Such reagents are always of the same general class- in this case, either carboxylic acids or organic amines in any set of parallel reactions. The conditions used for each reaction are identical to those described in the examples, except that a simplified work-up is employed, generally a simple wash either with acid or base if appropriate, then water. The presence of the product is detected by thin layer chromatography (TLC) using known products as representative standards. Further characterization by combination HPLC/MS is generally performed. No further purification is performed on these materials before they are submitted to biological assays.

As used herein, each R_{c} and R_{c'} is independently selected from the group consisting of H, C₁-C₆ alkyl, C₂-C₆ alkenyl, C₂-C₆ alkynyl, unsubstituted or substituted phenyl, and unsubstituted or substituted benzyl,

The starting materials in the methods below are either known or can be prepared in accordance with known methods. In particular, the following compounds are either known or can be prepared in accordance with known methods: the diamine A, the compounds of formulas A, VI, VIII, X, XI, XIV, XVIII, XIX, XXa, A', XXV, and Z-H, as well as esters of formula XI, and compounds of formula

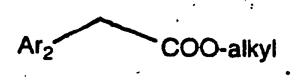

**Method 1.** If the group Ar₂ is an aromatic group with no I or Br substituents, then the following method may be used to prepare the useful intermediates (IV):

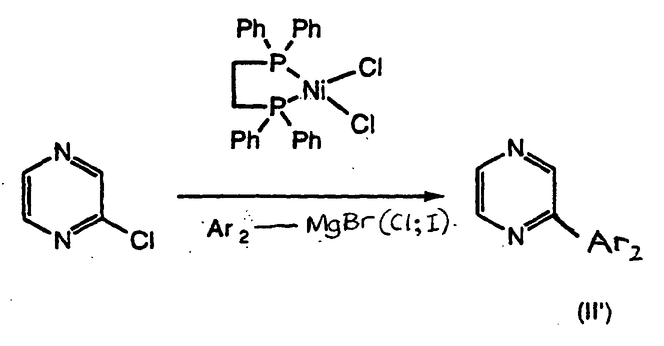

Transition metal catalyzed coupling of 2-chloropyrazine with an aromatic Grignard reagent in a dry, ether solvent, such as THF, yields the aryl-substituted pyrazine of formula II'. The catalyst shown, [1,2-bis-(diphenylphosphino)ethane]nickel^{II} chloride, is a preferred reagent for this transformation. Where Ar₂ has no halo substituents, reduction of a compound of formula II' by catalytic hydrogenation, using, for instance, palladium acetate, preferably in acetic acid solvent, results in preferential reduction of the pyrazine ring, leaving the aromatic ring unreduced, that is, it results in a compound of formula II. Similarly, 10% Pd on charcoal (Pd-C) can be used in an alcohol solvent, preferably methanol, with or without the addition of a small quantity (1 to 5 equivalents) of acetic acid. Reaction times of from 1 to 24 hours generally suffice for this reaction, which is preferentially run at room temperature or slightly above (up to about 50°C) and using from 1 to about 6 atmospheres pressure of hydrogen.

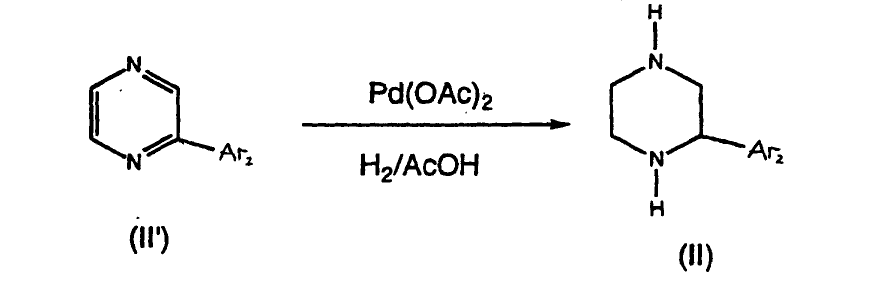

The intermediate of formula II may also be prepared from a compound of formula II', even if the group Ar₂ contains halogen atoms, by reduction using a strong hydride ion donor, preferably lithium aluminum hydride (LAH) or diisobutyl aluminum hydride (DIBAL-H) in an ether solvent, such as ether, THF or dimethoxyethane (DME).

Selective alkylation of a compound of formula II is possible using low temperature conditions. Thus, reacting a compound of formula II with a substituted aryl-alkyl halide of formula III where I is 0 to 2, results in the formation of the 4-substituted derivative of formula IV. Suitable conditions include use of a halogenated solvent, such as CH₂Cl₂, at low temperature. Suitable temperatures are from -78°C initially, allowing the reaction mixture to warm gradually to RT if the reaction is not completed after several hours. The reaction is catalyzed by the addition of an equivalent amount of an organic base, such as triethylamine and diisopropylethylamine (Hünig's base). 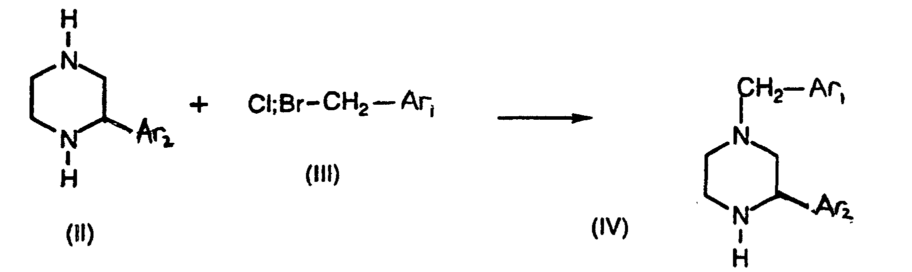 **Method 2.** If the group Ar₂ contains one or more halogen atoms on an aromatic ring and the other groups are as in Method 1, then an alternate route to a compound of formula IV is preferred. In addition, this method can be used to prepare compounds in which I is from 0 to 2. Mono-protection of the diamine of formula (A), preferably with BOC anhydride, or other agents known to introduce the t-butyloxycarbonyl protecting group, in an alcohol solvent, such as methanol, preferably at about -10°C, produces a compound of formula V.

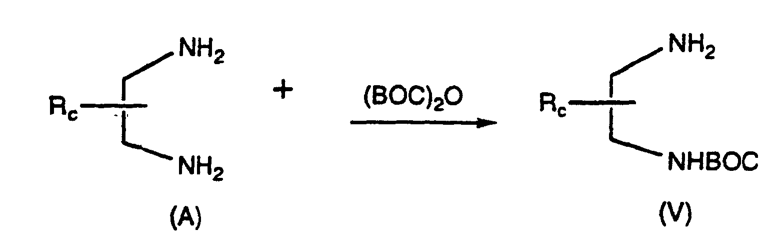

These compounds are used to perform a reductive amination reaction with the aldehyde of formula VI to produce an amine of formula VII. (In structures (A), (V), (VII), and (IX) herein, R_{c} can be bound to any position between the two nitrogens. In cyclic structures like (IVA) below, R_{c} can be bound to any available cyclic position that is occupied by carbon, and that is between the two nitrogens.)

Suitable conditions for this type of reaction include the use of an alcohol solvent, preferably methanol, made slightly acidic with a weak organic acid, such as acetic acid, and a reducing agent known to favor reductive amination reactions, preferably sodium cyanoborohydride, NaBH₃CN.

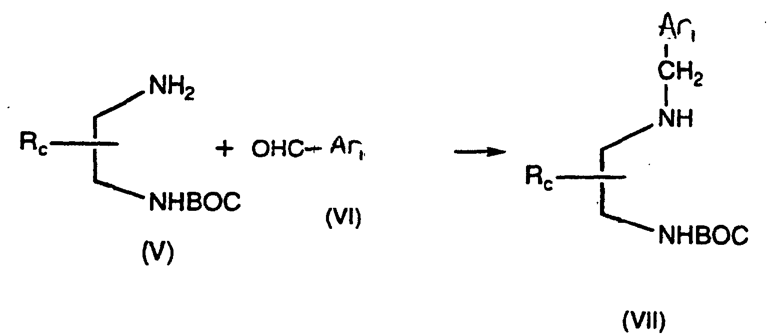

Reaction of a compound of formula VII with a phenacyl halide derivative of formula VIII, in which Ar₂ preferably represents a halogenated aromatic ring, but may be any of the claimed aromatic rings, in the presence of an organic base, such as di-isopropylethylamine, also known as Hünig's Base, in an ether solvent, such as THF, results in the formation of the intermediates of formula IX.

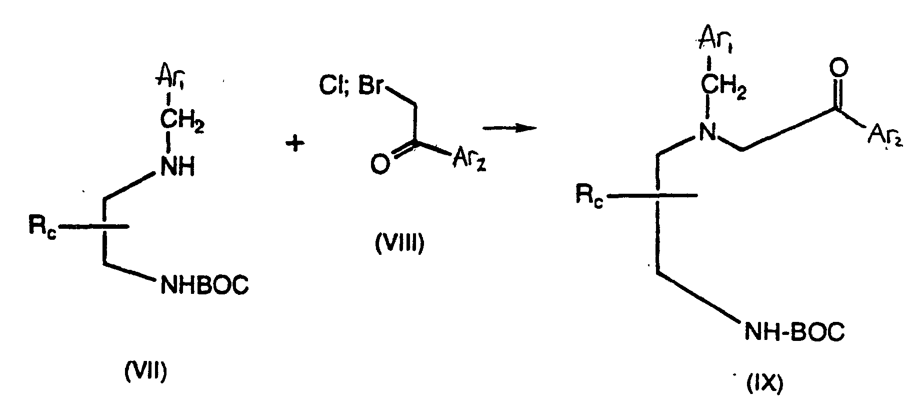

Removal of the BOC protecting group using a suitable acidic catalyst, such as trifluoroacetic acid, followed by an intramolecular reductive amination, under conditions such as those described above for the preparation of a compound of formula VII, leads to the formation of compounds of formula IVA.

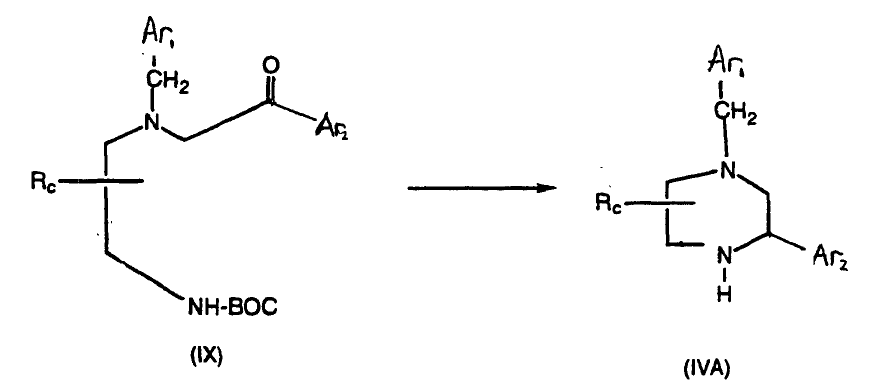

**Method 3.** An alternate route to compounds of the invention in which I is 0 to 2 is as follows. Standard coupling of an N-protected amino acid of formula X, wherein Ar₂ is as described above, such as Ar₂-CH(NHProt)CO₂H, with a glycine ester, or an amino acid ester derivative with 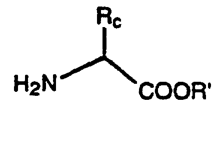 (R' is C₂-C₄ alkyl, for instance, the ethyl ester of formula XI . Et in the formulas herein means ethyl), produces a dipeptide of formula XII. A suitable protecting group is BOC, although many others may also be used. Other esters of glycine may also be used. Standard coupling techniques may be applied, an example being the use of N-hydroxybenztriazole (HOBT) and a water-soluble carbodiimide, such as 1-(3-dimethylaminopropyl)-3-ethylcarbodiimide (DEC), in a non-hydroxylic solvent such as CH₂Cl₂, DMF or a mixture of the two foregoing solvents. The reaction is run, preferably, at or below RT, and takes from 1 to 40 hours for completion, depending upon the substrates.

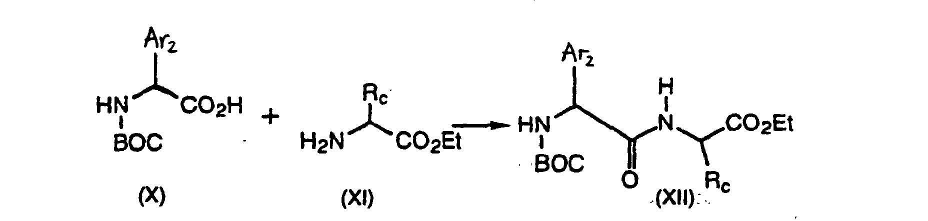

Removal of the protecting group under standard conditions, followed by treatment of the product with a base results in cyclization to the diketopiperazine of formula XIII. Suitable conditions for removal of the exemplified BOC group are well known in the art and include catalysis by trifluoroacetic acid (TFA). A suitable base for cyclization is the alkali metal salt of an alcohol in the alcohol itself used as solvent. For example, a solution of sodium ethoxide in ethanol may be used. The temperature is preferably around RT but may be slightly above or below, in the range 0°C to about 40°C. The reaction is generally complete within a few hours. Suitable reaction times are from 1 to 24 hours.

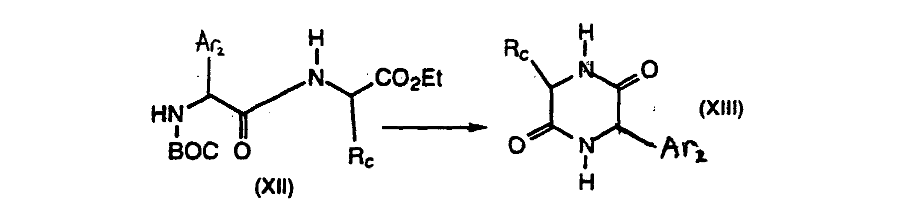

Reduction of the diketopiperazine of formula XIII to a compound of formula II may be accomplished preferentially with a strong hydride reducing agent, such as LAH or a solution of sodium bis(2-methoxyethoxy)aluminum hydride in toluene (also known as Red-Al®), or the BH₃.S(CH₃)₂ complex. Suitable solvents for this reaction are DME and other higher boiling ethers since the reaction is run at elevated temperatures, from about 50°C to about 110°C, preferably at about 90°C.

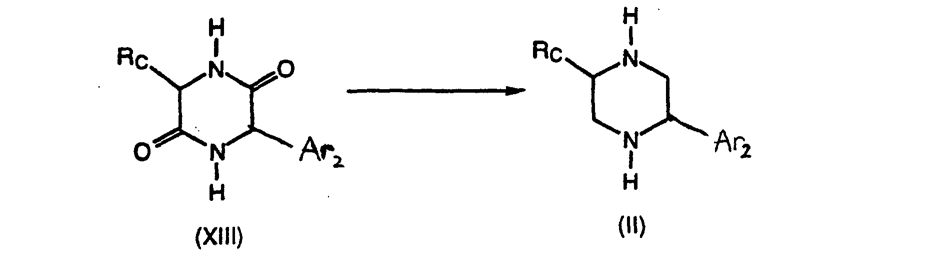

Alternatively, a compound of formula of II may be prepared by the scheme shown below ( J. Med. Chem., 9, 191 (1966)). As used herein L is any readily available ester residue such as C₁-C₇ alkyl, more preferably methyl or ethyl.

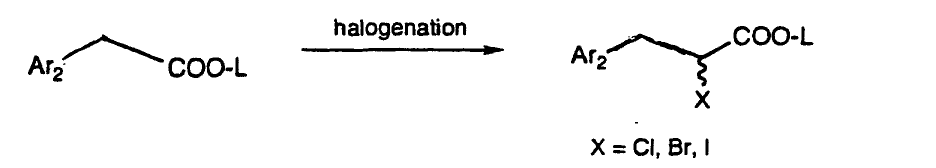

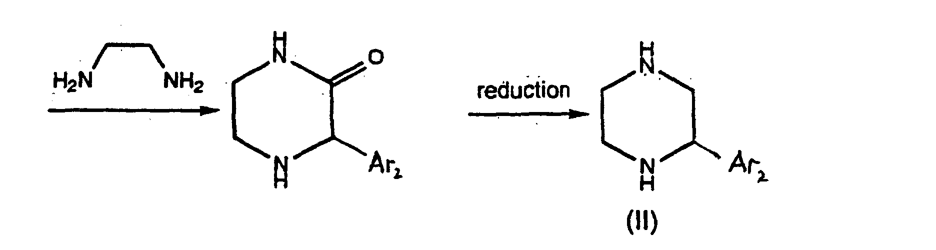

A compound of formula it may be converted to a compound of formula IV by the processes described in Method 1 above or Method 6 below.

**Method 4.** The intermediates of formula IV or IVA, formed via any of the previous methods, may be further processed as follows. A compound of formula IVA will be used in the Schemes. Reaction of a compound of formula IVA with an activated halo-acid, generally the acid halide of formula XIV, in which Hal represents Cl, Br, or I, yields the acylated derivative of formula XV that is, m is 1 for formula I. An organic base is used to take up the hydrogen halide formed in the reaction, suitable bases being triethylamine (TEA) and Hünig's Base. Suitable reaction media include halogenated solvents, such as methylene chloride and chloroform. The reaction is preferably run at low temperature, at least initially. Suitable temperatures are in the region of -50°C down to -80°C. Later in the reaction it may be desirable to allow the mixture to warm up to about RT to ensure completion of the reaction.

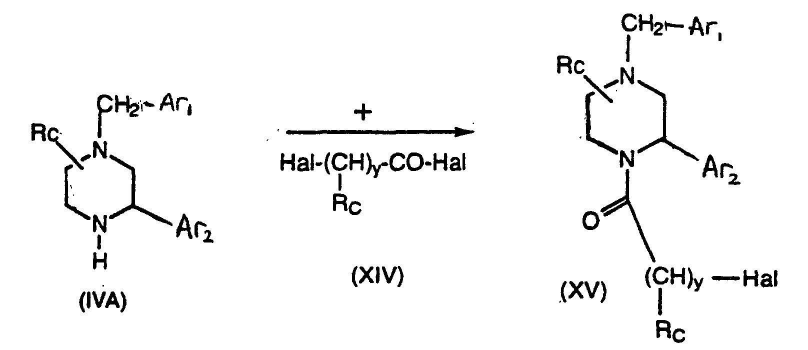

Reaction of the halogenated amides of formula XV with an amine of formula Z-H results in formation of the products of formula XVI, which are compounds of the invention in which X is O and m is 1. Compounds of formula XVI have been modified to show the fact that these products could have been prepared from compounds of formula IVA as well as from IV. Suitable solvents for this reaction are halogenated hydrocarbons, such as methylene chloride, and an organic base is present to absorb the H-Hal formed. Appropriate bases include Hünig's Base. The reaction is performed at or around RT, a suitable temperature being generally in the range of from 0°C to 40°C. Reaction is complete within 1 to 48 hours. 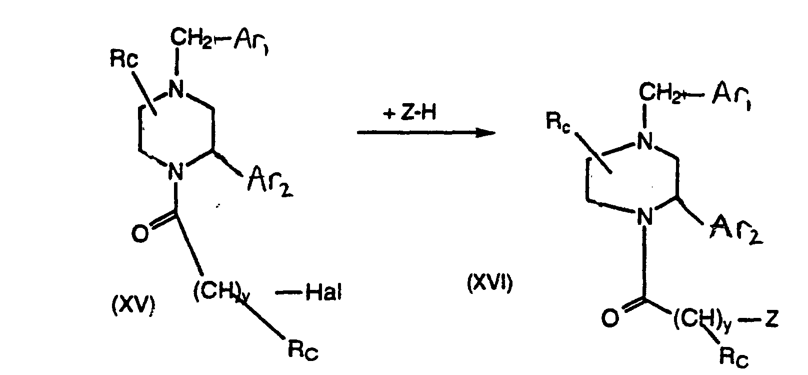 **Method 5.** Compounds of formula XVI where y ≠ 0 may be converted to other compounds of the invention of formula XVII by reduction under controlled conditions.

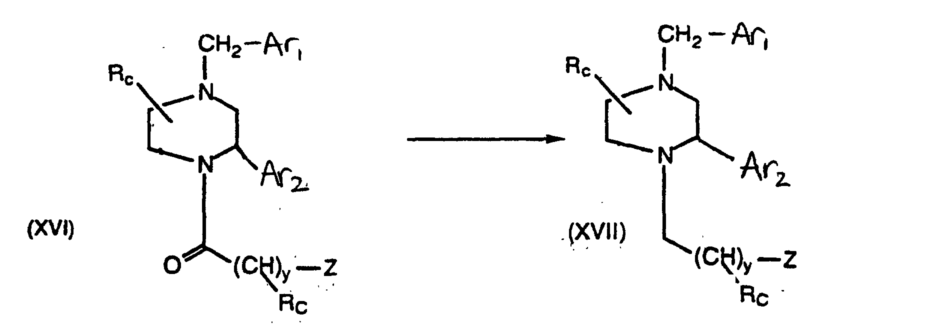

Suitable reducing agents to effect this transformation include the borane-dimethyl sulfide complex, as well as other less selective reagents, such as LAH, (assuming that no other group reactive to LAH is present), Red-Al®, and diborane in ether. Effective temperatures for the boranedimethylsulfide complex to reduce compounds of formula XVI, range from RT to the reflux temperature of the solution of the reagent in THF (about 80°C).

**Method 6.** Intermediates of the formula XVIII may be selectively acylated by coupling with an acid of the formula XIX. Standard coupling techniques may be applied, an example being the use of HOBT, a water-soluble carbodiimide, such as DEC, and an organic base, such as triethylamine, in a non-hydroxylic solvent, such as CH₂Cl₂, at a temperature of about -20°C initially. The mixture may be allowed to warm to RT to complete the reaction. The product of reaction is the amide of formula XX.

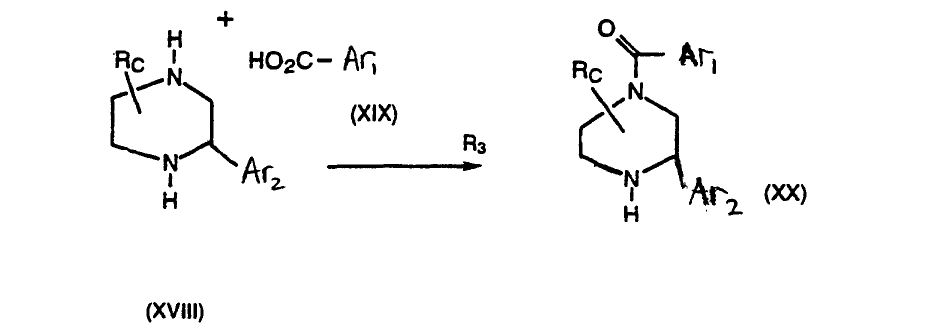

Compounds of the formula XX, may be further acylated using an acid halide of formula XXa. The reaction is run, preferably at about -78°C, over a period of 1 to 12 hours, in a halogenated solvent, such as methylene chloride or similar solvent. An organic tertiary amine is used to absorb the H-Hal produced in the reaction. Suitable amines include triethylamine and Hünig's Base. As used herein Hal means Cl, Br, or I.

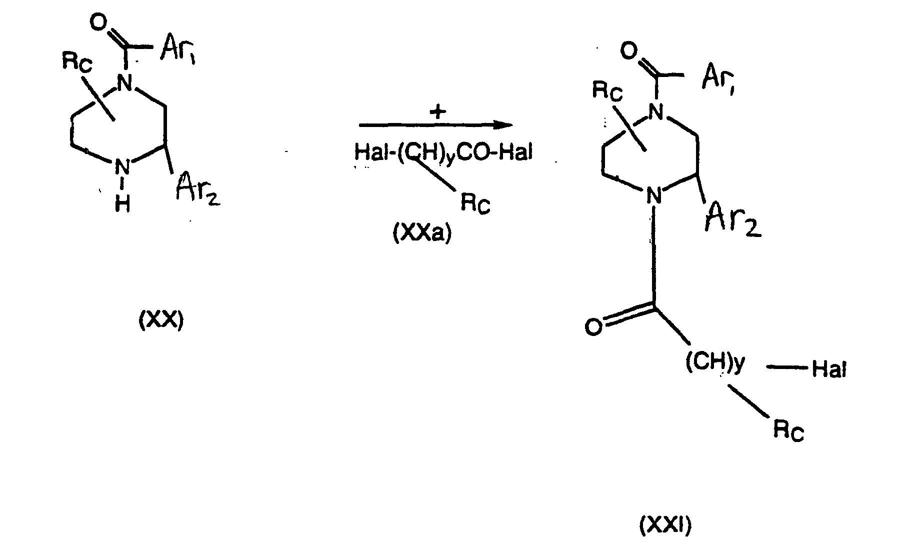

The compounds of formula XXI, that is, m is 1 in formula I, y = 1-3, I = 0-2 may be used for further reaction without isolation. Additional organic base, for instance, Hünig's Base, is added to the mixture followed by Z-H, at or around -78°C. The reaction is completed by allowing the mixture to warm to RT overnight yielding the compounds of formula XXII after work-up and purification by standard methods.

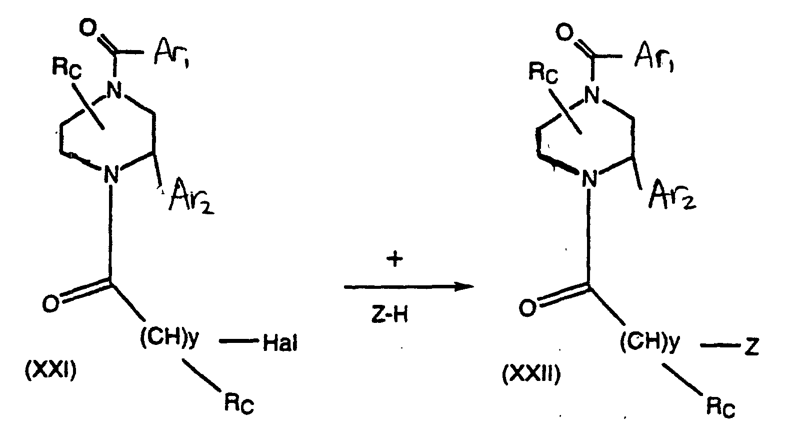

The compounds of formula XXII, in which y = 1-3 may be converted to other products of formula XXIII by reduction under controlled conditions.

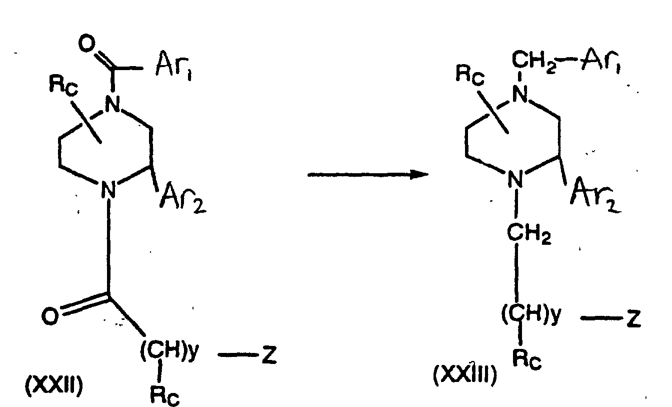

Suitable reducing agents to effect this transformation include the borane-methyl sulfide complex, as well as other less selective reagents, such as LAH, Red-Al®, and diborane in ether or other unreactive solvents, such as THF. Using the borane-methyl sulfide complex in THF, at the reflux temperature of the solution, which is about 80°C, the reaction is complete in about 2 hours to 48 hours depending on the precise substrate.

Some of the substrates Z-H for the alkylation reaction were synthesized from 4-amino-1-benzylpiperidine (A) by initial conversion to the t-BOC protected derivative(B) followed by removal of the benzyl group by hydrogenolysis over a suitable catalyst such as Pd(OH)₂ to yield the t-BOC protected piperidine (C). Subsequent elaboration of this piperidine can be accomplished by either alkylation or reductive alkylation depending on the availability of reagents for these reactions.

Reaction of the intermediate (C) with an aldehyde or ketone (D) under the conditions of reductive alkylation, such as in methanol and in the presence of NaBH₃CN with sufficient AcOH (acetic acid) present to allow the reaction to proceed at a suitable rate, produces the amine (E) from which the t-BOC group may be removed, for instance, with 4N-HCl in dioxane followed by basification for instance, with an aqueous solution of NaOH, to produce the compound of formula (F).

The same product, (E), may be prepared from (C) by alkylation with the halide derivative (G) in which "Hal" is Cl, Br, or I. Other activated leaving groups are also possible for this reagent , such as mesylates or tosylates. The reagent is preferably primary but the reaction can also often be made to work acceptably for secondary derivatives.

The product of the alkylation, (E), may be treated as described above to produce the 4-aminopiperidine (F) which represents one of the preferred forms of Z which can be used to convert a compound of formula XXI to a compound of formula XXII.

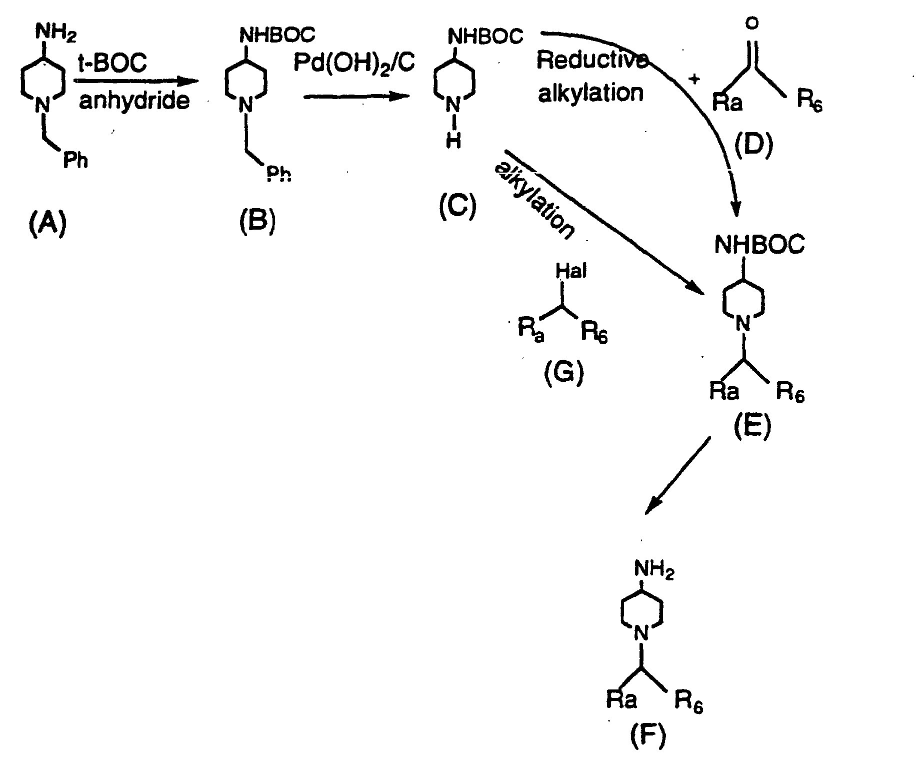

**Method 7.** The acylated derivatives of formula XX from Method 6 may be reduced to the saturated alkyl chain derivatives of formula XXIV.

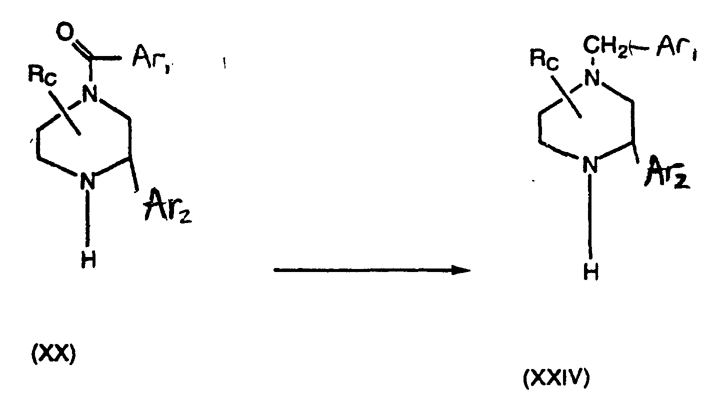

The process to conduct this conversion is the same as described in Method 6 for conversion of a compound of formula XXII to a compound of formula XXIII. The reagent of preference is the borane-methyl sulfide complex.

Reaction of the intermediate of formula XXIV with the acyl halide of formula XXV at temperatures of about -78°C produces the amides of formula XXVI. The reaction is run preferably in a halogenated solvent, such as methylene chloride, in the presence of an organic base to take up the H-Hal formed. A suitable base is Hünig's Base. The product, a compound of formula XXVI, may be used without isolation in the subsequent step.

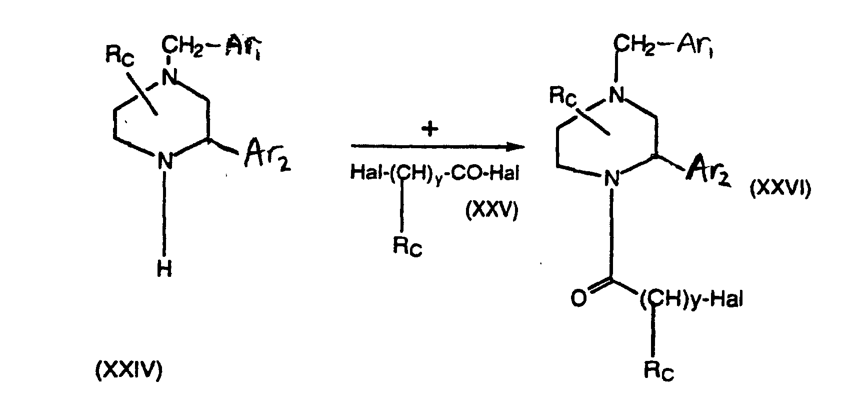

The halo-derivative of formula XXVI may be used without isolation to react with the amine compound of formula Z-H. An additional equivalent amount of a suitable organic base, such as Hünig's Base, is added to the mixture to consume H-Hal. The reaction is initially run at about -78°C but is allowed to warm gradually to RT to complete the reaction. The product, a compound of formula XXVII, is isolated by conventional techniques and may be purified by flash chromatography.

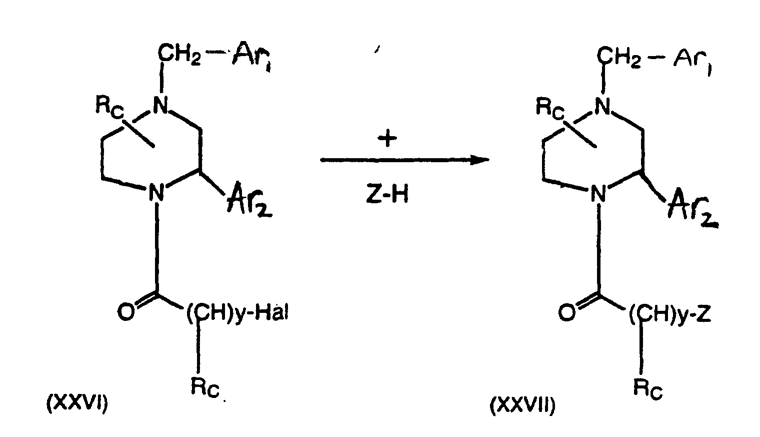

An alternate route to compounds of structure (XXII) also starts with compound (XVIII). Initial reaction with an amine protecting group reagent, preferably BOC anhydride, produces the N-t-butyloxycarbonyl derivative of the formula XXVIII.

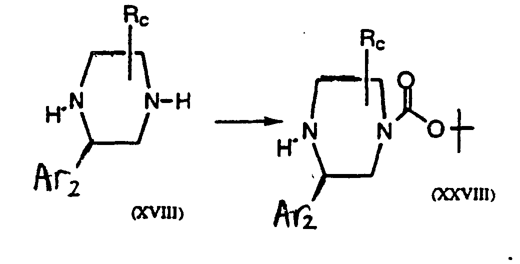

As before, reaction occurs preferentially at the nitrogen atom further away from the Ar₂ group. Reaction of this intermediate with a reagent of structure (XXa) as described above, leads to the halo-derivative (XXIX). Reaction of (XXIX) with Z-H, again as described above, produces the intermediate (XXX) which may be de-protected to produce (XXXI). Suitable reagents include trifluoroacetic acid and HCl.

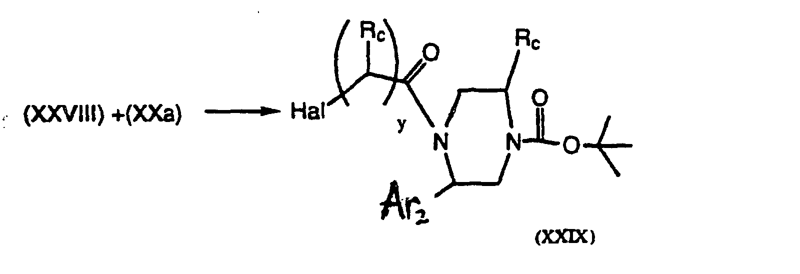

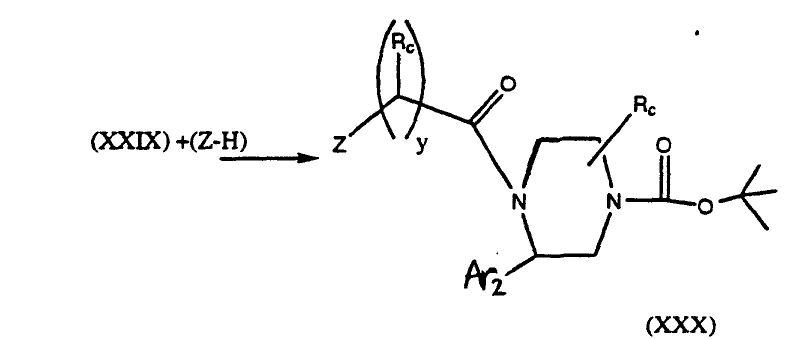

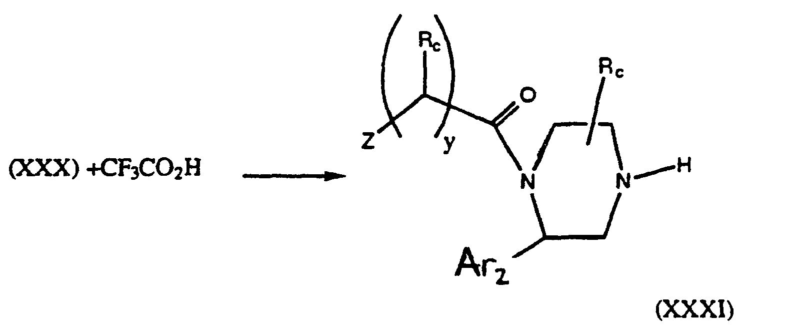

Reaction of (XXXI) with a carboxylic acid (XIX) under such coupling conditions as described above, leads to the products of formula (XXII).

### Method 7a.

Synthesis of the compounds of the invention wherein the pendant aromatic group Ar₂, or the pendant aromatic group Ar₂ and its sidechain, are located in the alternate ring position to the compounds of formula XXII (ie compounds of formula C below), may be prepared using compounds of formula XXVIII from method 7 as starting materials. Coupling of compounds of formula XXVIII with any of the acids of the invention Ar― CO₂H under standard coupling conditions, for instance using HOBT, Et₃N and DEC in CH₂Cl₂, produces the intermediate (A). Removal of the t-BOC or other protecting group under standard conditions releases the free amine (B). Acylation of (B) and further reaction with Z-H proceeds as described in Method 6 for the conversion of (XX) via (XXI) to (XXII) to produce compound (C) of the invention.

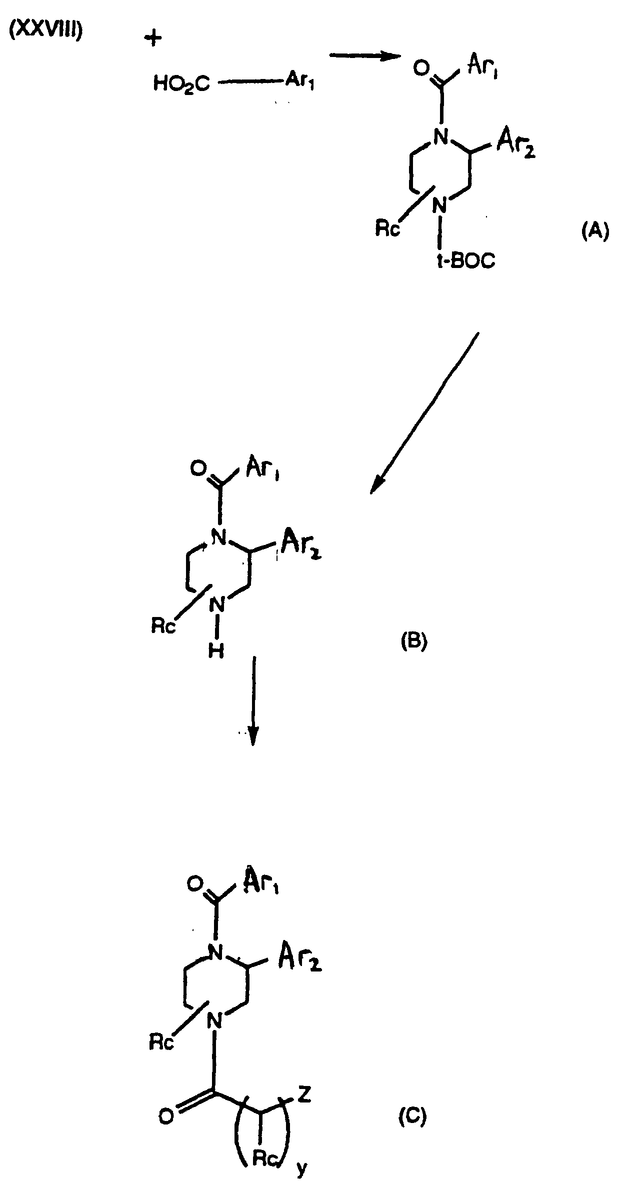

### Method 8.

A method for introducing a group, R_{c}, into the sidechain of a compound of the invention begins with a previously prepared compound of formula (XX). This may be coupled with a suitably protected amino-acid derivative of formula (XXXII) in which the t-BOC group is used as a representative protecting group. Use of a relatively reactive coupling agent, such as BOP-Cl of formula (XXXIII), is preferred and the reaction is run under standard coupling conditions well known to one skilled in the art. Suitable conditions include the use of CH₂Cl₂ and/or DMF as solvent, with triethylamine or Hünig's Base, and a temperature between 0°C initially and RT. Usual work-up conditions yield the protected intermediate of formula (XXXIV).

In the case of (XXXIV), in which the N-protecting group is t-BOC, the usual conditions for removal of such a group may be used to free the amine function. Various concentrations of CF₃CO₂H in CH₂Cl₂ will usually suffice. In some substrates a fairly dilute solution (e.g. 2N) will be sufficient whereas in other cases a more concentrated solution, up to neat TFA, may be necessary. In addition, other N-protecting groups may be employed and removed by methods well known in the art. An example is use of the N-Cbz which may be removed under either acidic or hydrogenolytic conditions. The result of deprotection is the amine intermediate of the formula (XXXV). 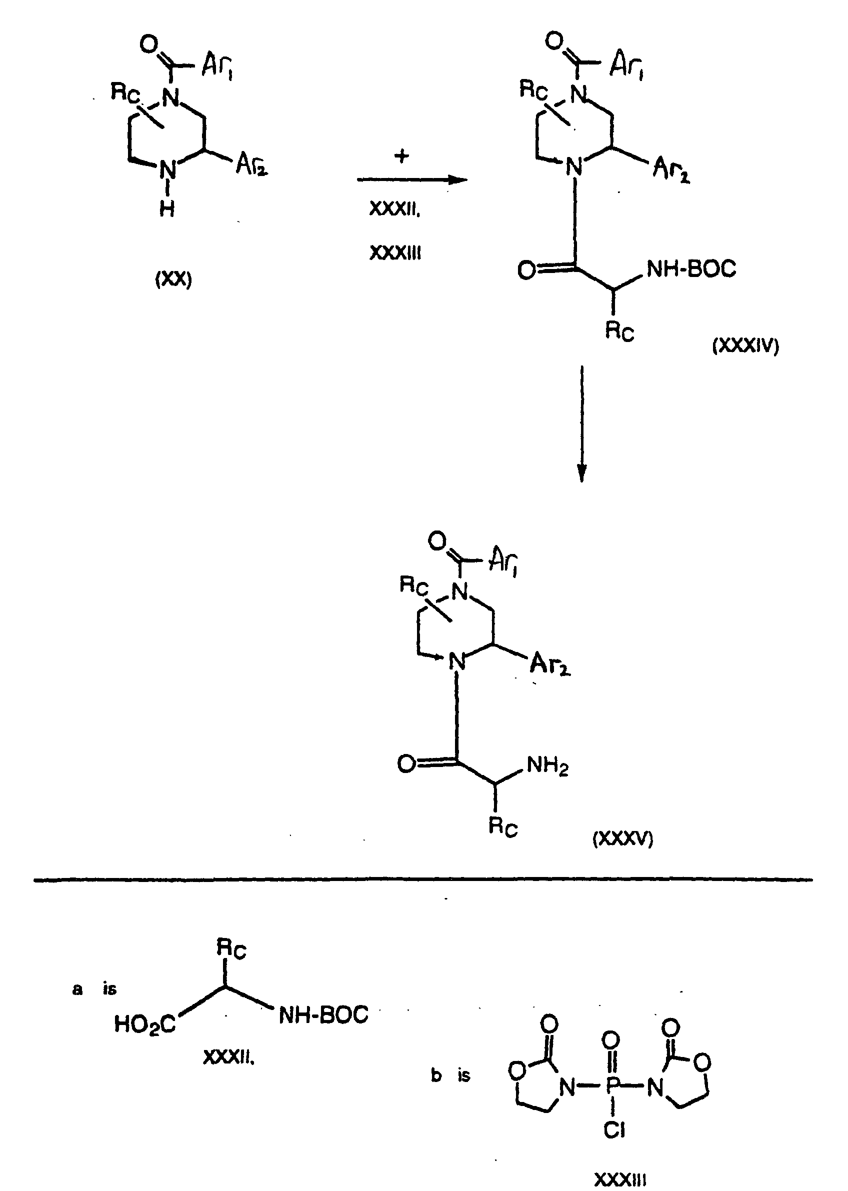 Conversion of intermediate of the formula (XXXV) to compounds of the invention is then carried out by a reductive alkylation process.

The group Z is introduced into the molecule using an aldehyde or ketone in which the aforementioned group is present at the carbon atom that is to be joined to the amino group of the formula (XXXV). An example of such an intermediate is compound of the formula (XXXVI).

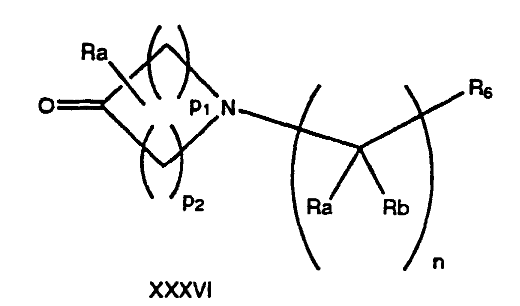

After the reaction this group becomes the Z group of the compounds of the invention, that is, the "Y-NH" group shown in compounds of the formula (XXXVII) just below 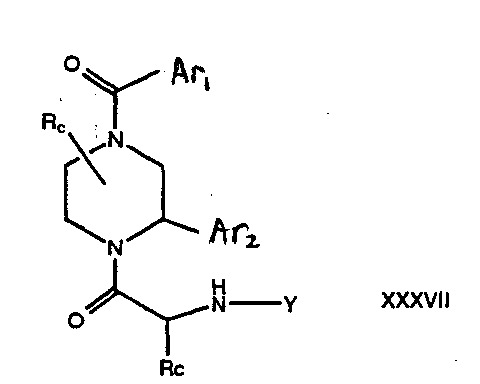 is equivalent to the "Z" group shown in the Summary of the Invention. Conditions for this reductive amination procedure are known in the art and are exemplified by the use of NaBH₃CN in MeOH with the addition of several equivalents of acetic acid. Generally, the reaction is performed at RT and is left to react overnight. Product is isolated by standard means, such as decomposition of excess reagent with H₂O and extraction of the product into an organic solvent such as CH₂Cl₂ or a mixture of Et₂O and CH₂Cl₂.

Using procedures similar to those described in the above or using procedures known to those skilled in the art, one can produce all of the compounds of formula I of the invention. For example one can obtain compounds of the invention of formula I wherein the R_{c} moiety is on various carbons of the piperazine ring.

The *in vitro* and *in vivo* activity of the compounds of formula I can be determined by the following procedures.

### In vitro procedure to identify NK₁ activity

Test compounds are evaluated for their ability to inhibit the activity of the NK₁ agonist Substance P on the isolated guinea pig vas deferens. Freshly cut vas deferens are removed from male Hartley guinea pigs (230-350g) and suspended in 25 ml tissue baths containing Kreb's Henseleit solution warmed to 37°C and constantly aerated with 95% O₂ and 5% CO₂. Tissues are adjusted to 0.5 g and allowed to equilibrate for a period of 30 minutes. The vas deferens are exposed to an electrical field stimulation (Grass S48 Stimulator) every 60 seconds at an intensity that will cause the tissue to contract 80% of its maximum capacity. All responses are recorded isometrically by means of a Grass force displacement transducer (FT03) and Harvard electronic recorder. Substance P inhibits the electrical field stimulated-induced contractions of the guinea pig vas deferens. In unpaired studies, all tissues (control or drug treated) are exposed to cumulative concentrations of Substance P (1X10⁻¹⁰ M - 7X10⁻⁷ M). Single log-concentrations of the test compounds are given to separate tissues and allowed to equilibrate for 30 minutes before a Substance P concentration-response curve is generated. At least 5 separate tissues are used for each control and individual drug-concentration for every drug assay.

Inhibition of the Substance P is demonstrated by a rightward shift of its concentration-response curve. These shifts are used to determine the pA₂ value, which is defined as the negative log of the molar concentration of the inhibitor which would require that twice as much agonist be used to elicit a chosen response. This value is used to determine relative antagonist potency.

### Isolated Hamster Trachea NK₂ Assay

General methodology and characterization of hamster trachea responses to neurokinin agonists as providing an NK₂ monoreceptor assay is found in C.A. Maggi, et al., *Eur. J. Pharmacol*. 166 (1989) 435 and J.L. Ellis, et al., *J. Pharm. Exp. Ther*. 267 (1993) 95.

Continuous isometric tension monitoring is achieved with Grass FT-03 force displacement transducers connected to Buxco Electronics preamplifiers built into a Graphtec Linearcorder Model WR 3310.

Male Charles River LAK:LVG (SYR) hamsters, 100-200 g fed weight, are stunned by a sharp blow to the head, loss of corneal reflex is assured, the hamsters are sacrificed by thoractomy and cutting the heart. Cervical trachea segments are removed to room temperature Krebs buffer, pH 7.4, aerated with 95% O₂ - 5% CO₂ gas and cleaned of adhering tissue. The segments are cut into two 3-4 mm long ring segments. Tracheal rings are suspended from transducers and anchored in 15.0 ml water jacketed organ baths by means of stainless steel hooks and 6-0 silk. Baths are filled with Krebs buffer, pH 7.4, maintained at 37°C and continuously aerated with 95% O₂ - 5% CO₂ gas. Tracheal rings are placed under 1.0 g initial tension and allowed a 90 min equilibration period with four 1 µM NKA challenge, wash and recovery cycles at 20 min intervals. 30 min vehicle pretreatment is followed by cumulative additions of rising doses of NKA (3 nM - 1 µM final concentration, 5 min intervals between additions). The final NKA response is followed by a 15 min wash and recovery period. 30 min pretreatment with a test compound or its vehicle is followed by cumulative additions of rising doses of NKA (3 nM - 10 µM final concentration if necessary, 5 minutes intervals between additions). The final NKA response is followed by a 1 mM carbachol challenge to obtain a maximal tension response in each tissue.

Tissue responses to NKA are recorded as positive pen displacements over baseline and converted to grams tension by comparison to standard weights. Responses are normalized as a % of the maximal tissue tension. ED₅₀'s are calculated for NKA from the control and treated NKA dose responses and compared. Test compounds resulting in an agonist dose ratio ≥ 2 at a screening concentration of 1 µM (i.e. pA_{2 ≥} = 6.0) are considered actives. Further dose response data is obtained for actives so that an apparent pA₂ estimate can be calculated. pA₂ is calculated either by estimation of Kᵢ as described by Furchgott (where pA₂ = - Log Kᵢ, R.F. Furchgott, *Pharm. Rev.* 7 [1995] 183) or by Shild Plot Analysis (O. Arunlakshana & H.O. Shild, *Br. J. Pharmacol.* 14[1959] 48) if the data is sufficient.

### Effect of NK₁ Antagonists on Substance P-Induced Airway Microvascular Leakage in Guinea Pigs

Studies are performed on male Hartley guinea pigs ranging in weight from 400-650 g. The animals are given food and water *ad libitum.* The animals are anesthetized by intraperitoneal injection of dialurethane (containing 0.1 g/ml diallylbarbituric acid, 0.4 g/ml ethylurea and 0.4 g/ml urethane). The trachea is cannulated just below the larynx and the animals are ventilated (V_{T} = 4 ml, f = 45 breaths/min) with a Harvard rodent respirator. The jugular vein is cannulated for the injection of drugs.

The Evans blue dye technique (Danko, G. et al., Pharmacol. Commun., 1, 203-209, 1992) is used to measure airway microvascular leakage (AML). Evans blue (30 mg/kg) is injected intravenously, followed 1 min later by i.v. injection of substance P (10 µg/kg). Five min later, the thorax is opended and a blunt-ended 13-guage needle passed into the aorta. An incision is made in the right atrium and blood is expelled by flushing 100 ml of saline through the aortic catheter. The lungs and trachea are removed en-bloc and the trachea and bronchi are then blotted dry with filter paper and weighed. Evans blue is extracted by incubation of the tissue at 37°C for 18 hr in 2 ml of formamide in stoppered tubes. The absorbance of the formamide extracts of dye is measured at 620 nm. The amount of dye is calculated by interpolation from a standard curve of Evans blue in the range 0.5-10 µg/ml in formamide. The dye concentration is expressed as ng dye per mg tissue wet weight. Test compounds were suspended in cyclodextran vehicle and given i.v. 5 min before substance P.

### Measurement of NK₂ Activity In Vivo

Male Hartley guinea pigs (400-500 gm) with ad lib. access to food and water are anesthetized with an intraperitoneal injection of 0.9 ml/kg dialurethane (containing 0.1 g/m diallylbarbituric acid, 0.4 g/ml ethylurea and 0.4 g/ml urethane). After induction of a surgical plane of anesthesia, tracheal, esophageal and jugular venous cannulae are implanted to facilitate mechanical respiration, measurement of esophageal pressure and administration of drugs, respectively.

The guinea pigs are placed inside a whole body plethysmograph and the catheters connected to outlet ports in the plethysmograph wall. Airflow is measured using a differential pressure transducer (Validyne, Northridge CA, model MP45-1, range ± 2 cmH₂O) which measures the pressure across a wire mesh screen that covers a 1 inch hole in the wall of the plethysmograph. The airflow signal is electrically integrated to a signal proportional to volume. Transpulmonary pressure is measured as the pressure difference between the trachea and the esophagus using a differential pressure transducer (Validyne, Northridge, CA, model MP45-1, range ± 20 cm H₂O). The volume, airflow and transpulmonary pressure signals are monitored by means of a pulmonary analysis computer (Buxco Electronics, Sharon, CT, model 6) and used for the derivation of pulmonary resistance (R_{L}) and dynamic lung compliance (C_{Dyn}).

### Bronchoconstriction Due to NKA

Increasing iv doses of NKA are administered at half log (0.01-3 µg/kg) intervals allowing recovery to baseline pulmonary mechanics between each dose. Peak bronchoconstriction occurs within 30 seconds after each dose of agonist. The dose response is stopped when C_{Dyn} is reduced 80-90% from baseline. One dose-response to NKA is performed in each animal. Test compounds are suspended in cyclodextran vehicle and given i.v. 5 min before the initiation of the NKA dose response.

For each animal, dose response curves to NKA are constructed by plotting the percent increase in R_{L} or decrease in C_{Dyn} against log dose of agonist. The doses of NKA that increased R_{L} by 100% (R_{L}100) or decreased C_{Dyn} by 40% (C_{Dyn}40) from baseline values are obtained by log-linear interpolation of the dose response curves.

### Neurokinin Receptor Binding Assay(s)

Chinese Hamster ovary (CHO) cells transfected with the coding regions for the human neurokinin 1 (NK₁) of the human neurokinin 2 (NK₂) receptors are grown in Dulbecco's minimal essential medium supplemented with 10% fetal calf serum, 0.1 mM non-essential amino acids, 2 mM glutamine, 100units/ml of penicillin and streptomycin, and 0.8 mg of G418/ml at 37°C in a humidified atmosphere containing 5% CO₂.

Cells are detached from T-175 flasks with a sterile solution containing 5mM EDTA in phosphate buffered saline. Cells are harvested by centrifugation and washed in RPMI media at 40°C for 5 minutes. The pellet is resuspended inTris-HCI (pH7.4) containing 1 uM phsphoramidon and 4 ug/ml of chymostatin at a cell density of 30 x 10⁶ cells/ml. The suspension is then homogenized in a Brinkman Polytron (setting 5) for 30-45 seconds. The homogenate is centrifuged at 800 x g for 5 min at 4°C to collect unbroken cells and nuclei. The supernatant is centrifuged in a Sorvall RC5C at 19,000 rpm (44,00 x g) for 30 min at 4°C. The pellet is resuspended, an aliquot is removed for a protein determination (BCA) and washed again. The resulting pellet is stored at -80°C.

To assay receptor binding, 50 µl of [³H]-Substance P (9-Sar, 11-Met [02]) (specific activity 41 Ci/mmol) (Dupont-NEN) (0.8 nM for the NK-1 assay) or [³H]-Neurokinin A (specific activity 114 Ci/ mmole) (Zenca) (1.0 nM for the NK-2 assay) is added to tubes containing buffer (50 mM Tris-HCI (pH 7.4) with 1 mM MnCl₂ and 0.2% Bovine Serum Albumin) and either DMSO or test compound. Binding is initiated by the addition of 100µl of membrane (10-20 µg) containing the human NK-1 or NK-2 receptor in a final volume of 200 µl. After 40 minutes at room temperature, the reaction is stopped by rapid filtration onto Whatman GF/C filters which have been presoaked in 0.3% polyethylenimine. Filters are washed 2 times with 3 ml of 50 mM Tris-HCl (pH7.4). Filters are added to 6 mls of Ready-Safe liquid scintillation cocktail and quantified by liquid scintillation spectrometry in a LKB 1219 RackBeta counter. Non-specific binding is determined by the addition of either 1 µM of CP-99994 (NK₁) or 1µM SR-48968 (NK₂) (both synthesized by the chemistry department of Schering-Plough Research Institute). IC₅₀ values are determined from competition binding curves and Ki values are determined according to Cheng and Prusoff using the experimentally determined value of 0.8 nM for the NK₁ receptor and 2.4 nM for the NK₂ receptor.

For all of the compounds of the invention, the NK₁ binding is in a range of about 0-100 % inhibition at 1 µM concentration. For all of the compounds of the invention, the NK₂ binding is in a range of about 0-100 % inhibition at 1 µM concentration. It should be understood that while the NK binding for certain compounds of the invention is as low as 0% at 1 µM concentration, that at higher concentrations these compounds may have NK binding inhibition activity.

The Kᵢ of a compound is that concentration at which the compound caused 50% inhibition of either NK₁ or NK₂. For those compounds of the invention having higher than 50% inhibition of NK₁ , Kᵢ's for NK₁ were determined. The Kᵢ's for NK₁ for such compounds fell within a range of about 0.1 nM to about 1 µM.

For those compounds of the invention having higher than 50% inhibition of NK₂ , Kᵢ's for NK₂ were determined. The Kᵢ's for NK₂ for such compounds fell within a range of about 0.1 nM to about 1 µM.

Compounds of formula I exhibit NK₁ and NK₂ antagonist activity to varying degrees, i.e., certain compounds have strong NK₁ antagonist activity, but weaker NK₂ antagonist activity. Others are strong NK₂ antagonists, but weaker NK₁ antagonists. While compounds with approximate equipotency are preferred, it is also within the scope of this invention to use compounds of with unequal NK₁/NK₂ antagonist activity when clinically appropriate.

Certain compounds of formula I have been found to be antagonists of both NK₁ and NK₂ receptors, and are therefore useful in treating conditions caused or aggravated by the activity of NK₁ and NK₂ receptors.

The present invention also relates to a pharmaceutical composition comprising a compound of formula I and a pharmaceutically acceptable carrier. Compounds of this invention can be administered in conventional oral dosage forms such as capsules, tablets, powders, cachets, suspensions or solutions, or in injectabte dosage forms such as solutions, suspensions, or powders for reconstitution. The pharmaceutical compositions can be prepared with conventional excipients and additives, using well known formulation techniques. Pharmaceutically acceptable excipients and additives include nontoxic and chemically compatible fillers, binders, disintegrants, buffers, preservatives, anti-oxidants, lubricants, flavorings, thickeners, coloring agents, emulsifiers and the like.

The daily dose of a compound of formula I for treating asthma, cough, bronchospasm, inflammatory disease, migraine, nociception and gastrointestinal disorders is about 0.1 mg to about 20 mg/kg of body weight per day, preferably about 0.5 to about 15 mg/kg, more preferably 0.5 to about 5 mg/kg. For an average body weight of 70 kg, the dosage range is therefore from about 1 to about 1500 mg of drug per day, preferably about 50 to about 100 mg , given in a single dose or 2-4 divided doses. The exact dose , however is determined by the attending clinician , and is dependent on the potency of the compound administered, the age, weight, condition and response of the patient.

The invention disclosed herein is examplified by the following examples, which should not be construed to limit the scope of the disclosure. Alternative mechanistic pathways and analogous structures within the scope of the invention will be apparent to those skilled in the art.

### EXAMPLE 1

### Preparation of (+/-)-1-[[3,5-bis(trifluoromethyl)phenyl]methyl)-3-phenylpiperazine, dihydrochloride salt, quarter hydrate.

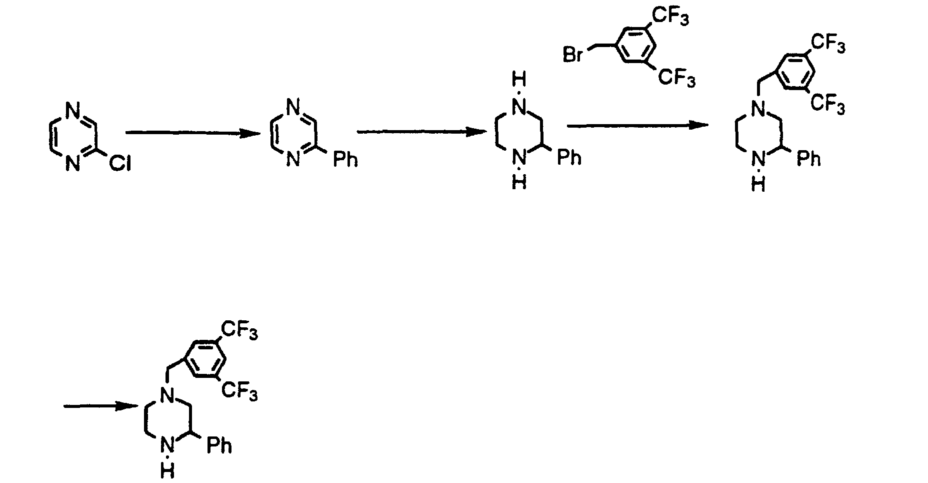

Chloropyrazine (20.68 gram, 177 mmol) and [1,2-bis(diphenylphosphino)ethane]nickel(II) chloride (41.08 gram, 77.8 mmol) in dry THF (1.5 liter) were mixed and stirred for 80 minutes in a flask (cooled with a water bath) under nitrogen. A solution of phenylmagnesium bromide (3M in Et₂O) (103ml, 309mmol) was added slowly through a dropping funnel into the cooled brick-red slurry at room temperature under nitrogen over 3.5 hours. After stirring at room temperature overnight, TLC showed that the reaction was complete. 3 N HCI (100 ml) was added slowly through a dropping funnel under nitrogen and the mixture was stirred for one hour. The THF layer was separated from the aqueous layer. The aqueous layer was adjusted to pH 12 with 6 N NaOH and extracted with EtOAc (100 ml, 3x). The organic fractions (THF and EtOAc) were combined and dried over MgSO₄, filtered and concentrated to give a solid. The product was purified by flash chromatography on 300 g of flash grade silica gel in 2.5 % EtOAc/CH₂Cl₂ to give 10.79 gram (69 mmol, 39%) of 2-phenylpyrazine, m.p. 69-70 °C; FAB mass [M+1]⁺ 157;
Found, C, 76.55; H, 5.22; N, 17.71. Calcd. for C₁₀H₈N₂, C, 76.90; H, 5.16; N, 19.93.

To a solution of 2-phenylpyrazine (11.64 gram, 74.53 mmol) in acetic acid (58.2 ml) was added palladium acetate Pd(OAc)₂ (2.33 gram, 9.94 mmol). The mixture was hydrogenated at 50 psi for four hours. After the reaction was complete, the catalyst was filtered off and rinsed with a small portion of acetic acid. The filtrate was concentrated under house vacuum to give a brown-black solid which was suspended in deionized water (300 ml) and adjusted to pH 13 with 20 % NaOH solution. The product was extracted from aqueous solution with EtOAc (200 ml, 3x), dried over MgSO₄, filtered and evaporated to dryness to give 2-phenylpiperazine (7.2 gram). An additional 1.6 g of 2-phenylpiperazine was obtained by evaporating the aqueous fraction to a solid and triturating the solid with CH₂Cl₂. Total yield of 2-phenyl-piperazine was 73 %. The crude material was crystallized from EtOAc and hexane for characterization, m.p. 86-88 °C; FAB mass [M+1]⁺ 163;
Found, C, 74.04; H, 8.66; N, 17.15. Calcd. for C₁₀H₁₄N₂, C, 74.04 ; H, 8.69; N, 17.26.

To a solution of 2-phenylpiperazine (4.0 gram, 24.65 mmol) in dry CH₂Cl₂ (200 ml) at -78 °C under nitrogen was added Et₃N (5.15 ml, 36.97 mmol) followed by the dropwise addition of a CH₂Cl₂ solution(46.60 ml) of bis(trifluoromethyl)benzyl bromide ( 4.66 ml, 24.65 mmol). The flask was kept at -78 °C then it was gradually warmed to room temperature overnight. After TLC showed that the reaction was complete, the material was washed with brine (150 ml, 2x), dried over MgSO₄, filtered, and evaporated under vacuum to yield a tan solid. The crude product was purified by flash silica gel chromatography (150 g), eluting with 2.5 % MeOH/CH₂Cl₂ to give (+,-) 1-[[3,5-bis(trifluoromethyl)phenyl]methyl]-3-phenyl-piperazine (6.96 gram, 17.92 mmol, 72.7%) as an oil. A portion of this oil (0.5 gram, 1.287 mmol)) was converted to its hydrochloride salt by dissolving the oil in CH₂Cl₂ (20 ml) and treating with 2.3 M HCl-EtOH ( 1.3 ml, 2.99 mmol). After stirring at room temperature for 10 minutes, all solvents were removed under high vacuum and the residue was dried overnight, m.p. 229-233 °C; FAB mass [M+1]⁺ 389;
Found, C, 48.83; H, 4.28; N, 5.87; Cl, 14.77; F, 24.03. Calcd. for C₁₉H₁₈N₂F₆•2 HCl•0.25 H₂O, C, 48.99; H, 4.43; N, 6.01; Cl, 15.22; F, 24.47.

### EXAMPLE 2 (Comparative)

### Preparation of (+,-)-4-[[3,5-bis(trifluoromethyl)phenyl]methyl]-2-phenyl-1-[[[1-(phenylmethyl)-4-piperidinyl]amino]acetyl]piperazine, trihydrochloride salt, dihydrate.

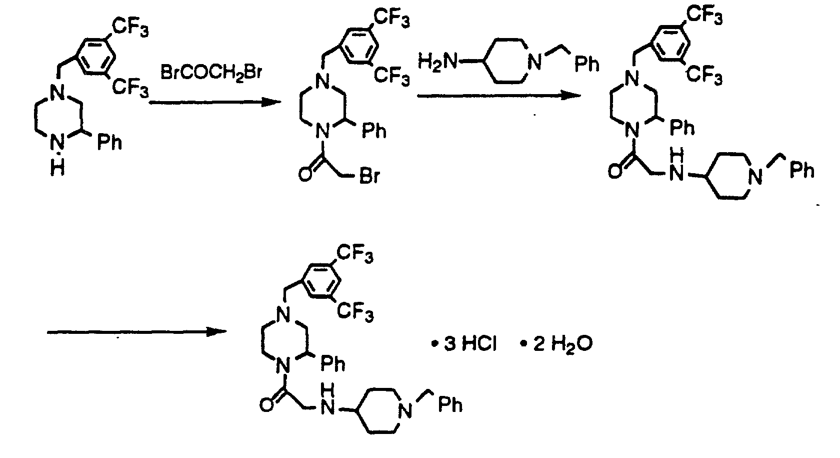

To a solution of (+,-) 1-[[3,5-bis(trifluoromethyl)phenyl]methyl]-3-phenyl-piperazine (0.76 gram, 1.975 mmol) in dry CH₂Cl₂ (15.2 ml) at -78 °C was added Et₃N (0.286 ml, 2.055 mmol) followed by the dropwise addition of bromoacetyl bromide (0.179 ml, 2.055 mmol). After stirring at -78 °C for 4 hours, the reaction was diluted with CH₂ Cl₂ (200 ml), washed with brine (100 ml, 2x) and dried over MgSO₄. After filtration, the solvent was removed to give a light yellow solid which was used without further purification. FAB mass [M+1]⁺ 509.2 (79 Br).

The product from the previous reaction (1.067 gram, 2.096 mmol) was dissolved in dry CH₂Cl₂ (10.67 ml) and cooled to -78 °C under nitrogen. To this cooled solution were added 4-amino-1-benzylpiperidine (0.44 ml, 2.11 mmol) and diisopropylethylamine (0.402 ml, 2.3 mmol). The reaction was gradually warmed to room temperature overnight under nitrogen. After completion, CH₂Cl₂ (300 ml) was added and the organic layer was washed with brine (100 ml, 2x), dried over MgSO₄ and filtered. The filtrate was evaporated under vacuum to give a crude oil which was purified by flash chromatography on flash grade silica gel (100 g), eluting with 2.5% NH₃-MeOH-2.5% EtOH/CH₂Cl₂ to give a light yellow oil (0.76 g, 1,229 mmol, 59%). A portion of the oil (0.27 gram, 0.436 mmol) was converted to its hydrochloride salt by dissolving in CH₂Cl₂ (13.5 ml) and treating with 2.3 M HCl-EtOH (0.938 ml, 2.182 mmol). After stirring at room temperature for 40 minutes, solvent was evaporated and the residue was vacuum dried overnight, m.p. 199-202 °C; FAB mass [M+1]⁺ 619.5;
Found, C, 51.73; H, 5.98; N, 7.18; Cl, 13.69; F, 14.75. Calcd. for C₃₃H₃₆N₄OF₆ • 3 HCl • 2 H₂O, C, 51.87 H, 5.67; N, 7.33; Cl, 13.92; F, 14.91.

### EXAMPLE 3 (Comparative)

By a process analogous to that described in Example 2, the following compound was prepared by using (1S,4S,)-benzyl-2,5-diazobicyclo[2.2.1] heptane dihydrobromide in place of 4-amino-1-benzylpiperidine .

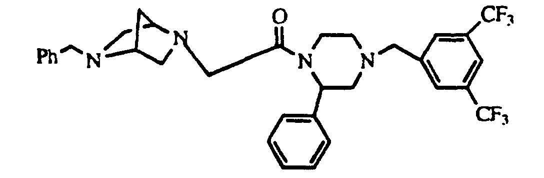

| | | | |
|---|---|---|---|
| free form | m.p. 45-47 °C | High Res.Mass | Cal'd 617.2715 Found 617.2731 |

### EXAMPLE 4 (comparative)

### Preparation of (+,-)-4-[[3,5-bis(trifluoromethyl)phenyl]methyl]-2-phenyl-N-[1-(phenylmethyl)-4-piperidinyl]-1-piperazineethanamine, tetrahydrochloride salt, monohydrate.

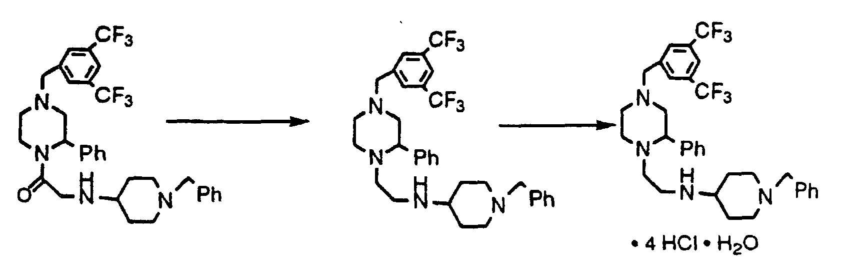

To a solution of (+,-)-4-[[3,5-bis(trifluoromethyl)phenyl]methyl]-2-phenyl-1-[[[1-(phenylmethyl)-4-piperidinyl]amino]acetyl]piperazine (0.48g, 0.776 mmol) in THF (12 ml) was added 10 M BH₃ • S(CH₃)₂ (0.388 ml, 3.88 mmol). The mixture was heated in an oil bath at 80 °C under nitrogen overnight. After completion, excess BH₃ was decomposed by dropwise addition of MeOH to the cooled solution under nitrogen. MeOH was evaporated and the residue was redissolved in EtOH (14.4 ml). K₂CO₃ (0.235 gram, 1.707 mmol) was added and the mixture was refluxed at 80 °C for five hours. After TLC showed that the reaction was complete, the solid was filtered off and the filtrate was evaporated under vacuum. The residue was redissolved in EtOAc (300 ml), washed with brine (100 ml) and dried over MgSO₄. It was filtered and evaporated under vacuum to give an oil which was purified by flash chromatography on flash grade silica gel (80 g), eluting with 3% NH₃-MeOH/CH₂Cl₂ to give the desired material as an oil (0.373 gram, 0.615 mmol, 79%). A portion of the oil (0.36 gram) was converted to its hydrochloride salt by dissolving in dry CH₂Cl₂ (18 ml), followed by the addition of 2.3 M HCl-EtOH (1.3 ml). Solvents were removed after stirring at room temperature for 0.5 hour and the residue was vacuum dried, m.p. 238-241 °C; FAB mass [M+1]⁺ 605.6;
Found, C, 51.96; H, 5.83; N, 7.01; Cl, 14.52; F, 18.21. Calcd. for
C₃₃ H₃₈N₄F₆ • 4 HCl • H₂O, C, 51.57; H, 5.77; N, 7.29, Cl, 14.83; F, 18.45.

### EXAMPLE 5

### Preparation of 2-(3,4-dichlorophenyl)piperazine

### METHOD 1

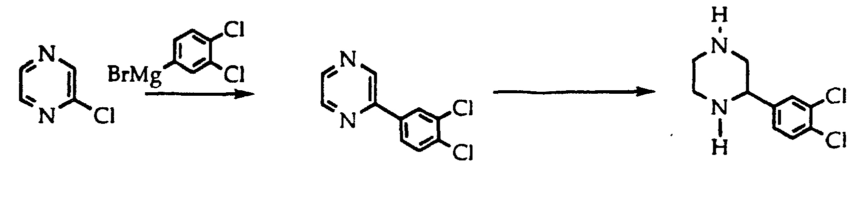

2-(3,4-dichlorophenyl)pyrazine was prepared according to the analogous method described in Example 1. m.p. 118-119 °C; FAB mass [M+1]⁺³⁵ Cl 225.

To a solution of 2-(3,4-dichlorophenyl)pyrazine (10g, 44.43 mmol) in dry THF (150 ml) was added slowly a solution of DIBAL-H (1M in THF, 444.3 ml) through a dropping funnel at 10 °C under N₂. The color of solution turned into red wine at the end of addition. The solution was gradually warmed up to room temperature overnight. After completion (checked by TLC) the reaction was quenched slowly by the addition of saturated Na₂SO₄ solution until no more H2 evolved. White precipate was formed after stirring for 1.0 h. The precipitate was filtered off, rinsed with THF, dried over MgSO₄ and evaporated to dryness. The crude material (10 g) was purified by flash chromatography on 300 g of flash grade silica gel in 7.5 % NH₃₋MeOH/CH₂Cl₂ to give 4.11g (17.77 mmol, 40%) of 2-(3,4-dichloro-phenyl)piperazine. m.p. 74-76°C; FAB mass [M+1]⁺ 35 Cl 231.

### METHOD 2.

2-(3,4-dichlorophenyl)pyrazine was also synthesized according to the method published in J.Med.Chem. 9,191,1966.

General method for the synthesis of 2-aryl-piperazine derivatives.

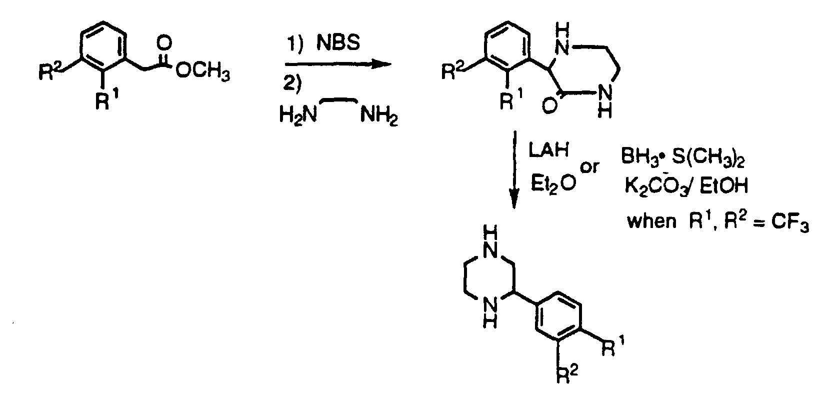

R¹ = Cl, H or other substituents i.e. OCH₃, CF₃, Br, I, F, etc.

R² = Cl, H or other substituents i.e. OCH₃, CF₃, Br, I, F, etc.

### Resolution of 2-(3,4-dichlorophenyl)-piperazine

### Step 1

Treat a solution of piperazine (10.6 g, 45.8 mmol) in methanol (130 mL) with two equivalents of N-acetyl-L-leucine (15.9 g, 91.5 mmol) and heat until all of the material dissolves. Add EtOAc (660 mL) to this solution and let sit at ambient temperature overnight. Decant the solvent phase from the precipitated salt and concentrate in vacuo to obtain 18.5 g of salt with the piperazine enriched 3.0:1.0 of enantiomer A to B. Concentrate the precipitated salt to give 12.3 g of a colorless solid with the piperazine enriched 1.0:7.1 of enantiomer A to B.

### Step 2

Dissolve the precipitated salt from step 1 in 0.5 N NaOH (400 mL) and extract with CH₂Cl₂ (4 x 150 mL). Combined organic layers, dry (MgSO₄) and concentrate to give 3.8 g piperazine free base. Recrystallize the free base two times in hexane (100 and 70 mL) to give 2.5 g of piperazine (98% ee of enantiomer B) as colorless crystals.

### Step 3

Recrystallize the salt from the solvent phase in step 1 with 350 mL of EtOAc:methanol (5:1) to give 15.3 g of salt from the solvent phase. Recrystallized this salt with 250 mL of EtOAc:methanol (5:1) to give 9.9 g of salt from the solvent phase that is 5.5:1.0 enriched with piperazine A to B. Add the salt to 0.5 N NaOH (250 mL) and extract with CH₂Cl₂ (3 x 100 mL). Combine organic layers, dry (MgSO₄) and concentrate to give 2.8 g of crude free base. Recrystallize from hexane (65 mL) to give 1.0 g of piperazine (98% ee of enantiomer A).

Analytical procedure for measuring piperazine enantiomeric purity.

The enantiomeric purity of the piperazine is measured by chiral HPLC analysis of the di-tert-butoxycarbonyl piperazine derivative. The di-tert-butoxycarbonyl derivative is prepared by adding a small piperazine sample (free base or salt)(∼ .2 mg) to di-tert-butyl dicarbonate (∼ 1 mg) and methanol (0.5 mL) and heating at 80°C for 1 hour. If the piperazine sample is a salt, triethylamine (20 µL) is also added. The derivative is analyzed by HPLC using a ChiralPak AD column eluting with 95:5 hexane-isopropyl alcohol.

### EXAMPLE 6 (Comparative)

### Preparation of (+,-)-4-[[3,5-bis(trifluoromethyl)phenyl]methyl]-2-(3,4-dichlorophenyl)-1-[[[1-(phenylmethyl)-4-piperidinyl]amino]acetyl]-piperazine.

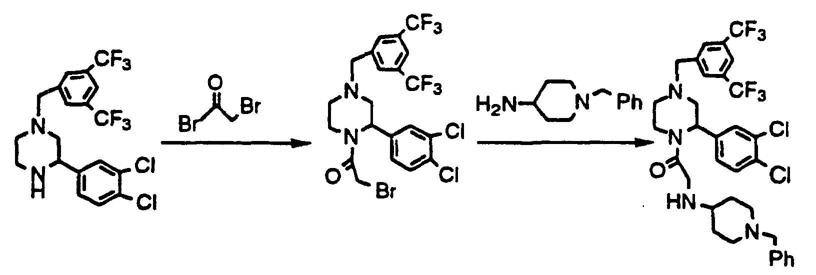

By a procedure analogous to the procedure described in the first part of Example 2, using (+,-)-1-[[3,5-bis(trifluoromethyl)phenyl]-methyl]-3-(3,4-dichlorophenyl)-piperazine (Example 5) in place of (+,-)-1-[[3,5-bis(trifluoromethyl)-phenyl]methyl]-3-phenyl-piperazine, the bromoacetyl derivative was obtained in 78% from its starting material as a solid, m.p. 146-148 °C; FAB mass [M+1]^{+ 35}Cl 577. 579.

By a procedure analogous to the procedure described in the second part of Example 2, the title compound was obtained as a solid (48%) after purification by flash chromatography on flash grade silica gel (70g ), eluting with 4% MeOH/CH₂Cl₂, m.p. 53-55 °C; FAB mass [M+1]⁺³⁵Cl 687;
Found, C, 56.98; H, 4.72; N, 8.13; Cl, 10.67; F, 16.30. Calcd. for C₃₃H₃₄N₄Cl₂F₆O • 0.25 H₂O, C, 57.27; H, 5.03; N, 8.10; Cl, 10.25; F, 16.47.

### EXAMPLE 7 (comparative)

By a procedure analogous to the procedure described in Example 2, but using (+,-)-1-[[3,5-bis(trifluoromethyl)phenyl]methyl]-3-(3,4-dichlorophenyl)-piperazine in place of (+,-)-1-[[3,5-bis(trifluoromethyl)phenyl]methyl]-3-phenyl-piperazine, and employing (1S,4S)-2-benzyl-2,5-diazabicyclo[2.2.1]heptane dihydrobromide, in place of 4-amino-1-benzylpiperidine, the following compound was prepared. 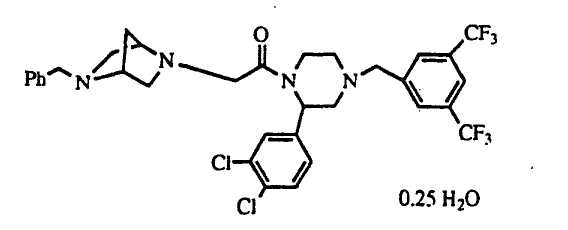 m.p. 55-57^{°C} ; [M+1]⁺
FAB mass 685
based on ³⁵Cl

### EXAMPLE 8

### Preparation of (+,-)-4-[[3,5-bis[trifluoromethyl)phenyl]acetyl]-2-phenyl-1-[[[1-(phenylmethyl)-4-piperidinyl]amino]acetyl]piperazine.

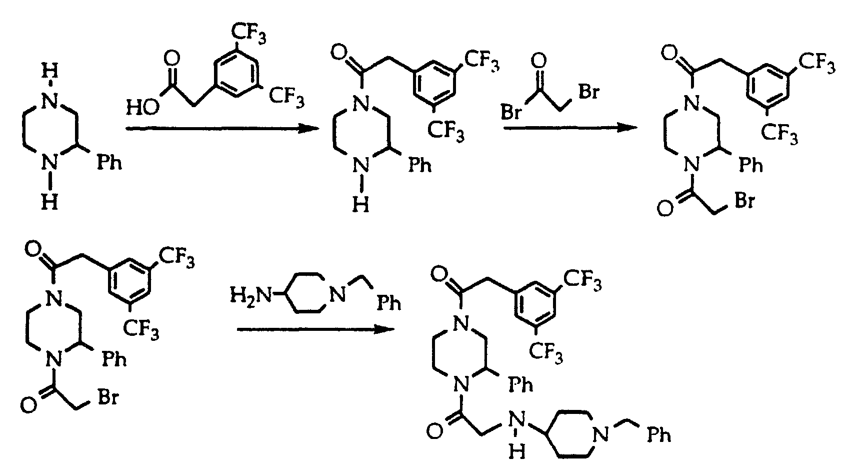

To cooled CH₂Cl₂ (127ml) containing 2-phenyl-piperazine (Example 1, 1.0 gram, 6.164 mmol), 3,5-bis(trifluoromethyl)phenylacetic acid (1.797g, 6.472 mmol), and N-hydroxybenzotriazole monohydrate (0.874g, 6.472 mmol) at -20 °C were added Et₃N (0.9ml, 6.472 mmol) and N,N-dimethylaminopropylethylcarbodimide (DEC) under nitrogen. The reaction was kept at -20 °C for an hour and gradually warmed to RT overnight. After stirring 20 hours, the reaction was complete and CH₂Cl₂ (200 ml) was added. The organic solution was washed with 5% NaHCO₃ (100 ml) and brine (100 ml, 3x), dried over MgSO₄, filtered and concentrated under vacuum to give 2.5 g of crude product. The product was purified by flash chromatography on flash grade silica gel (120 g), eluting with 3% NH₃-MeOH/CH₂Cl₂ to give a gummy solid (2.08g, 4.996 mmol, 81%). A portion of this solid (1.0 g) was crystallized from hexane and characterized to yield a solid, m.p. 80-82 °C; FAB mass [M+1]⁺ 417.2;

Calcd. for C₂₀H₁₈ON₂F₆, C, 57.69; H, 4.36; N, 6.73; F, 27.38.

Found, C, 57.91; H, 4.55; N, 6.69; F, 27.61.

To a solution of the above compound (1.11g, 2.642 mmol) in dry CH₂Cl₂ (22.2 ml) at -78 °C was added diisopropylethylamine (0.483 ml, 2.774 mmol) followed by the dropwise addition of bromoacetyl bromide (0.246 ml, 2.774 mmol). After stirring at -78 °C for 7 hours under nitrogen, additional diisopropylethylamine (0.51 ml, 2.9 mmol) and 4-amino-1-benzylpiperidine (0.605 ml, 2.9 mmol) were added at -78 °C. The reaction was gradually warmed to RT overnight. After the reaction was complete, the reaction was diluted with CH₂Cl₂ (150 ml), washed with brine (50 ml, 3x) and dried over MgSO₄. After filtration, the solvent was removed under vacuum to give a light yellow solid which was purified by flash chromatography on flash grade silica gel (150 g), eluting with 5% NH₃-MeOH/CH₂Cl₂ to give the title compound as a white solid (0.94,g, 1.453 mmol, 55 %), m.p.49-52 °C; FAB mass [M+1]⁺ 647.3;

Calcd. for C₃₄H₃₆O₂N₄F₆, C, 63.15; H, 5.16; N, 8.66; F, 17.62. Found, C, 62.73; H, 5.77; N, 8.56; F, 17.68.

### EXAMPLE 9 (Comparative)

### Preparation of (+,-)-4-[2-[3,5-bis(trifluoromethyl)phenyl]ethyl]-2-phenyl-N-[1-(phenylmethyl)-4-piperidinyl]-1-piperazineethanamine, four hydrochloride salt, hemihydrate.

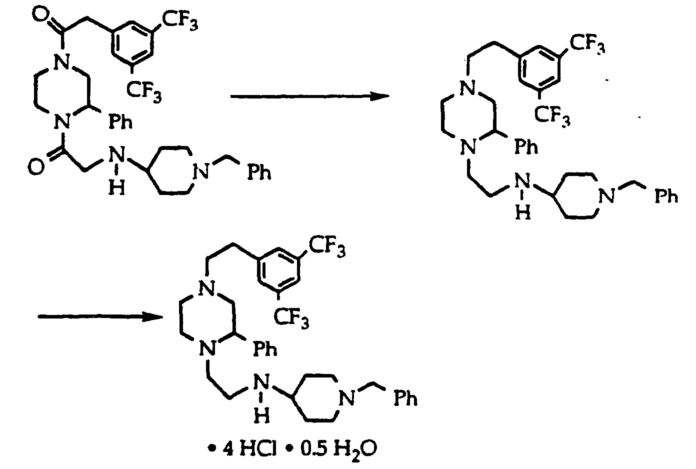

To a solution of the product from Example 16 (0.463 gram, 0.72 mmol) in dry THF (21.6 ml) was added a 10 M solution of BH₃ • S(CH₃)₂ (0.716ml, 7.16 mmol) at RT under nitrogen. The solution was heated at 80 °C under nitrogen for 24 hours. After cooling to RT, MeOH (5 ml) was added slowly to decompose excess BH₃ • S(CH₃)₂. All solvents were removed under vacuum and the residue was redissolved in absolute EtOH (14.1 ml), followed by the addition of K₂CO₃ (0.22g. 1.58 mmol). The mixture was heated at 80 °C for 6.5 hours under nitrogen. After cooling K₂CO₃ was filtered and EtOH was removed to give a residue which was redissolved in EtOAc (150 ml) and washed with brine (50 ml, 2x). It was dried over MgSO₄, filtered, and evaporated under vacuum to give a solid (0.42 g) which was purified by flash chromatography on flash grade silica gel (80 g), eluting with 4% NH₃-MeOH/ CH₂Cl₂ to give a white solid (0.14 g, 0.227 mmol, 49%).

The above material (0.14g, 0.227 mmol) was treated with CH₂Cl₂ (6.8 ml) and 2.3M HCl-EtOH (0.592 ml, 1.362 mmol). After stirring at RT for 10 minutes, the solution was evaporated under high vacuum to give a white solid, m.p. 162-190 °C; High Res. MS [M+1]⁺; Calcd. for C₃₄H₄₁N₄F₆, 619.3235, Found, 619.3222.

Calcd. for C₃₄H₄₀N₄F₆ • 4 HCl • 0.5 H20, C, 52.79; H, 5.86; N, 7.24, F, 14.73; Cl, 18.33. Found, C, 52.58; H, 6.10; N, 7.21; F, 14.77; Cl, 16.71.

### EXAMPLE 10

### Preparation of (+,-)-2-phenyl-1-[[[(1-phenylmethyl)-4-piperidinyl]amino]acetyl]-4-[(3,4,5-trimethoxylphenyl)acetyl]piperazine, hemihydrate.

By a procedure analogous to the procedure described in Example 8, using 3,4,5-trimethoxyphenylacetic acid in place of 3,5-bis(trifluoromethyl)phenylacetic acid, the title compound was prepared as a solid, m.p. 53-56 °C, High Res. MS:[M+1]+ Calcd. for C35H45N405
601.3390; Found, 601.3393.

Calcd. for C₃₅H₄₄N₄O₅ • 0.5 H₂O, C, 68.94; H, 7.43; N, 9.19. Found, C, 69.21; H, 7.53; N, 9.22.

### EXAMPLE 11 (comparative)

### Preparation of (+,-)-2-phenyl-N-[1-(phenylmethyl)-4-piperidinyl]-4-[2-(3,4,5-trimethoxyphenyl)ethyl]-1-piperazineethanamine, four hydrochloride salt, monohydrate.

By essentially the same process as described in Example 9, using the product from Example 10 in place of the product from Example 8, the title compound was prepared as a solid, m.p. 167 °C (wet, no sharp melting point); High Res. MS: [M+1]⁺ Calcd. for C₃₅H₄₉N₄O₃, 573.3805, Found, 573.3810.

Calcd. for C₃₅H₄₈N₄O₃ • 4 HCl • H₂O, C, 57.07; H, 7.39; N, 7.61; Cl, 19.25. Found, C, 57.16; H, 7.88; N, 7.64, Cl, 18.71.

### EXAMPLE 12 (Comparative)

### Preparation of (+/-)-4-[2-[3,5-bis(trifluoromethyl)phenyl)ethyl]-2-phenyl-1-[[[1-(phenylmethyl)-4-piperidinyl]amino]acetyl]piperazine, trihydrochloride salt.

By a process analogous to the reduction process described in the first part of Example 9, using (+,-)-[[3,5-bis(trifluoromethyl)phenyl]acetyl]-3-phenylpiperazine (described in the first part of Ex. 8) as a starting material, (+,-)-4-[2-[bis(trifluoromethyl)-phenyl]ethyl]-2-phenyl piperazine was prepared as a solid after purification by flash chromatography, m.p. 193-195 °C, FAB mass [M+1]⁺ 403.3. This material (0.38g, 0.94 mmol) was converted to its bromoacetyl derivative according to the same procedure as described in the second step of Example 8. After reaction was complete, the material was alkylated with 4-amino-1-benzylpiperidine without isolation, using the same procedure as described in the third step of Example 16. The title compound was obtained as a solid by flash chromatography, then converted to its HCI salt by treatment with HCI/MeOH solution. m.p. 214-216 °C, High Res. MS: [M+1]⁺ Calcd. for C₃₄H₃₉N₄OF₆, 633.3028; Found, 633.3034.

### EXAMPLE 13 (comparative)

### Preparation of (+,-)-2-phenyl-1-[[[(1-phenylmethyl)-4-piperidinyl]amino]acetyl]-4-[2-(3,4,5-trimethoxyphenyl)ethyl]piperazine.

By a reduction process analogous to that described in the first part of Example 9, using (+,-)-3-phenyl-1-[(3,4,5-trimethoxyphenyl)acetyl]piperazine as a starting material, the reduced product, (+,-)-2-phenyl-4-[[2-(3,4,5-trimethoxy)phenyl]-ethyl]piperazine was prepared as a solid after purification by flash chromatography, m.p. 160-162°C, FAB mass [M+1]⁺ 357.4. This material (0.53g, 1.48 mmol) was converted to its bromoacetyl derivative according to the same procedure as described in the second step of Example 8. After reaction is complete, the bromoacetyl derivative is alkylated in situ with 4-amino-1-benzylpiperidine, using the same procedure as described in the third step of Example 8.

The title compound can be obtained and purified by flash chromatography.

### EXAMPLE 14

### Preparation of (+,-)-[3,5-bis(trifluoromethyl)benzoyl]-3-(3,4-dichlorophenyl)-piperazine

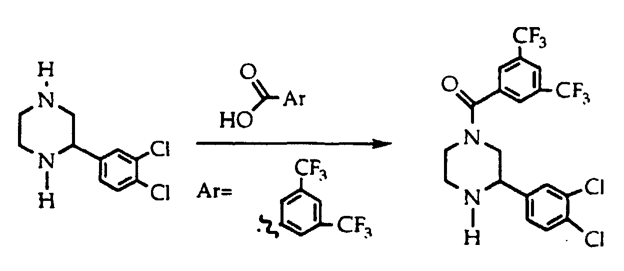

To a cooled solution of CH₂Cl₂ (103 ml) containing 2-(3,4-dichlorophenyl)piperazine (1.15 gram, 5.0 mmol), 3,5-bis(trifluoromethyl)benzoic acid (1.34g, 5.09 mmol), and N-hydroxybenzotriazole monohydrate (0.688g, 5.09mmol) at -20 °C were added Et₃N (0.711ml, 5.09 mmol) and N,N-dimethylaminopropylethylcarbodimide (DEC) (0.967g, 5.09 mmol) under nitrogen. The reaction was kept at -20 °C for an hour and gradually warmed to RT overnight. After stirring 20 hours, the reaction was complete and CH₂Cl₂ (200 ml) was added. The organic solution was washed with 5% NaHCO₃ (80 ml) and brine (80 ml, 2x), dried over MgSO₄, filtered and concentrated under vacuum to give 2.1 g of crude product. The product was purified by flash chromatography on flash grade silica gel (120 g), eluting with 2% NH₃-MeOH/CH₂Cl₂ to give a foam solid (1.25g, 2.65 mmol,53%). m.p. 50-53 °C; FAB mass [M+1]^{+ 35} Cl 470.9;
Calcd. for C₁₉H₁₄ON₂F₆Cl₂, C, 48.42; H, 2.99; N, 5.94; F, 24.19; Cl, 15.05.
Found, C, 48.57; H, 2.90; N, 5.94; F, 23.90; Cl, 15.03.

### EXAMPLE 15

### Preparation of (+,-)-4-[3,5-bis(trifluoromethyl)benzoyl]-2-(3,4-dichlorophenyl)-1-[[[1-(phenylmethyl)-4-piperidinyl]amino]acetyl]piperazine

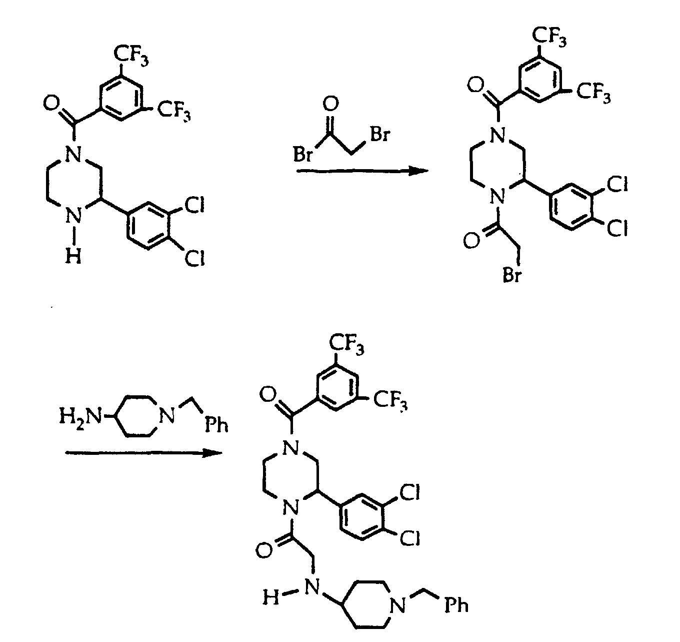

To a solution of (+,-)-[3,5-bis(trifluoromethyl)benzoyl]-3-(3,4-dichlorophenyl)piperazine (0.6 g, 1.274 mmol) in dry CH₂Cl₂ (12.0 ml) at -78 °C was added diisopropylethylamine (0.266ml, 1.53 mmol) followed by the dropwise addition of bromoacetyl bromide (0.124 ml, 1.40 mmol). After stirring at -78 °C for 3.5 hours under nitrogen, additional diisopropylethylamine (0.234 ml, 1.342 mmol) and 4-amino-1-benzylpiperidine (0.279ml, 1.342 mmol) were added at -78 °C. The reaction was gradually warmed to RT overnight. After the reaction was complete, the reaction was diluted with CH₂Cl₂ (200 ml), washed with brine (80 ml, 3x) and dried over MgSO₄. After filtration, the solvent was removed under vacuum to give a light yellow solid which was purified by flash chromatography on flash grade silica gel (150 g), eluting with 5% NH₃-MeOH/CH₂Cl₂ to give the title compound as a white solid (0.55g, 1.274 mmol, 62 %), m.p.66-69 °C; FAB mass [M+1]^{+ 35}Cl 701.

Calcd. for C₃₃H₃₂O₂N₄F₆Cl₂, C, 56.50; H, 4.60; N, 7.99; F, 16.25, Cl, 10.11. Found, C, 56.57; H, 4.66; N, 7.94; F, 16.07, Cl, 9.90.

### EXAMPLE 16

By employing methods analogous to those described in Example 14 and Example 15, using appropriate amino reagents, and using parallel synthesis methods, the following compound was obtained.

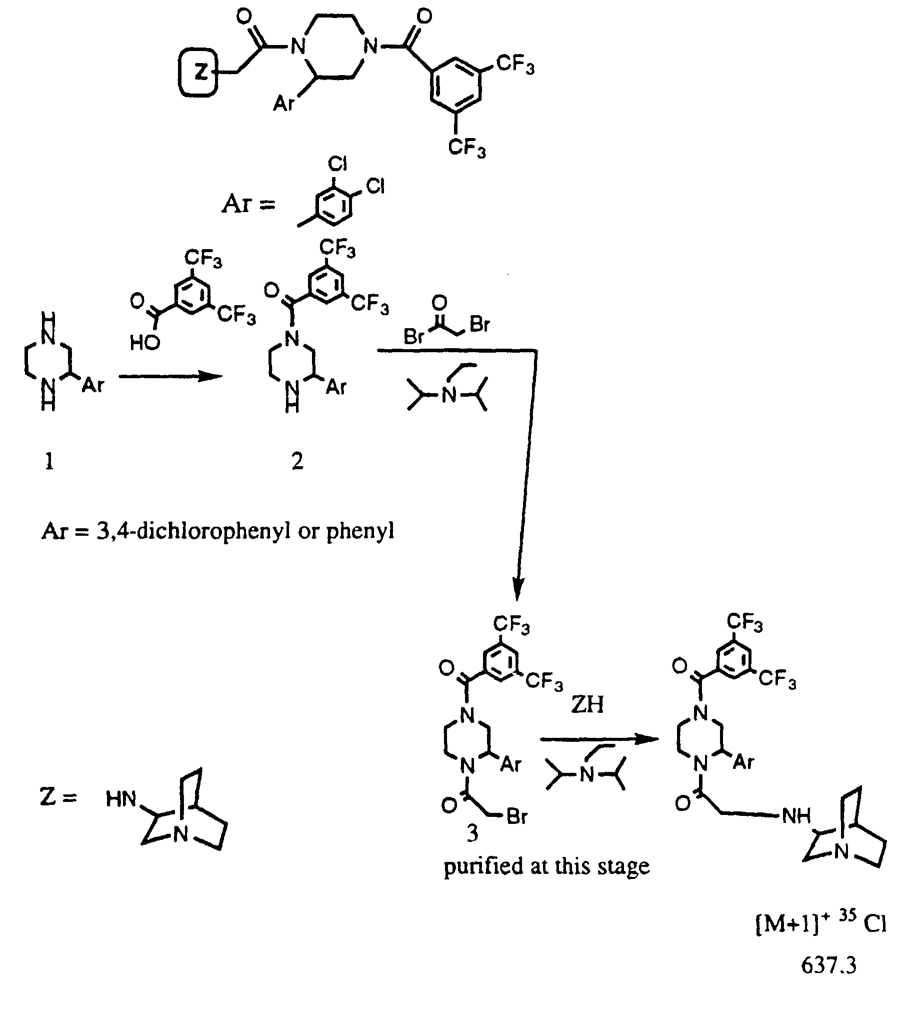

### EXAMPLE 17

### Preparation of (+)-4-[3,5-bis(trifluoromethyl)benzoyl)-2-(3,4-dichlorophenyl)-1-[[[1-(phenylmethyl)-4-piperidinyl]amino]acetyl]piperazine dihydrochloride dihydrate

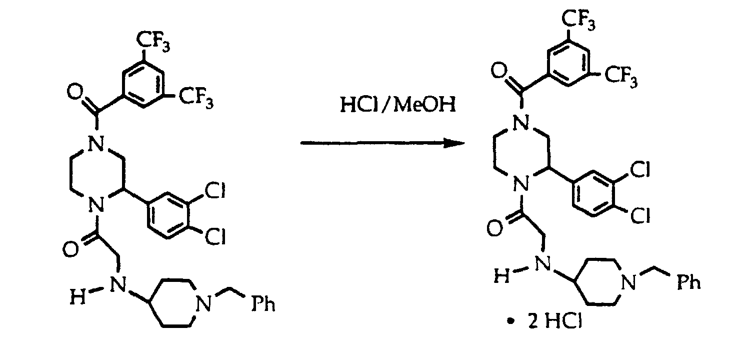

The compound from Example 15 (100 mg) was separated on a Chiral Pak AD column (5x 50 cm), eluting with hexane : isopropanol (80 : 20). The first fraction was evaporated to give 37 mg of enantiomer A which was converted to its hydrochloride salt by dissolving in MeOH and treating with three equivalents of anhydrous HCl/MeOH for 20 minutes. After the solvent was evaporated a white solid was obtained: m.p. 205-220 °C; [α]_{D}^{25.1} = + 21.1(MeOH); FAB mass [M+1)^{+ 35}Cl 701.

Calcd. for C₃₃H₃₂O₂N₄F₆Cl₂ •2 HCl • 2 H₂O, C, 48.90; H, 4.73; N, 6.91; F,14.06, Cl, 17.49. Found: C, 49.34 H, 4.84; N, 6.82. High Resolution Mass: Calc. for [M+1]⁺ C₃₃H₃₃O₂N₄F₆Cl₂ 701.1885, Found: 701.1879.

### EXAMPLE 18

### Preparation of (-)-4-[3,5-bis(trifluoromethyl)benzoyl]-2-(3,4-dichlorophenyl)-1-[[[1-(phenylmethyl)-4-piperidinyl]amino]acetyl]piperazine dihydrochloride dihydrate

The compound from Example 15 (100 mg) was separated on a Chiral Pak AD column (5x 50 cm), eluting with hexane : isopropanol (80 : 20). The second fraction was evaporated to give 45mg of enantiomer B which was converted to its hydrochloride salt by dissolving in MeOH and treating with three equivalents of anhydrous HCl/MeOH for 20 minutes. After the solvent was evaporated a white solid was obtained: m.p. greater than 258 °C; [α]_{D} ^{25.1} = -18.5 (MeOH), FAB mass [M+1]^{+ 35}Cl 701.

Calcd. for C₃₃H₃₂O₂N₄F₆Cl₂•2 HCl•2 H₂O, C, 48.90; H, 4.73; N, 6.91; F,14.06, Cl, 17.49. Found: C, 48.88 H, 4.83; N, 6.71. High Resolution Mass: Calc. for [M+1]⁺ C₃₃H₃₃O₂N₄F₆Cl₂ 701.1885, Found: 701.1885.

### EXAMPLE 19

### Preparation of (+,-)-2-(3,4-dichlorophenyl)-1-[[[1-(phenylmethyl)-4-piperidinyl]amino]acetyl]-4-(3,4,5-trimethoxybenzoyl)piperazine

By employing methods analogous to those described in Example 14 and Example 15, and using 3,4,5-trimethoxybenzoic acid in place of 3,5-bis-(trifluoromethyl)benzoic acid in the coupling reaction, the title compound was obtained in 60% yield, m.p.67-70 °C; FAB mass [M+1]^{+ 35}Cl 655.

Calcd. for C₃₄H₄₀O₅N₄Cl₂, C, 62.29; H, 6.15; N, 8.54; Cl, 10.81. Found, C, 61.87; H, 6.15; N, 8.46; Cl, 10.62.

### EXAMPLE 20

### Preparation of (+,-)-2-(3,4-dichlorophenyl)-4-[3-(1-methylethoxy)benzoyl]-1-[[[1-(phenylmethyl)-4-piperidinyl]amino]acetyl]piperazine, hemihydrate

By employing methods analogous to those described in Example 14 and Example 15, and using 3-(1-methylethoxy)benzoic acid in place of 3,5-bis-(trifluoromethyl)benzoic acid in the coupling reaction, the title compound was obtained in 49.5% yield, m.p.58-61 °C; FAB mass [M+1]^{+ 35}Cl 623.3.

Calcd. for C₃₄H₄₀O₃N₄Cl₂• 0.5 H₂O, C, 64.55; H, 6.53; N, 8.86; Cl, 11.20. Found, C, 64.55; H, 6.64; N, 8.92; Cl, 11.26.

### EXAMPLE 21

### Preparation of (+,-)-2-(3,4-dichlorophenyl)-4-[2-methoxybenzoyl]-1-[[[1-(phenylmethyl)-4-piperidinyl]amino]acetyl]piperazine

By employing methods analogous to those described in Example 14 and Example 15, and using 2-methoxybenzoic acid in place of 3,5-bis-(trifluoromethyl)benzoic acid in the coupling reaction, the title compound was obtained in 42.0% yield, m.p.71-73 °C. FAB mass [M+1]⁺ ³⁵Cl 595.2.

### EXAMPLE 22

### Preparation of (+,-)-4-[3,5-bis(trifluoromethyl)benzoyl]-2-(3,4-dichlorophenyl)-1-[[5-(phenylmethyl)-2,5-diazabicyclo[2.2.1]heptan-2-yl]acetyl]piperazine

By employing methods analogous to those described in Example 14 and Example 15, and using 5-phenylmethyl-2,5-diazabiicyclo[2.2.1.]heptane in place of 1-amino-4-benzylpiperidine as the alkylating reagent, the title compound was obtained in 75% yield, m.p.75-77 °C. FAB mass [M+1]^{+ 35} Cl 699.2;
Calcd. for C₃₃H₃₀O₂N₄F₆Cl₂, C, 56.66; H, 4.32; N, 8.01; Cl, 10.14; F, 16.30.
Found, C, 56.55; H, 4.41; N, 7.95; Cl, 9.93; F, 16.53.

### EXAMPLE 23

### Preparation of (+,-)-4-[[3,5-bis(trifluoromethyl)phenyl]acetyl]-2-phenyl-1-[[[1-(phenylmethyl)-4-piperidinyl]amino]acetyl]piperazine.

By employing methods analogous to those described in Example 14 and Example 15, using 2-phenylpiperazine in place of 2-(3,4-dichloro-phenyl)piperazine and 3,5-bis(trifluoromethyl)phenylacetic acid in place of 3,5-bis(trifluoromethyl)benzoic acid in the coupling reaction, the title compound was obtained in 55 % yield, m.p..49-52 °C; FAB mass [M+1]⁺ 647.3;
Calcd. for C₃₄H₃₆O₂N₄F₆, C, 63.15; H, 5.61; N, 8.66; F, 17.62.
Found, C, 62.73; H, 5.77; N, 8.59; F, 17.68.

### EXAMPLE 24

### Preparation of (+,-)-2-phenyl-1-[[[(1-phenylmethyl)-4-piperidinyl]amino]acetyl]-4-[(3,4,5-trimethoxylphenyl)acetyl]piperazine, hemihydrate.

By employing methods analogous to those described in Example 14 and Example 15, and using 2-phenylpiperazine in place of 2-(3,4-dichlorophenyl)-piperazine and 3,4,5-trimethoxyphenylacetic acid in place of 3,5-bis(trifluoromethyl)benzoic acid, the title compound was prepared as a solid, m.p. 53-56 °C, High Res. MS:[M+1]⁺ Calcd. for C₃₅H₄₅N₄O₅, 601.3390; Found, 601.3393.

Calcd. for C₃₅H₄₄N₄O₅ • 0.5 H20, C, 68.94; H, 7.43; N, 9.19.

Found, C, 69.21; H, 7.53; N, 9.22.

### EXAMPLE 25

### Preparation of (+,-)-4-[3,5-bis(trifluoromethyl)benzoyl]-2-phenyl-1-[[[1-(phenylmethyl)-4-piperidinyl]amino]acetyl]piperazine

By employing methods analogous to those described in Example 14 and Example 15, and using 2-phenylpiperazine in place of 2-(3,4-dichlorophenyl)-piperazine, the title compound was prepared with 71 % yield as a solid: m.p. 65-67 °C, FAB MS [M+1)^{+ 35}Cl 633.4
Calcd. for C₃₃H₃₄N₄O₂F₆• 0.25 H₂O, C, 62.16; H, 5.46; N, 8.80; F, 17.89.
Found, C, 62.00; H, 5.65; N, 8.78; F, 18.08.

### EXAMPLE 26

### Preparation of (+,-)-4-[3,5-dimethylbenzoyl]-2-(3,4-dichlorophenyl)-1-[[[1-(phenylmethyl)-4-piperidinyl]amino]acetyl]piperazine

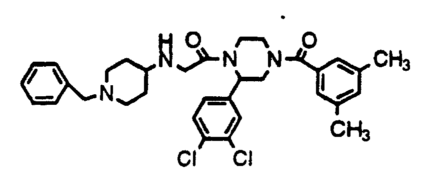

By employing methods analogous to those described in Example 14 and Example 15, and using 3,5-dimethylbenzoic acid in place of 3,5-bis(trifluoromethyl)-benzoic acid, the title compound was prepared as a solid, m.p.69-70 °C; FAB mass [M+1]^{+ 35}Cl 593.1 Calcd. for C₃₃H₃₈N₄O₂Cl₂: C, 66.77; H, 6.45; N, 9.44; Cl, 11.94.
Found: C, 66.64; H, 6.74; N, 9.48; Cl, 11.89.

This racemic compound was resolved into Enantiomers A and B by the analogous methods described in Example 17 and Example 18.
Enantiomer A m.p. 64-66⁰ C [α] D²⁵ = + 26.3
   FAB mass [M+1]^{+ 35}Cl 593.3;
Enantiomer B m.p. 64-66°C [α]_{D}²⁵ = - 34.8
   FAB mass [M+1]^{+ 35}Cl 593.3;

### EXAMPLE 27

By employing methods analogous to those described in Example 14, Example 15, Example 16, and Example 26, using appropriate amino reagents, the following compounds were obtained.

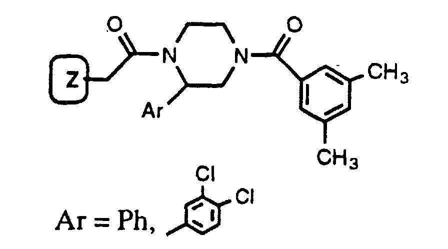

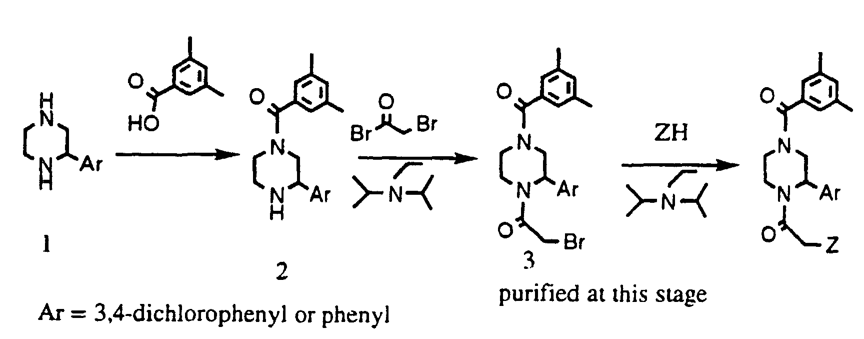

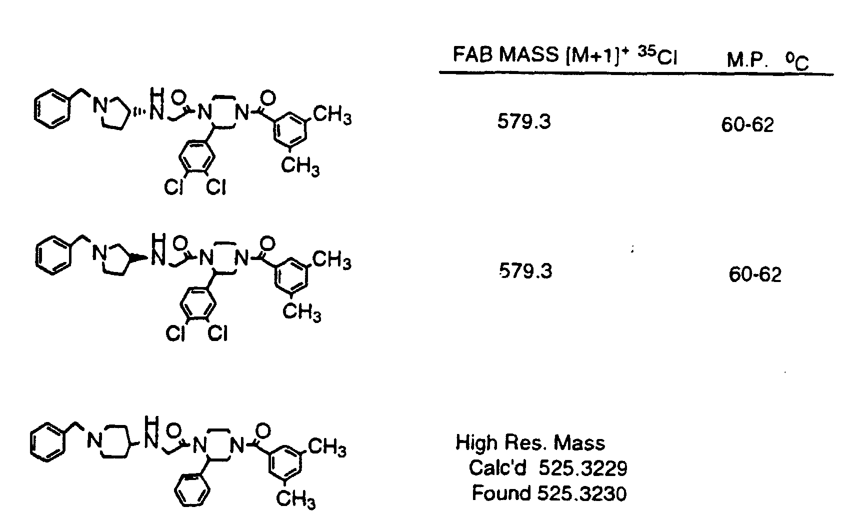

### EXAMPLE 28

### Preparation of (+,-)-2-(3,4-dichlorophenyl)-4-(3,5-dimethylbenzoyl)-1-[[[1-(2-furanylmethyl)-4-piperidinyl)amino]acetyl]]piperazine

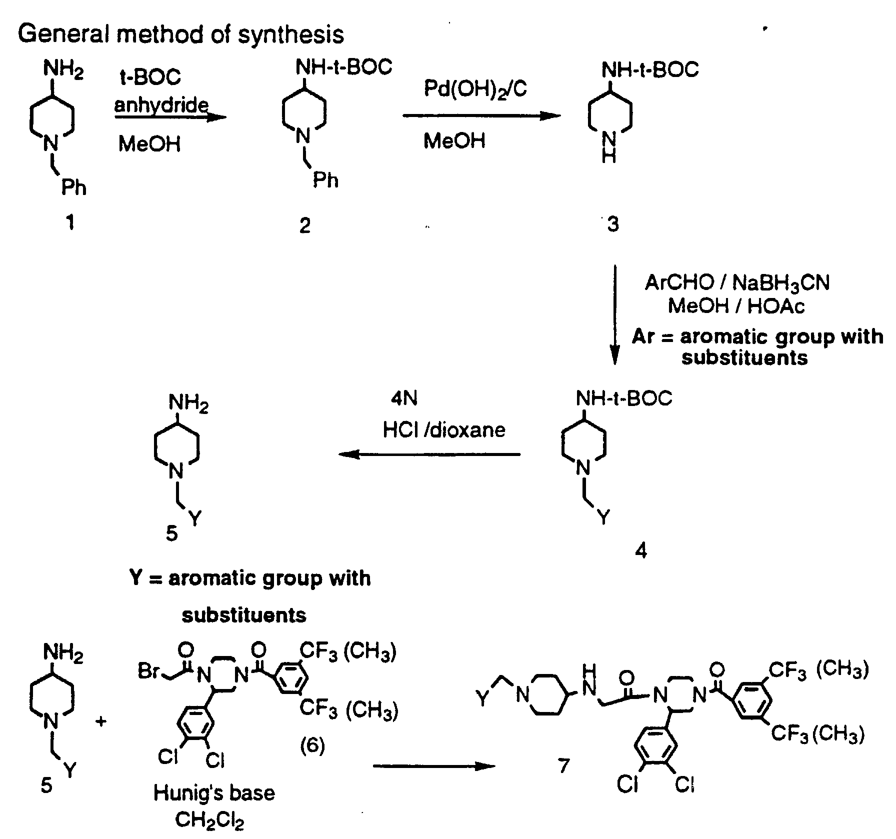

To a solution of 4-amino-1-benzylpiperidine (9.5, 50mmol) in methanol (150ml) at -10°C was added a solution of di-t-butyldicarbonate (10.9g, 50mmol) in methanol (60 ml). The mixture was gradually warmed to room temperatures overnight. After the reaction was complete , solvent was removed to give a white solid (2) FAB MASS [M+1]⁺³⁵Cl 291.3

Compound (2) (11.6g, 40 mmol) was dissolved in methanol (140 ml). To this solution was added Pd(OH)₂(20%) on carbon (2.4g) and the mixture was hydrogenolyzed at 47 pounds per square inch. After the reaction was complete, the catalyst was filtered. The filtrate was evaporated to give a white solid of compound 3 (8g, 40 mmol).

A mixture of compound 3 (0.7g, 35 mmole) and 2-furaldehyde in MeOH (10 ml) was stirred at RT for 10 min. NaBH₃CN (0.59, 8 mmol) and acetic acid (1 ml) were added later. The mixture was stirred at RT overnight under an atmosphere of nitrogen. After the reaction was complete, CH₂Cl₂ (50 ml) was added and the mixture was washed with saturated NaHCO₃ solution (30ml) and brine (30 ml 2x). The organic layer was evaporated after drying over MgSO₄ to give a crude tan solid 4 ( Y is 2-furanylmethyl) which was used without purification. Compound 4 was dissolved in dry CH₂Cl₂ (2ml) and treated with 4N HCl/dioxane (5ml) solution. Solvents and excess HCl were evaporated after stirring the reaction at RT for 2 hours. It gave compound 5 as an off-white solid (0.65g) as HCI salt.

Compound 5 was converted in situ to the free base with Hünig's base then reacted with the bromoacetyl key intermediate (6) in the above scheme to give the title compound analogous to the method described in Example 15.
m.p. 65-67 °C; FAB mass [M+1]^{+ 35}Cl 583

### EXAMPLE 29

By procedures analogous to the methods described in Example 28 and Example 15, the following compounds were obtained.

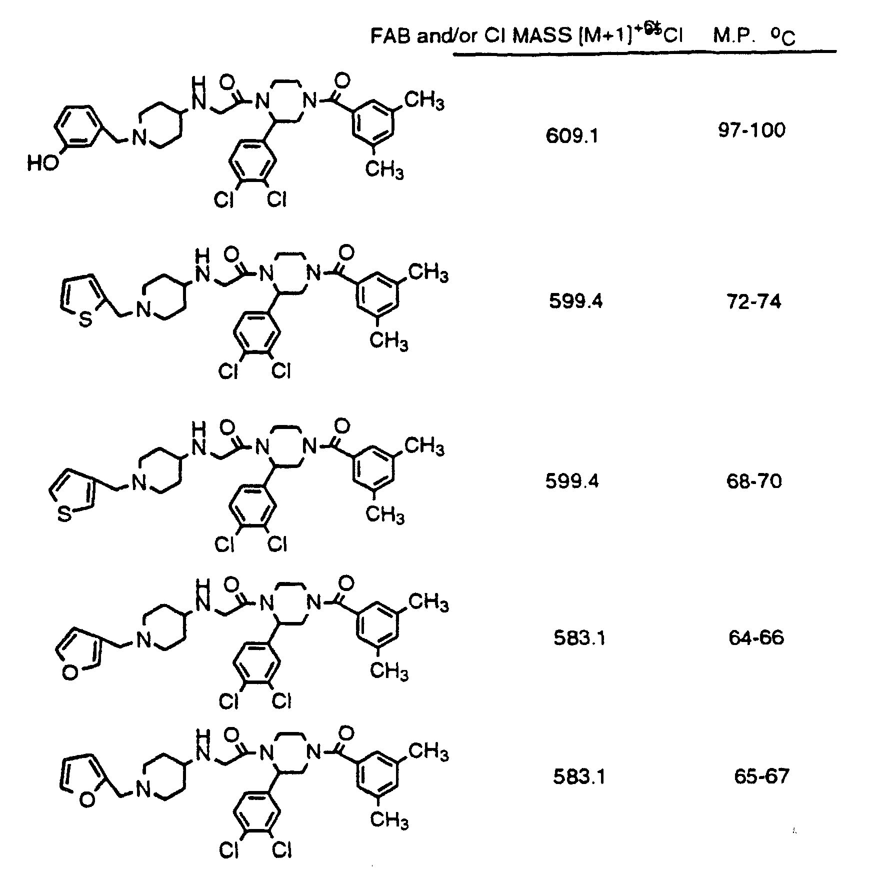

### EXAMPLE 30

### Preparation of (+,-)-1-[[[1-[[[1,1'-biphenyl]-4-yl]methyl]-4-piperidinyl]amino]acetyl]-2-(3,4-dichlorophenyl)-4-(3,5-dimethylbenzoyl)piperazine

By methods analogous to those described in Example 28, compound (3) (0.69, 3 mmol) from Example 28, was mixed with 4-(chloromethyl)biphenyl (0.61g, 3 mmol) (Y= C₆H₅-C₆H₅), Hünig's base (0.43g, 3 mole) in CH₂Cl₂ (10 ml) and stirred at RT for two days. After completion, the reaction mixture was diluted with CH₂Cl₂ (50 ml) and washed with brine 930 ml, 2x). The CH₂Cl₂ layer was dried over MgSO₄, filtered, and concentrated to give a white solid (1g). The crude product was purified on flash grade silica gel, eluting with 2% NH₄OH-MeOH/ CH₂Cl₂ system to give compound 4 (Y= C₆H₅-C6H5) as in Example 39 as a white solid (0.7g, 64%).

The next two reactions leading to the synthesis of the title compound are analogous to Example 28 described above. FAB Mass [M+1]^{+ 35}Cl 669; m.p.87-89 °C.

### EXAMPLE 31

By procedures analogous to the methods described in Examples 16, 28, and 30, the following compounds were obtained.

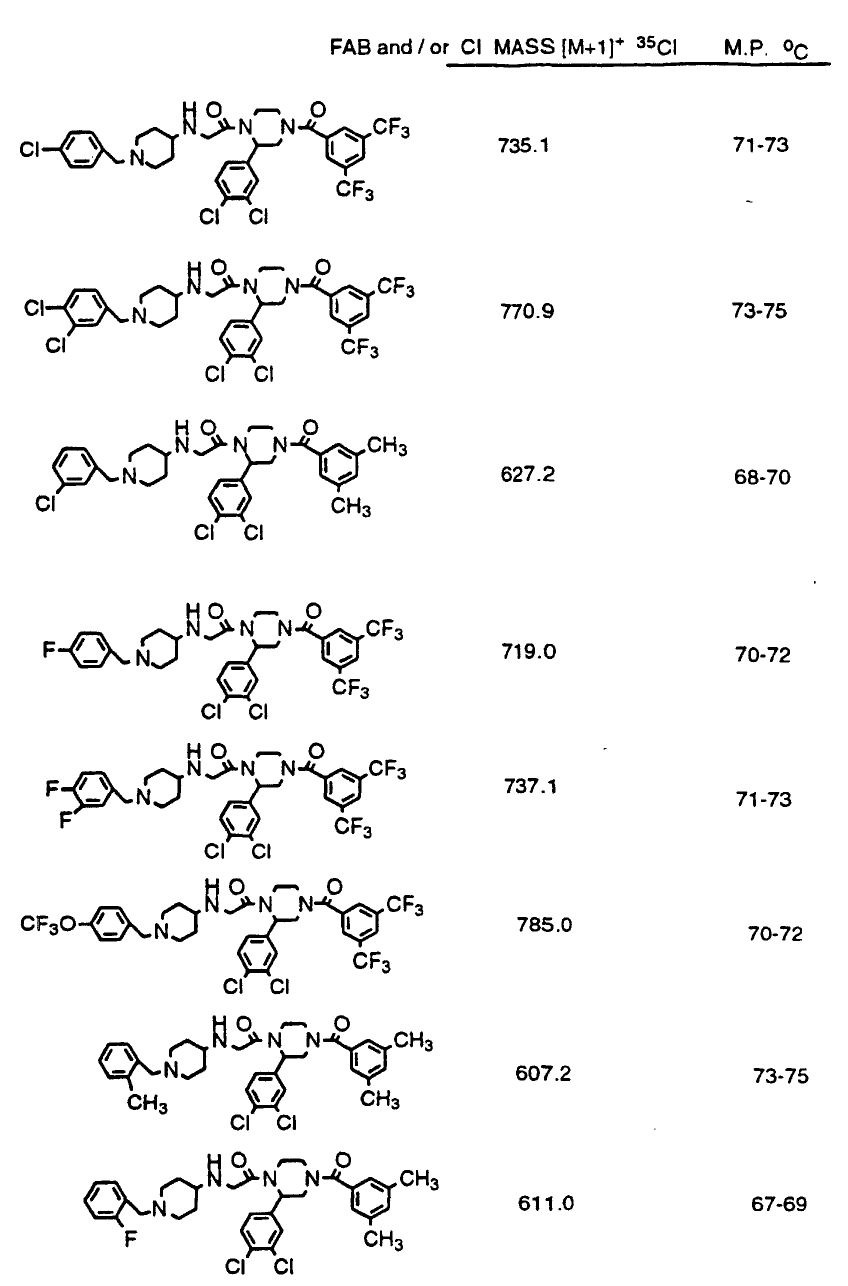

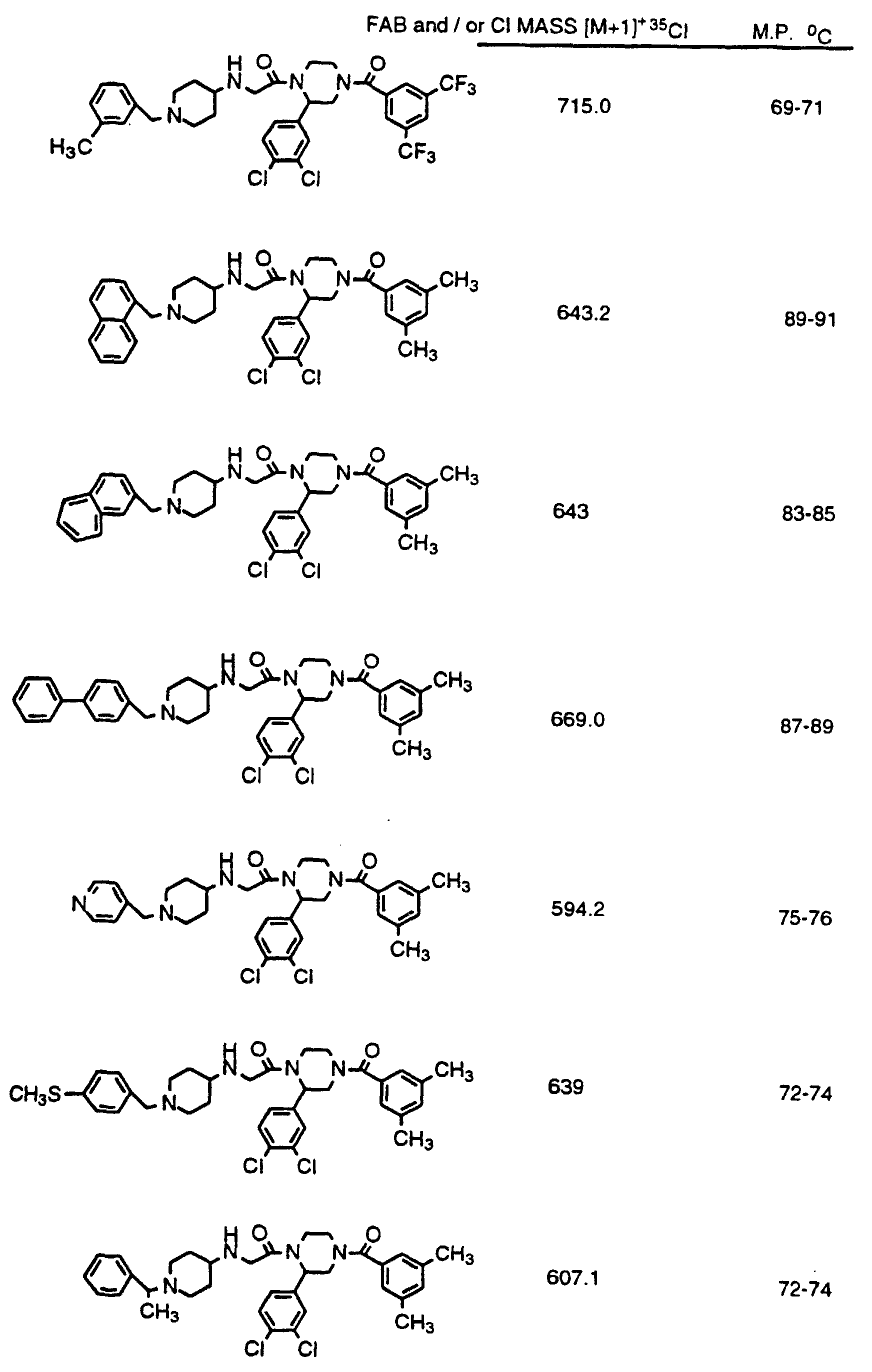

### EXAMPLE 32

### Preparation of (+,-)-2-(3,4-dichlorophenyl)-4-[(4-fluoro-1-naphthalenyl)carbonyl]-1-[[[1-(phenylmethyl)-4-piperidinyl]amino]acetyl]piperazine

### GENERAL METHODS

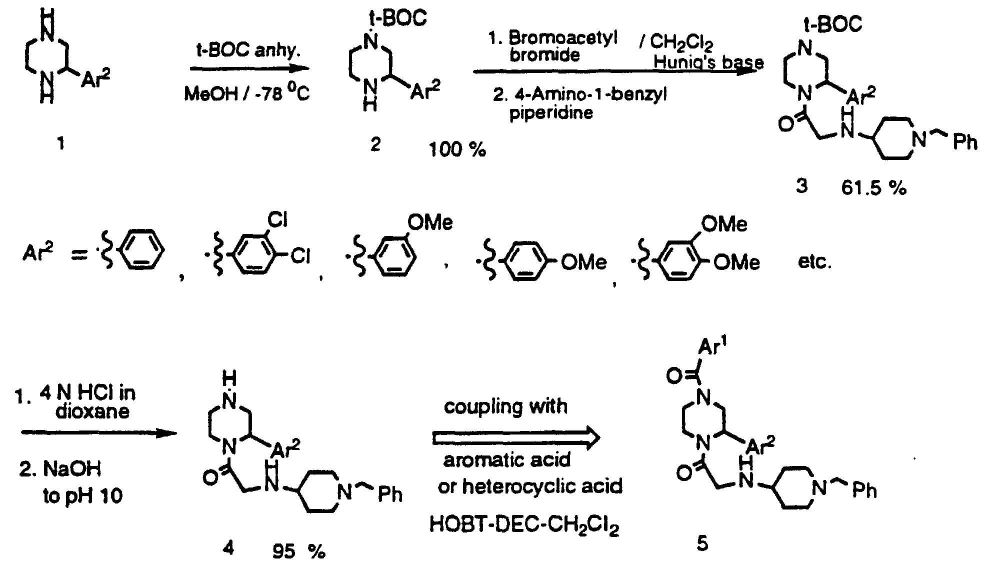

To a cooled solution of 2-(3,4-dichlorophenyl)piperazine (1; Ar² = 3,4-dichlorophenyl) (20g, 86.53 mmol) in MeOH (900 ml) at -78 °C was added dropwise a solution of t-BOC anhydride (19.47g, 86.53 mmol) in MeOH (263 ml) over 3h period under N₂. The solution was gradually warmed up to RT overnight. After reaction was complete, the solvent was evaporated and the residue was dried under high vacuum overnight to give 2 as a white solid. (28g) (Ar² = 3,4-dichlorophenyl) FAB Mass [M+1]^{+ 35} Cl 331.2.

To a cooled solution of compound 2 (23.8g, 71.85 mmol) in CH₂Cl₂ (500 ml) at -78 °C was added a solution of bromoacetylbromide (6.88 ml, 79.04 mmol) in CH₂Cl₂ (10ml) through a dropping funnel under N₂ over a 10 min. period. After stirring at -78 °C for 3 h, TLC showed that the reaction was complete. To this cooled solution were added Hünig's base (13.76 ml,79 mmol) and 4-amino-1-benzylpiperidine (29.30 ml, 143.7 mmol). It was kept at -78 °C for one hour then gradually warmed up to RT overnight. After completion, CH₂Cl₂ (200 ml) was added and washed with brine (200 ml, 3x), dried over MgSO₄, filtered and concentrated to give a light brown residue of compound 3 (46g) (Ar2 = 3,4-dichlorophenyl). Compound 3 was purified by flash chromatography on 400g of flash grade silica gel, eluting with 3.5 % NH₃-MeOH/CH₂Cl₂ to give 24.8 g (44.2 mmol, 61.5 %) of pure compound 3 (Ar² = 3,4-dichlorophenyl). FAB mass [M+1]⁺ ³⁵Cl 561.3

To a solution of compound 3 (Ar²= 3,4-dichlorophenyl) ( 16g, 28.49 mmol) in CH₂Cl₂ (142.5 ml) at 0 °C was added 4N HCl-dioxane solution (71.24 ml, 284.9 mmol) through a dropping funnel. The reaction was gradually warmed up to RT and stirred for 4h. After completion, the solvents were evaporated to give a light yellow solid which was dissolved in H₂O (400 ml) and brought to pH 10 with 1 N NaOH. The product was extracted from basic aqueous solution with CH₂Cl₂(200ml, 4x), dried over MgSO₄, filtered and concentrated to give compound 4 (Ar² = 3,4-dichlorophenyl) as a light yellow solid (12.5g, 27.09 mmol, 95%). FAB Mass [M+1]⁺³⁵ Cl 461.1 Compound 4 was the key intermediate which was used to couple with various aromatic acid for the synthesis of many target compounds.

To a solution of compound 4 (Ar² = 3,4-dichlorophenyl) (200 mg, 0.433 mmol) in CH₂Cl₂ (5 ml) were sequentially added 4-fluoro-1-naphthoic acid (84 mg, 0.433 mmol), HOBT (58.5 mg, 0.434 mmol), Et₃N (63.4 ml, 0.455 mmol) and DEC (85 mg, 0.434 mmol) at RT. The reaction was stirred at RT under N2 overnight. After completion, the reaction was diluted with EtOAc (150 ml) and washed with brine (50 ml, 3x), dried over MgSO₄, filtered and concentrated to give a crude product 5 (Ar² = 3,4-dichlorophenyl, Ar¹ = 4-fluoro-1-naphthyl) which was purified by flash chromatography ( 50 g flash grade silica gel), eluting with 4% sat'd NH₃-MeOH in CH₂Cl₂ to give the title compound (0.21g, 0.331 mmol, 76.5%). Fab Mass [M+1]⁺³⁵ Cl 633.2; m.p. 78-81 °C.

### EXAMPLE 33

The following compounds were prepared according to the procedures in Example 32. The key intermediate compound 4 in Example 32 (Ar² = 3,4-dichlorophenyl) was coupled with the appropriate aromatic acid to obtain the target compounds. Those compounds without melting points were prepared via parallel synthesis.

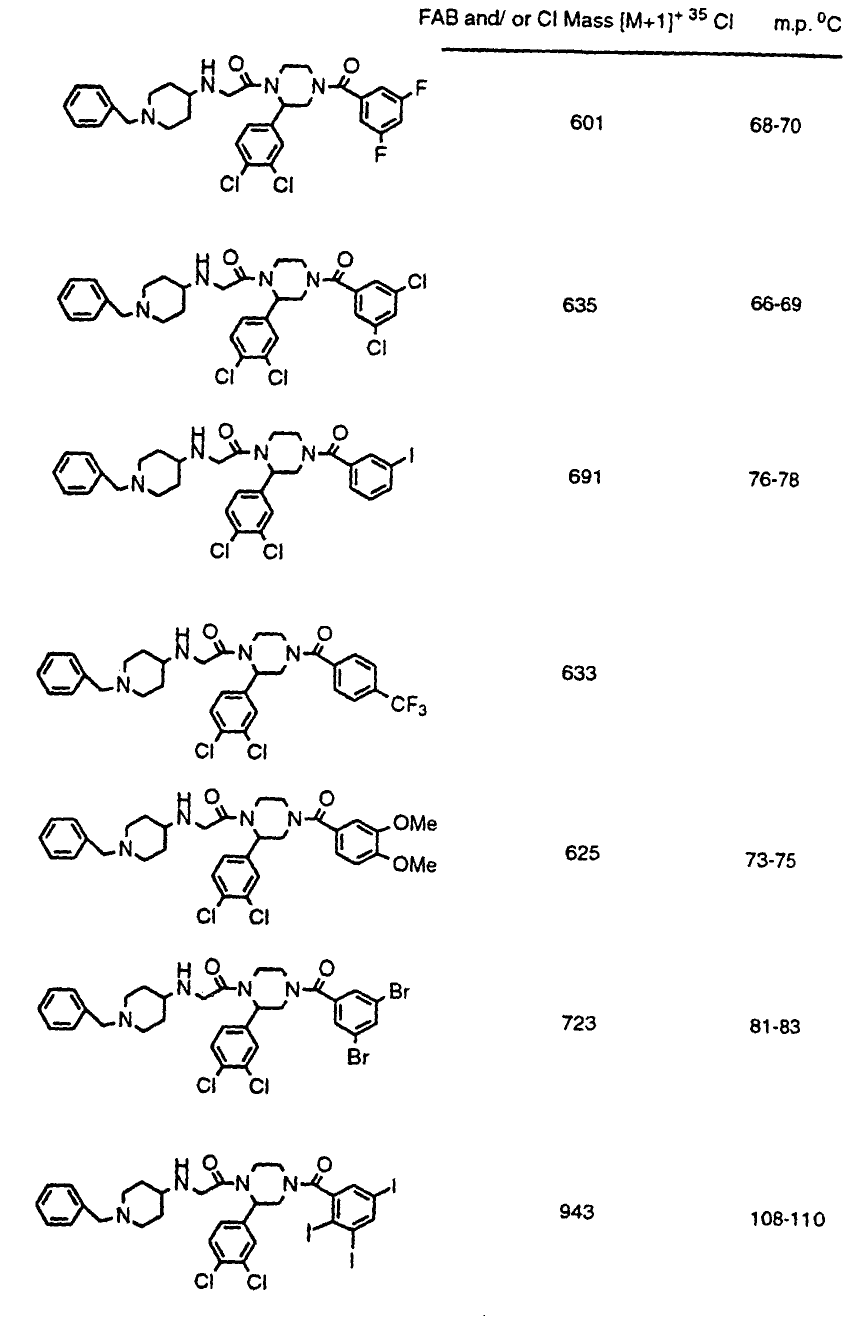

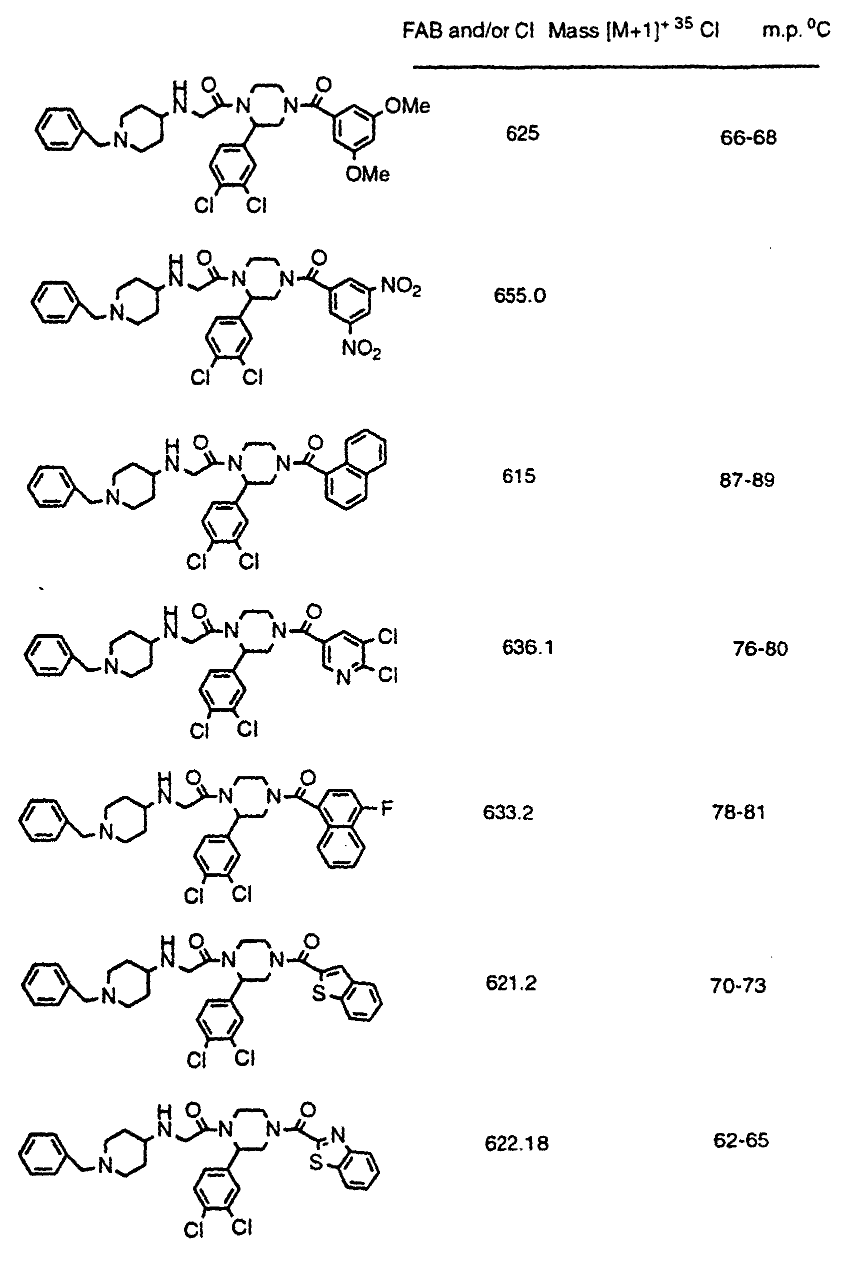

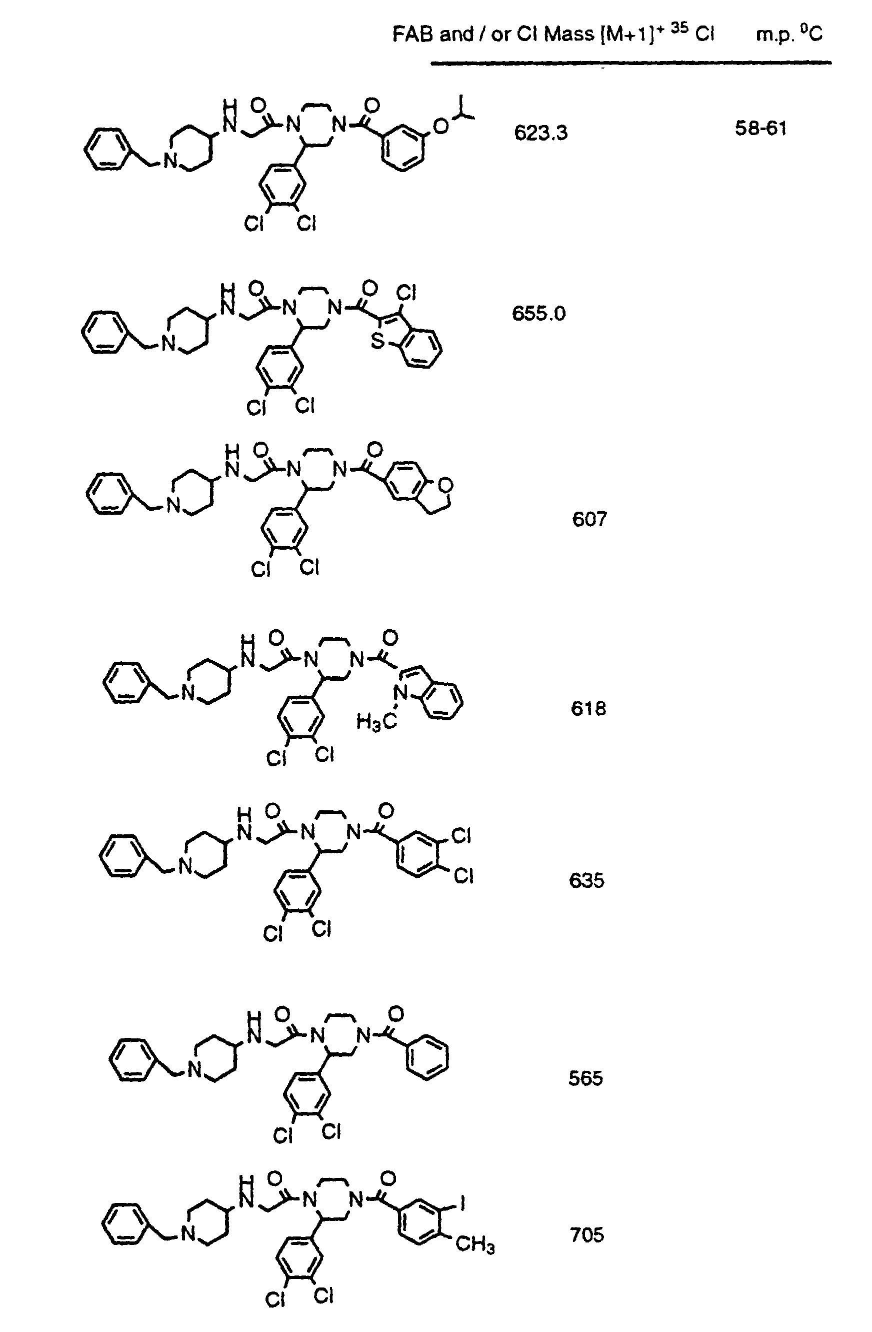

### EXAMPLE 34

The following compounds were prepared according to the procedures in Example 32. The key intermediate compound 4 in Example 32 ( Ar² = phenyl) was prepared first then coupled with the appropriate aromatic acid as described in Example 32 to obtain the target compounds. Those compounds without melting points were made by parallel synthesis.

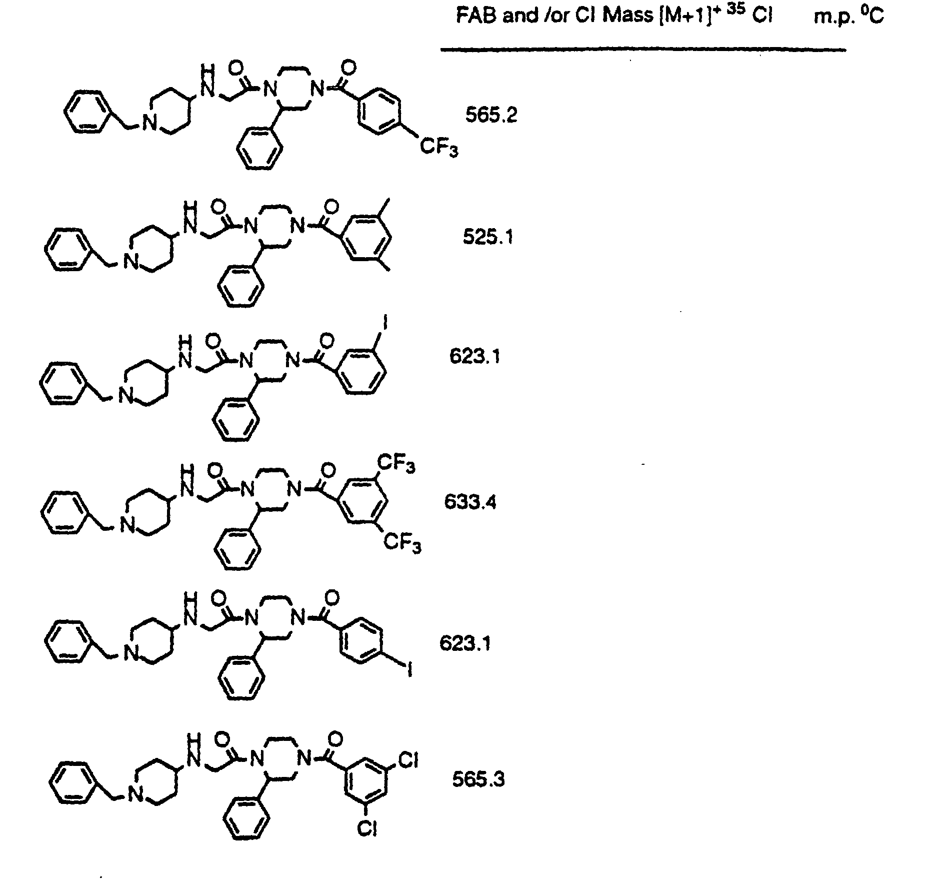

### EXAMPLE 35

### Preparation of (+,-)-4-(3,5-Dimethylbenzoyl)-1-[[[1-(phenylmethyl)-4-piperidinyl]amino]acetyl]-2-[4-(trifluoromethyl)phenyl]piperazine, 1.2 hydrate

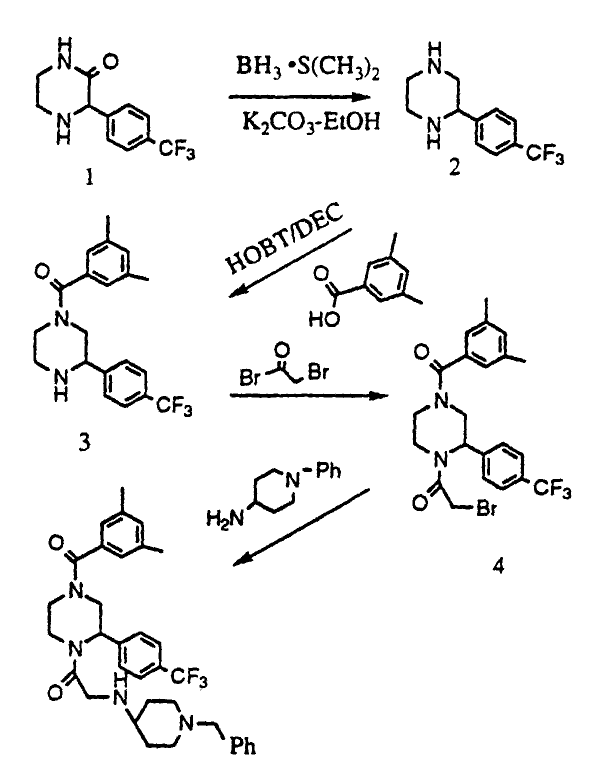

3-(4-Trifluoromethylphenyl)-2-piperazinone (1) was prepared according to the procedures published in J.Med.Chem. 9, 191, 1966. To a solution of compound 1 (0.65g, 2.66 mmol) in anhydrous THF (22 ml) was added dropwise10 M BH₃•S(CH₃)₂ (0.798 ml) at RT. The mixture was refluxed for 22 hr. The mixture was cooled in an ice-water bath and quenched with MeOH (5 ml). The solvent was evaporated and the residue was dissolved in absolute EtOH (30 ml). To this solution was added anhydrous K₂CO₃ (0.8g) and the mixture was refluxed for 1 h then stirred at RT for 1 h , filtered and concentrated to give an orange solid which was purified by flash chromatography using flash grade silica gel (24g), eluting with 2.5%-4% of satd. NH₃/MeOH in CH₂Cl₂ to give compound 2 (0.143g, 23.4%). Compound 2 was carried on the next three steps in the above diagram according to the procedures described in Example 14 and Example 15 for the preparation of the title compound. FAB Mass [M+1]⁺ ³⁵Cl 593.1

### EXAMPLE 36

The following compounds were prepared according to the procedures in Example 5 (Method 2), Example 14, and Example 15 by using appropriate reagents.

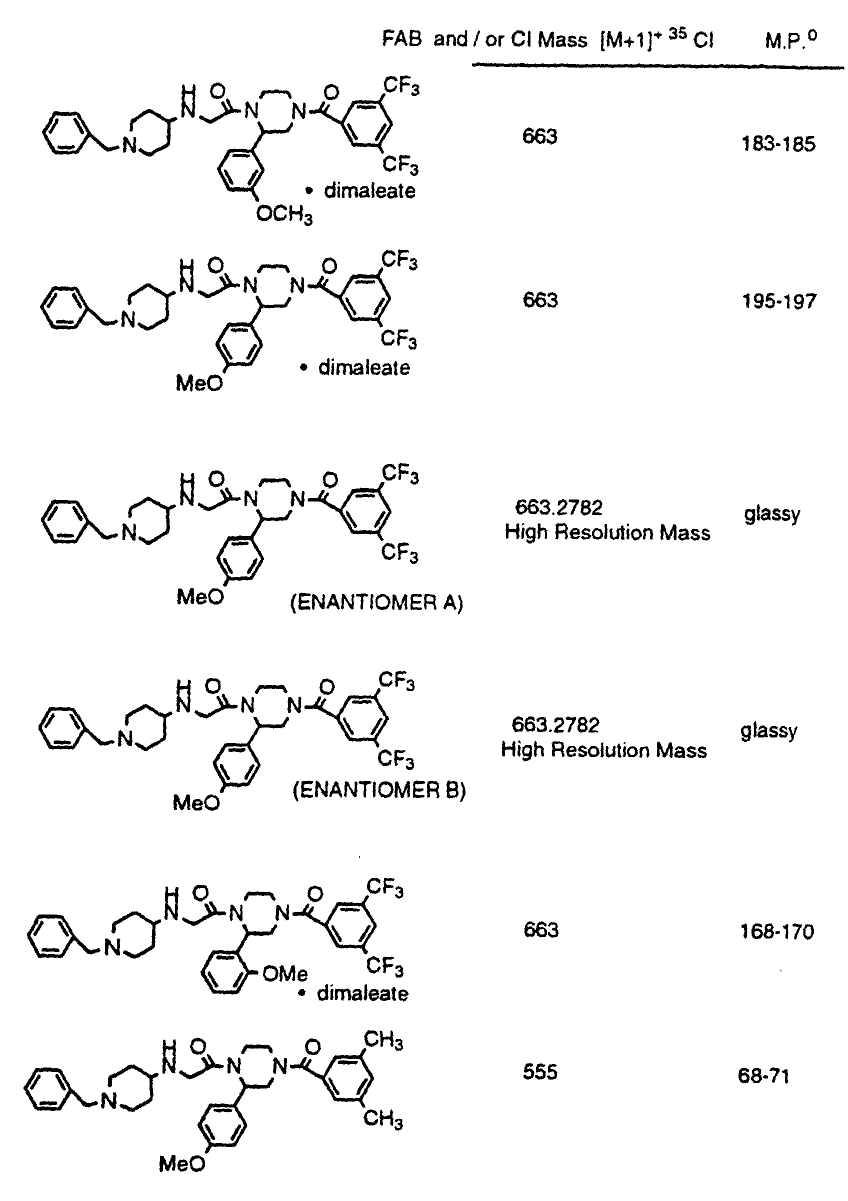

### EXAMPLE 37

### Preparation of (+,-)-4-[3,5-Bis(trifluoromethyl)benzoyl]-2-(3-hydroxyphenyl)-1-[[[1-(phenylmethyl)-4-piperidinyl]amino]acetyl]piperazine, dimaleate, hemihydrate

To a stirred solution of (+,-)-4-[3,5-bis(trifluoromethyl)benzoyl]-2-(3-methoxyphenyl)-1-[[[1-(phenylmethyl)-4-piperidinyl]amino]acetyl]piperazine, dimaleate ( 1g, 1.51 mmol) in anhydrous 1,2-dichloroethane (50 ml) at 0 °C was added a 1 M solution of BBr₃ • S(CH₃)₂ in CH₂Cl₂ (15 ml). The mixture was stirred at 0 °C-RT for 1 h then gradually heated to 80 °C and maintained at 80 °C for 1 h. After cooling, the solution was poured into ice-water and basified with NH₄OH. The product was extracted from aqueous solution with CH₂Cl₂( 100 ml, 3x) and combined. The organic extract was washed with brine, dried (MgSO₄), filtered and concentrated to give a solid (0.92 g) which was purified by flash silica chromatography, eluting with MeOH-CH₂Cl₂-28%NH₄OH (90 : 10 : 0.2) to yield 0.62g of the desired compound as a syrup. FAB Mass [M+1]^{+ 35} Cl 649.4 To a solution of this syrup (0.61g, 0.94 mmol) in EtOAc (10 ml) was added a solution of maleic acid (0.218g, 1.88 mmol) in EtOAc (20 ml). The precipitate was collected and recrystallized from MeOH-EtOAc to give the title compound as a white solid (0.58g). FAB Mass [M+1]^{+ 35} Cl 649.3; m.p. 186-187 °C. Calcd for C₃₃H₃₄F₆N₄O₃ • 2 (C₄H₄O₄) • 0.5 H₂O : C, 55.34; H, 4.87; N 6.30. Found: C, 55.18; H, 5.14; N, 6.35.

### EXAMPLE 38

### Preparation of (+,-)-4-[3,5-Bis(trifluoromethyl)benzoyl]-2-(4-hydroxyphenyl)-1-[[[1-(phenylmethyl)-4-piperidinyl]amino]acetyl]piperazine, dimaleate, hemihydrate

Following the same procedure described in Example 37 using (+,-)-4-[3,5-bis(trifluoromethyl)benzoyl]-2-(4-methoxyphenyl)-1-[[[1-(phenylmethyl)-4-piperidinyl]amino]acetyl]piperazine, dimaleate as starting material, the title compound was obtained as a white solid dimaleate salt. FAB Mass [M+1]^{+ 35} Cl 649.3; m.p. 175-178 °C.

### EXAMPLE 39

### Preparation of 2-(R,S)-(3,4-dichlorophenyl)-4-(3,5-dimethylbenzoyl)-1-[3-hydroxy-1-oxo-2(S)-[[1-(phenylmethyl)-4-piperidinyl]amino]propyl]piperezine, hemihydrate

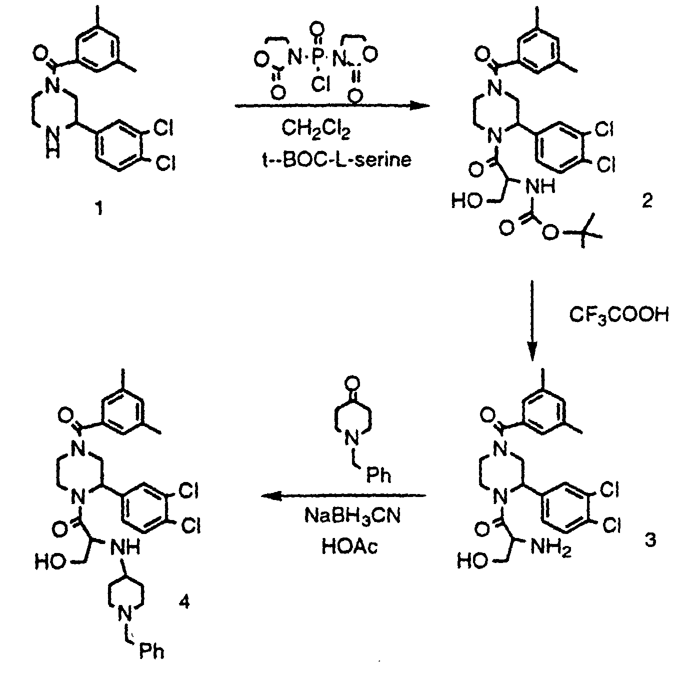

To a solution of 2-(R,S)-(3,4-dichlorophenyl)-4-(3,5-dimethylbenzoyl)piperazine (1.0 g, 2.753 mmol) in CH₂Cl₂ (10 ml) were added bis (2-oxo-3-oxazolidinyl) phosphinic chloride (0.72g, 2.753 mmol) and Hünig's base (0.48 ml, 2.753 mmol). The mixture was stirred at RT for 4 days. The reaction was diluted with CH₂Cl₂ (200 ml), washed with brine (80 ml, 3x), dried (MgSO₄), filtered and evaporated to dryness to give 2.3g crude product. The crude material was purified by silica flash chromatography, eluting with 2% sat'd NH₃/ MeOH in CH₂Cl₂ to give (0.21g, 0.38 mmol, 14%) of compound 2 in Example 39. The tBOC protecting group of compound 2 was removed by treatment with CF3COOH in CH₂Cl₂ overnight to give compound 3.

To a solution of compound 3 ( 180 mg, 0.32 mmol) in MeOH (3.2 ml) at RT was added 1-benzyl-4-piperidone (60 µl, 0.32 mmol). After stirring at RT for 1h, NaBH₃CN (26 mg, 0.47 mmol) and acetic acid (32 µl) were added. The mixture was stirred at RT ovemignt. The reaction was stopped by addition of H₂O (100ml) and adjusted to pH 11 with 1 N NaOH. The product was extracted from the aqueous layer with CH₂Cl₂ (50 ml, 3x) and combined, dried (MgSO₄), filtered and evaporated to dryness. The crude material was purified by silica flash chromatography, eluting with 4% sat'd NH₃-MeOH/CH₂Cl₂ to give the title compound as a white solid. FAB Mass[M+1]^{+ 35}Cl 623; m.p.69-72 °C.

### EXAMPLE 40

### Preparation of 2-(R,S)-(3,4-dichlorophenyl)-4-(3,5-dimethylbenzoyl)-1-[4-methyl-1-oxo-2(R,S)-[[1-(phenylmethyl)-4-piperidinyl]amino]pentyl]piperazine

By employing the methods described in Example 39 using (D, L)-isoleucine in place of L-serine, the title compound was obtained as a white solid. FAB Mass [M+1]^{+ 35}Cl 649.1; m.p. 68-71 °C.

### EXAMPLE 41

### Preparation of (+,-)-N-[2-[2-(3,4-Dichlorophenyl)-4-(3,5-dimethylbenzoyl)-1-(piperazinyl]-2-oxoethyl]-N-[1-phenylmethyl)-4-piperidinyl]acetamide, hemihydrate

To a solution of (+,-)-2-(3,4-dichlorophenyl)-4-(3,5-dimethylbenzoyl)-1-[[[(phenylmethyl)-4-piperidinyl]amino]acetyl]piperazine (Example 26) (0.26g, 0.43 mmol) in anhydrous CH₂Cl₂ (2.5 ml) at -78 °C were added Hünig's base (0.1 ml, 0.57 mmol), and acetyl chloride (32 ml, 0.45 mmol). The mixture was gradually warmed to RT overnight under N₂. The reaction was quenched with saturated NaCl solution and extracted with CH₂Cl₂ twice, combined and washed with brine twice. The organic layer was dried over MgSO₄, filtered and concentrated to give a solid which was purified by flash silica chromatography, eluting with 4% sat'd NH₃/MeOH in CH₂Cl₂ to give a white solid 0.22g (0.35mmol, 79%).
FAB Mass [M+1]^{+ 35}Cl 635.2; m.p.99-102 °C.

### EXAMPLE 42

### Preparation of (+,-)-2-(3,4-dichlorophenyl)-4-(3,5-dimethylbenzoyl)-1-[[[2-hydroxyethyl][1-(phenylmethyl)-4-piperidinyl]amino]acetyl]piperazine, hemihydrate

To a mixture of 4-amino-1-benzylpiperidine (2.13g, 11.2 mmol) and 2-bromoethanol (0.15g, 4.08 mmol) in anhydrous CH₂Cl₂ (20 ml) was added Hünig's base (2.6 ml, 5.7 mmol). The solution was refluxed for 24 hours. After evaporating off CH₂Cl₂, the crude material was purified by silica flash chromatography to afford 2-[[1-(phenylmethyl)-4-piperidinyl]amino]ethanol (0.88g, 3.8 mmol) with 92% yield. FAB Mass [M+1]^{+ 35}Cl 235.

By employing an analogous method to that described in Example 15, using β-hydroxyethyl-4-amino-1-benzylpiperidine in place of 4-amino-1-benzylpiperidine, the title compound was obtained as a white solid after silica flash chromatography. FAB Mass [M+1]^{+ 35}Cl 637; m.p. 70-73 °C.

### EXAMPLE 43

### Preparation of (+,-)-N-[2-[2-(3,4-dichlorophenyl)-4-(3,5-dimethylbenzoyl)-1-piperazinyl]-2-oxoethyl]-N,N-dimethylamino-1-(phenylmethyl)-4-piperidinaminium bromide, dimethanolate

To a solution of 4-amino-1-benzylpiperidine (1.47g, 7.7 mmol) in CF₃CH₂OH (14 ml) were added molecular sieve 4 A° (5g), and paraformaldehyde (0.51g, 17 mmol). After stirring at RT for 1 h, NaCNBH₃ (2.5g, 39.8 mmol) was added and stirred for 16 h at RT. The reaction was stopped by addition of H₂O and the product was extracted with (4:1) (Et₂O:CH₂Cl₂). Organic fractions were combined and washed with brine (2x), dried over MgSO₄, filtered and concentrated to give a crude material which was purified by silica flash chromatography to afford 4-dimethylamino-1-benzylpiperidine (80 % yield). FAB Mass [M+1]^{+ 35}Cl 219.

By employing an analogous method to that described in Example 15, using N,N-dimethyl-4-amino-1-benzylpiperidine in place of 4-amino-1-benzylpiperidine, the title compound was obtained as a white solid after silica flash chromatography. FAB Mass [M+1]^{+ 35}Cl 621.2

### EXAMPLE 44

### Preparation of (+,-)-2-(3,4-dichlorophenyl)-4-(3,5-dimethylbenzoyl)-1-[[methyl [1-(phenylmethyl)-4-piperidinyl]amino]acetyl]piperazine

To a solution of 1-benzyl-4-piperidone (1.9g, 10 mmol) in MeOH (10 ml) were added methylamine (8 ml, 16 mmol) and 3 A° molecular sieves. After stirring at RT for 1 h, the reaction was cooled in an ice-bath and 4 N HCl in dioxane (2.5ml, 10 mmole) and NaCNBH₃ (1.2g, 20 mmol) were added. The reaction was stirred at RT overnight. After the reaction was complete, H₂O was added the pH was adjusted to 10 with 50% NaOH solution. The product was extracted with EtOAc (100 ml, 3x)from aqueous solution and combined. The combined organic solution was washed with brine, dried over MgSO₄, filtered and concentrated to give an oil which was purified by silica flash chromatography, eluting with 8% sat'd NH₃/MeOH in CH₂Cl₂ to give 4-methylamino-1-benzyl piperidine (1.77g, 8.66 mmol, 86%). FAB Mass [M+1]^{+ 35}Cl 205.

By employing an analogous method to that described in Example 15, using 4-methylamino-1-benzylpiperidine in place of 4-amino-1-benzylpiperidine, the title compound was obtained as a white solid after silica flash chromatography. FAB Mass [M+1]^{+ 35}Cl 607.

### EXAMPLE 45

### Preparation of (+,-)-2-(3,4-dichlorophenyl)-4-(3,5-dimethylbenzoyl)-2-methyl-1-[[[1-(phenylmethyl)-4-piperidinyl]amino]acetyl]piperazine, 0.6 methanol

A solution of 3,4-dichlorophenylacetic acid (5.126g, 25 mmol) in THF was added dropwise over 15 min. to a cooled solution of 1.0 M Li(TMS)₂/ THF (55ml, 55 mmol) at -78 °C under N₂. The reaction mixture was stirred in a water-ice bath for 2 h then cooled to -78 °C and Mel (12 ml ) was added. The mixture was gradually warmed to RT. After reaction was complete, the precipitate was filtered off and rinsed with THF. The precipitate was dissolved in H₂O and acidified to about pH 2.0 then extracted with EtOAc ( 100ml, 3x). The extracts were combined, dried (MgSO₄), filtered and concentrated to give 91% yield of 1-(3,4-dichlorophenyl) propionic acid (4.984g, 22.7 mmol) as an oil. FAB Mass[M+1]^{+ 35} Cl 219.

1-(3,4-dichlorophenyl)propionic acid (20.23g, 99.1 mmol) was dissolved in MeOH (200ml). To this solution was added conc. H₂SO₄ (2 ml) and the solution refluxed for 1h. After cooling, the solution was basified with NaHCO₃ and extracted with CH₂Cl₂. Following by regular work-up, methyl 1-(3,4-dichlorophenyl)propionate was obtained as an oil.

By employing an analogous method as described in J. Med.Chem. 9,191,1966, methyl 1-bromo-1-(3,4-dichlorophenyl)propionate was obtained by bromination of methyl 1-(3,4-dichlorophenyl)propionate with NBS/CCl₄.

A mixture of methyl 1-bromo-1-(3,4-dichlorophenyl) propionate (3.39g, 10.9 mmol) and ethylenediamine (7ml) was stirred at RT for 3.5 hours. After reaction was complete, brine (100 ml) was added and the product was extracted from the aqueous layer with CH₂Cl₂ (50 ml, 2x). The combined organic extracts were washed with brine, dried over MgSO₄, filtered and concentrated to give a 95% yield of 3-(3,4-dichlorophenyl)-3-methyl-2-piperazinone which was reduced with LiAlH₄/Et₂O at 40 °C to 2-methyl-2-(3,4-dichlorophenyl)-1-piperidine as described in J. Med.Chem. 9, 191, 1966.

The title compound was prepared according to the analogous methods described in Examples 14 and 15. FAB Mass [M+1]+ 35 Cl 607.2; m.p.58-61 °C.

### EXAMPLE 46

### Preparation of (+,-)-2-(3,4-dichlorophenyl)-4-(4-fluoro-1-naphthalenylcarbonyl)-2-methyl-1-[[[1-(phenylmethyl)-4-piperidinyl]amino]acetyl]piperazine

By employing analogous methods described in Example 14, Example 15 and Example 45, the title compound was obtained as a white solid. FAB Mass [M+1]^{+ 35}Cl 647.2; m.p.93-96 °C.

### EXAMPLE 47

### Preparation of (+,-)-4-[3,5-dimethylbenzoyl)-4-hydroxymethyl-2-(3,4-dichlorophenyl)-1-[[[1-(phenylmethyl)-4-piperidinyl]amino]acetyl]piperazine

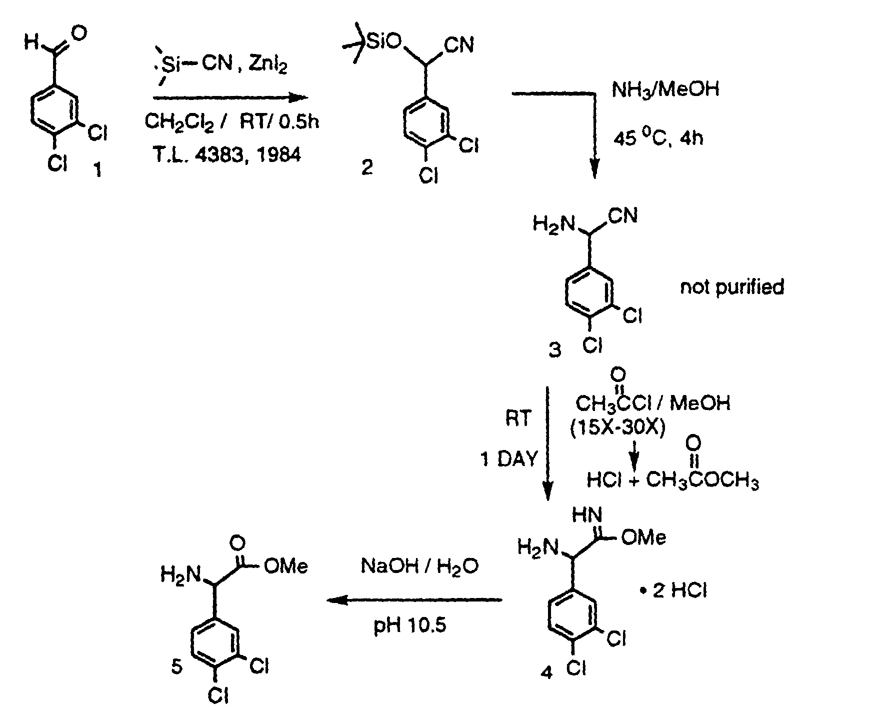

α-Amino-(3,4-dichlorophenyl)-acetonitrile (3) was prepared according to the procedure in Tetrahedron Letters 4383, 1984. Compound (3) was reacted with HCl/MeOH solution which was generated from acetyl chloride in MeOH to give compound (4). The methyl α-amino-(3,4-dichlorobenzene) acetate (5) was obtained by hydrolysis of (4) at pH 10.5. FAB Mass [M+1]⁺ ³⁵Cl 243.1

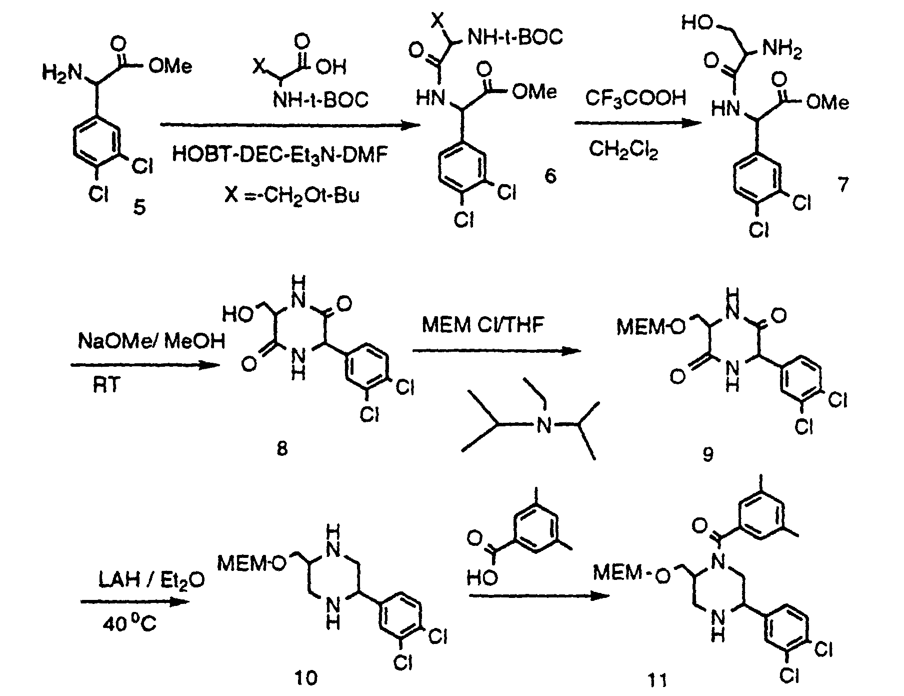

Methyl α-amino-(3,4-dichlorobenzene) acetate (5) was coupled with O-t-Bu-N-t-BOC-L-serine to give compound (6) which was deprotected with CF₃COOH to give compound (7), FAB Mass [M+1]^{+ 35}Cl 321. Compound (7) was cyclized by treatment with 25% NaOMe/ MeOH solution in MeOH to give compound (8), FAB Mass [M+1]^{+ 35}Cl 289.

Compound (8) was reacted with 2-methoxyethoxymethyl chloride (MEMCl) in THF in the presence of Hünig's base to give compound (9) which is reduced with LAH to afford compound (10). Compound (10) is coupled with 3,5-dimethylbenzoic acid according to the procedure described in Example 14 to give compound 11. By employing analogous procedures described in Example 15, the title compound is obtained as follows.

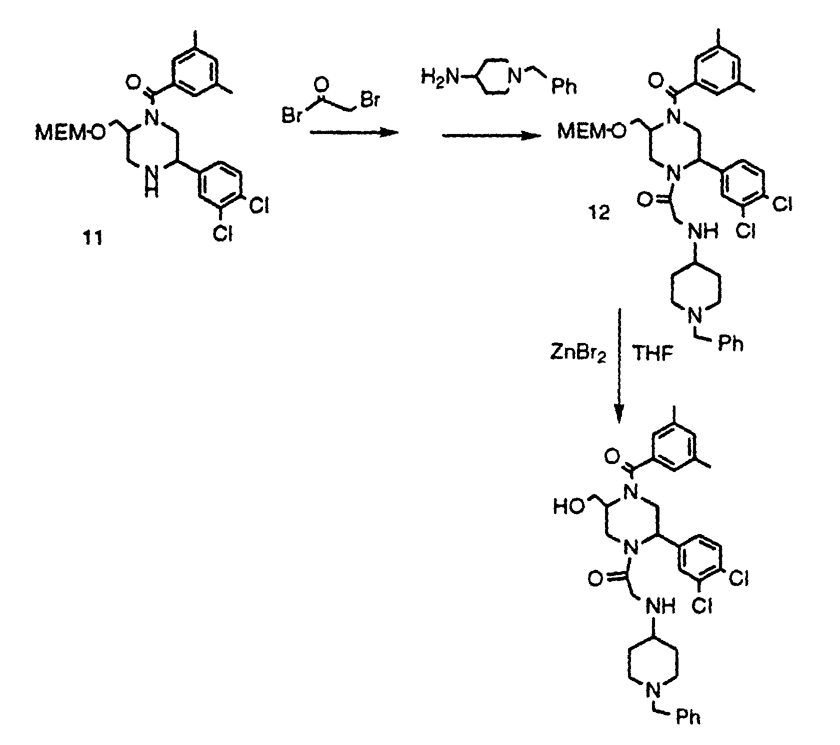

### EXAMPLE 48

### Preparation of (+,-)-1-[3,5-Bis[trifluoromethyl)benzoyl]-2-(3,4-dichlorophenyl)-4-[[[1-(phenylmethyl)-4-piperidinyl]amino]acetyl]piperazine, dimaleate, monohydrate

1,1-Dimethylethyl-3-(3,4-dichlorophenyl)-1-piperazinecarboxylate, i.e. compound 2 in Example 32 was coupled with 3,5-dimethylbenzoic acid described in Example 14. The t-BOC group was removed with trifluoroacetic acid. By analogous methods as described in Example 15, the title compound was obtained as a white solid, FAB Mass [M+1]^{+ 35}Cl 701; m.p. 170-172 °C.

## Claims

1. A compound of the formula: 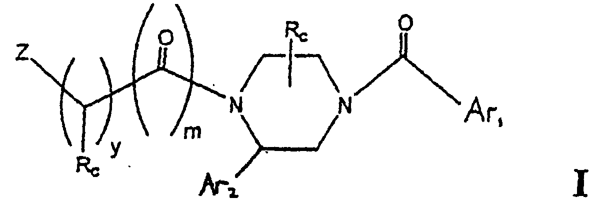
m is 1, and y is 1 to 3; or m is 2, and y is 0;
and with the further proviso that no more than one R_{c} is other than H in the 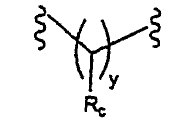 moiety;
each R_{c} is independently H, C₁-C₆ alkyl, -(CH₂)ₙ₁-R4 where n₁ is 1 to 6;
R₄ is -ORₐ, 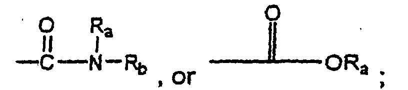
each Rₐ and R_{b} is independently selected from the group consisting of H, C₁-C₆ alkyl, phenyl, phenyl substituted by R¹, R², and/or R³; benzyl, benzyl substituted by R¹, R², and/or R³; allyl; or when Rₐ and R_{b}
are attached to the same nitrogen, then Rₐ and R_{b} together with the nitrogen to which they are attached, form a 4 to 7 member ring;
wherein each R₁ and R₂ is independently H, C₁-C₆ alkyl, CF₃, C₂F₅, Cl, Br, I, F, NO₂, ORₐ, CN, NRₐR_{b}, 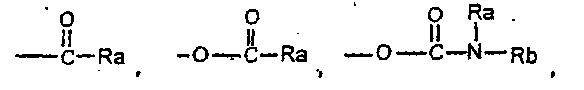 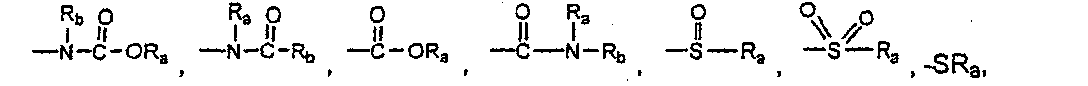 and 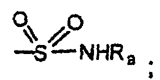 and where Rₐ is not H in 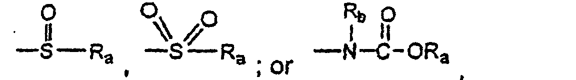 or when R₁ and R₂ are on adjacent carbons on a ring, they can form 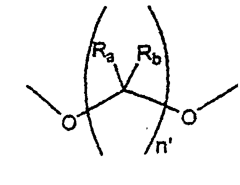 wherein n' is 1 or 2; and each R₃ is independently H, C₁-C₆ alkyl, CF₃, C₂F₅, 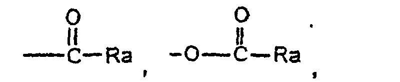 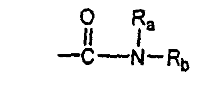 Cl, Br, I, or F, ORₐ, OCF₃, or phenyl;
Ar₁ is R¹,R²,R3-substituted heteroaryl, wherein heteroaryl is selected from pyridyl, benzothienyl, benzofuranyl, benzothiazolyl, indolyl or quinolyl, 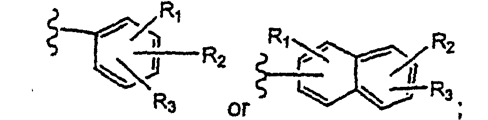
Ar₂ is 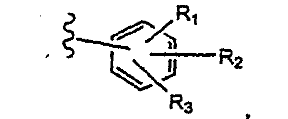
Z is 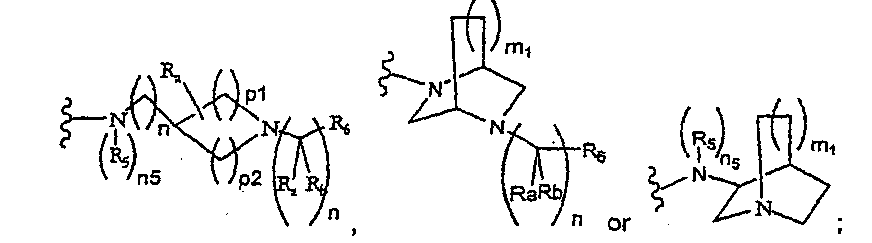
each R₅ is independently selected from the group consisting of H, 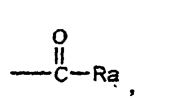 C₁-C₆ alkyl; with the proviso that when R₅ is C₁-C₆ alkyl, two R₅ can be attached to the nitrogen to form a quaternary salt; n₅ is 1, or n₅ is 2 with the proviso that each R₅ is independently C₁-C₆ alkyl; and m₁ is 0 or 1;
p₁ and p₂ are each independently 1 to 4 with the proviso that p₁ and p₂ added together are 2 to 6;
R₆ is thienyl, furanyl, pyridyl, tetrazolyl, imidazolyl, 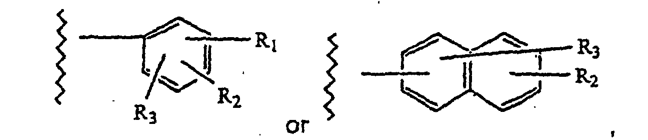
n is 0 to 2 ;
or a pharmaceutically acceptable salt thereof.

2. A compound according to claim 1, wherein m is 1, and y is 1.

3. A compound according to claim 1 wherein Z is

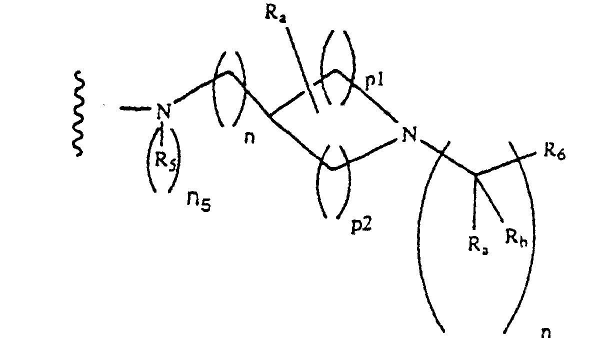

4. A compound according to claim 1 wherein Z is

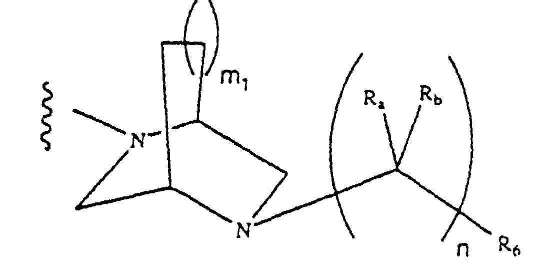

5. A compound according to claim 1 wherein Z is

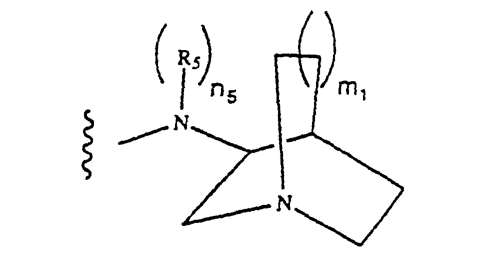

6. A compound according to claim 1 of the formula

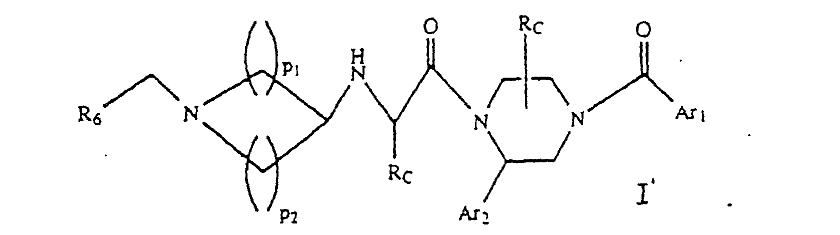

7. A compound according to claim 6 wherein R_{c} is H,
p₁ and p₂ are 2, Ar₁ and Ar₂ are both

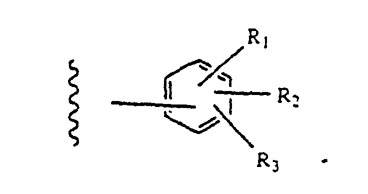

8. A compound according to claim 6 wherein R₆ is
thienyl, furanyl, pyridyl, tetrazolyl, imidazolyl,

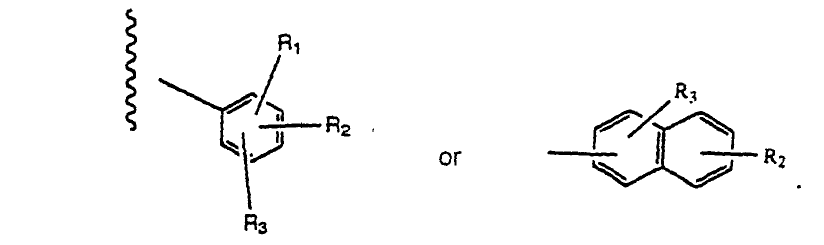

9. A compound according to claim 4 wherein R₆ is

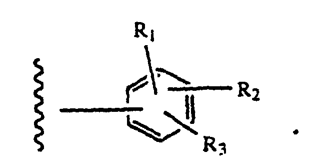

10. A compound according to claim 1 selected from the group consisting of 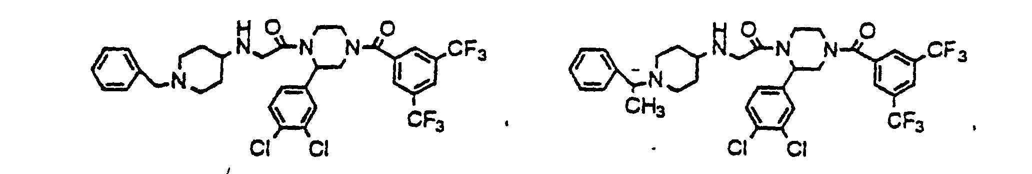 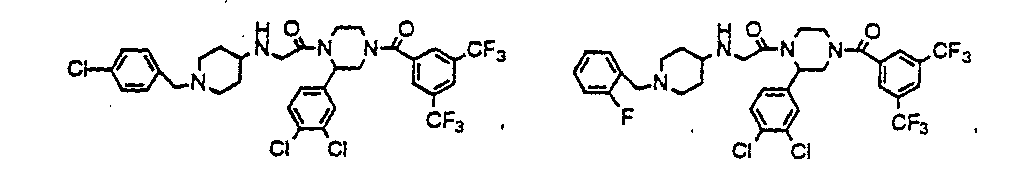 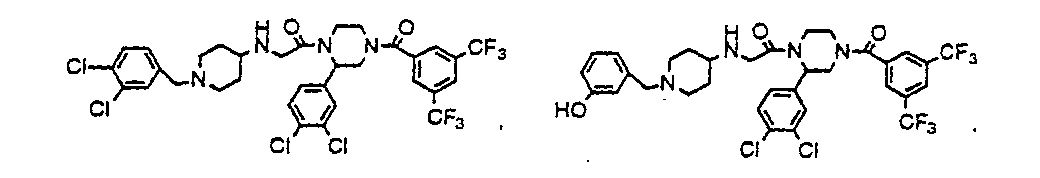 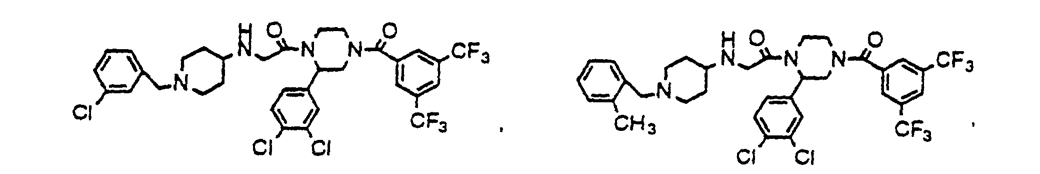 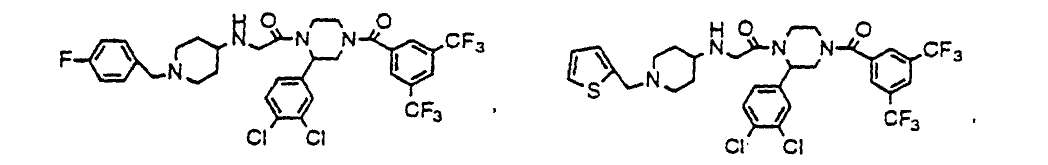 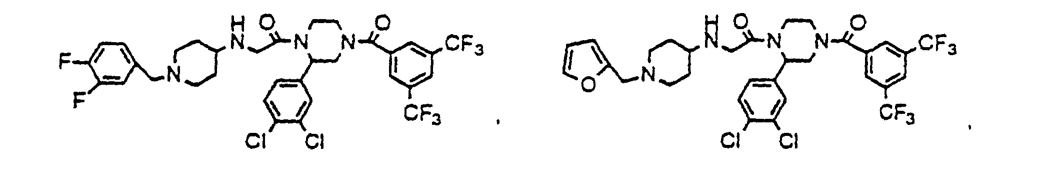 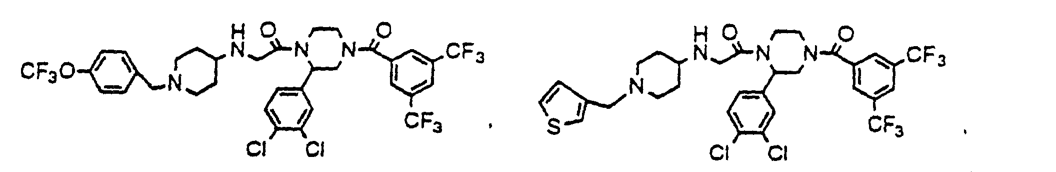 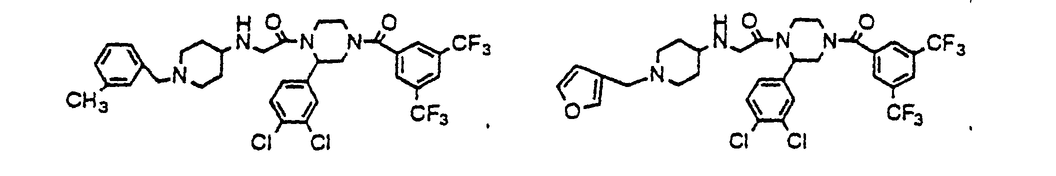 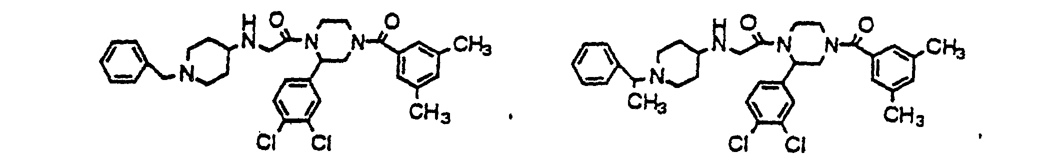 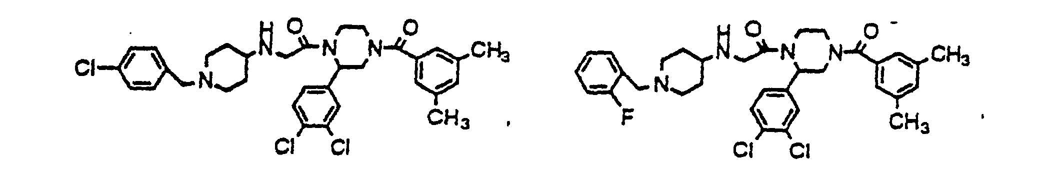 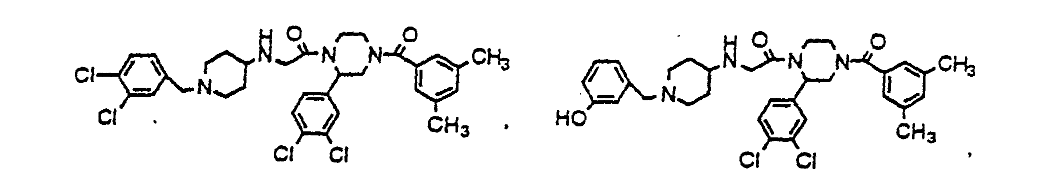 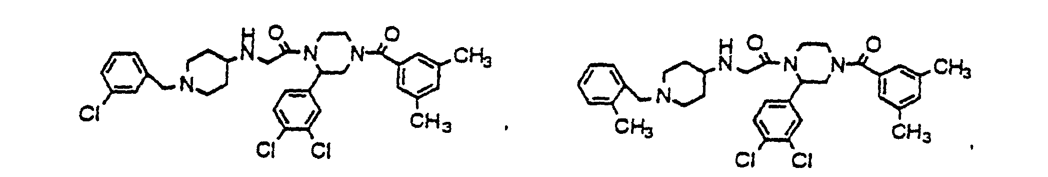 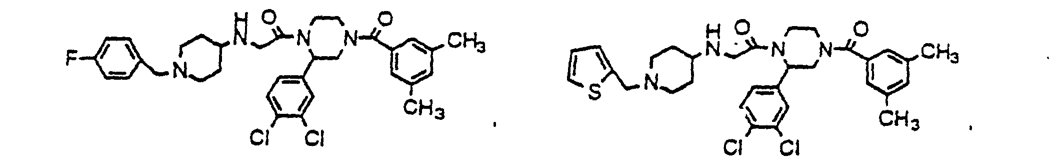 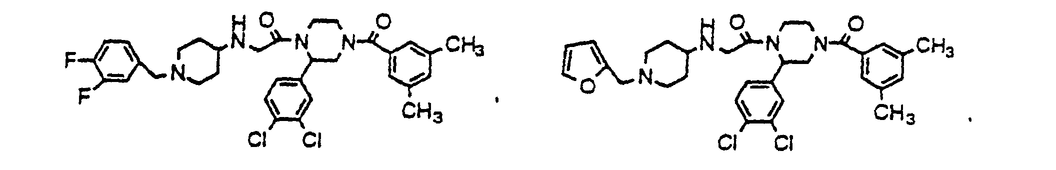 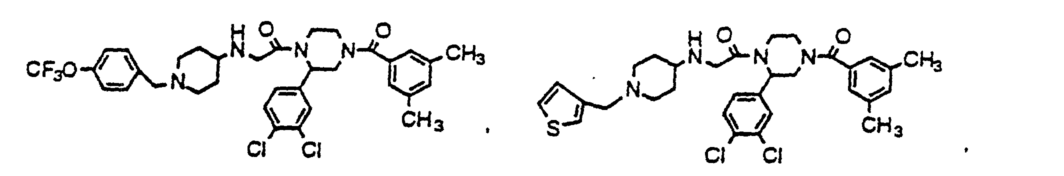 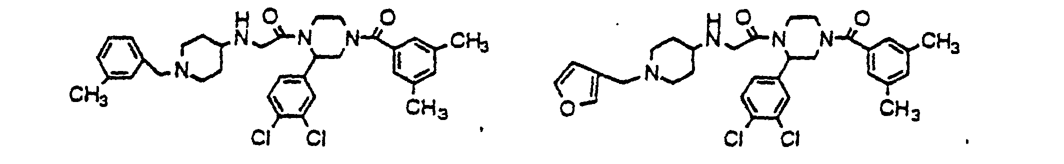 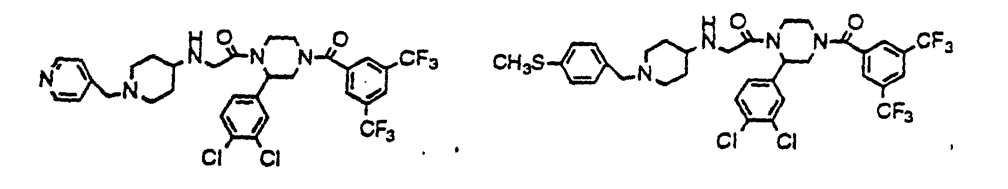 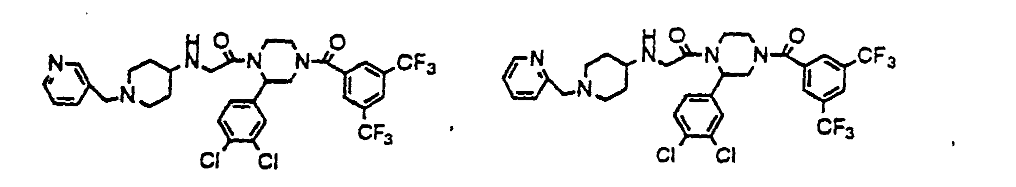 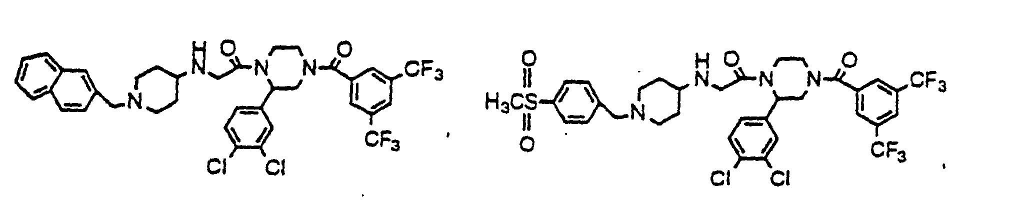 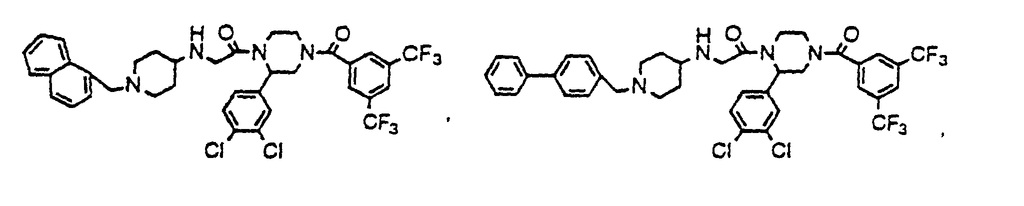 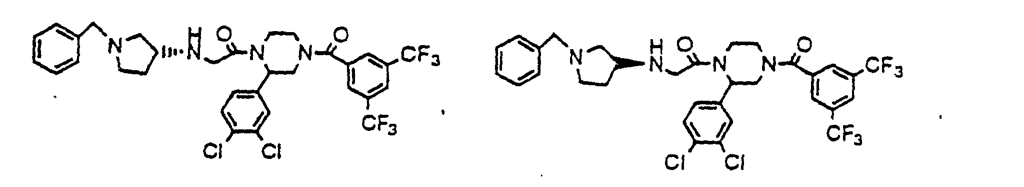 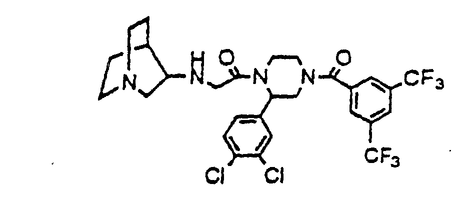 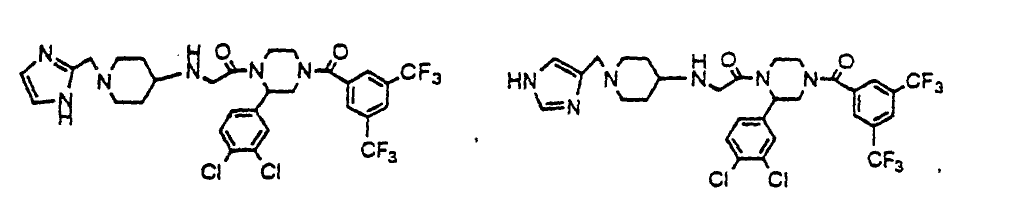 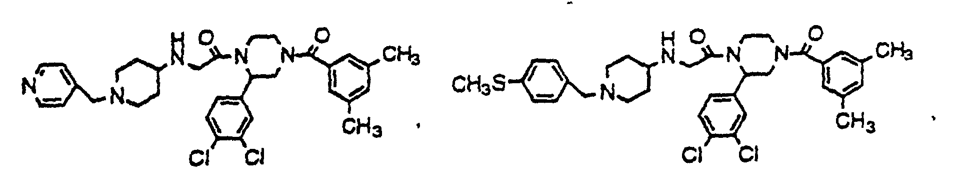 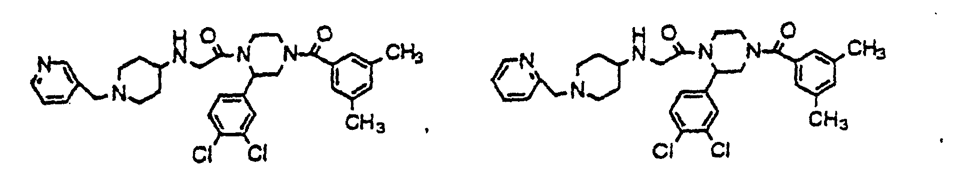 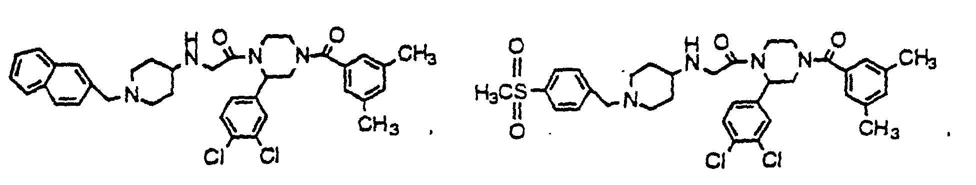 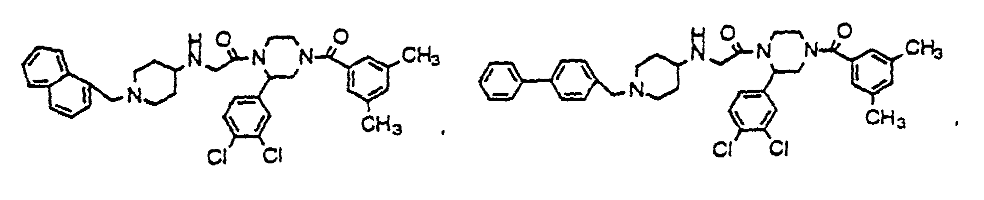 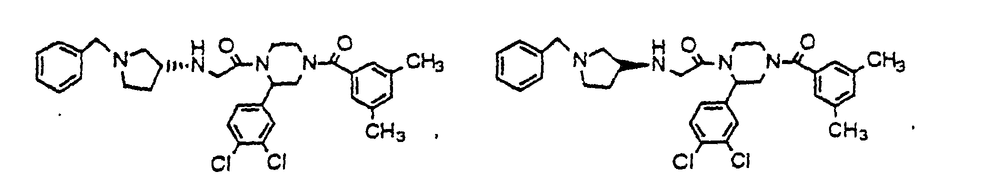 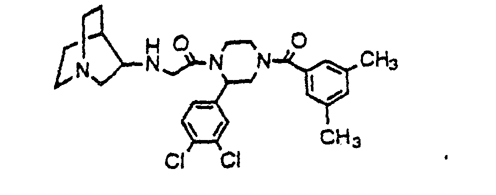 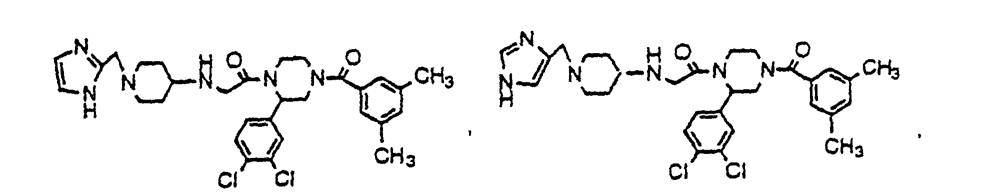 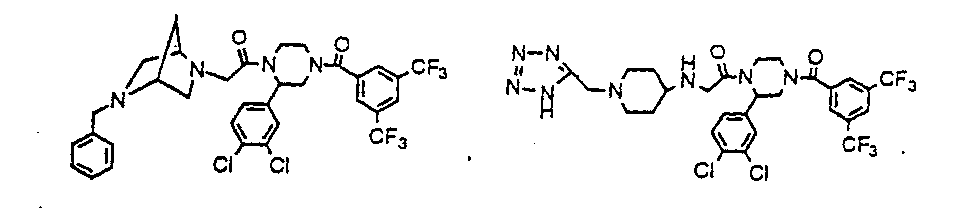 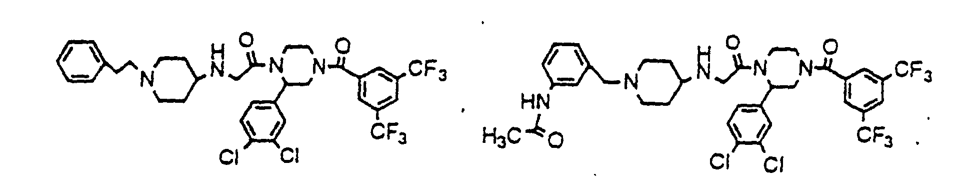 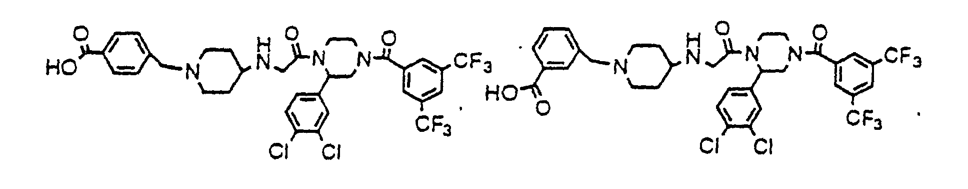 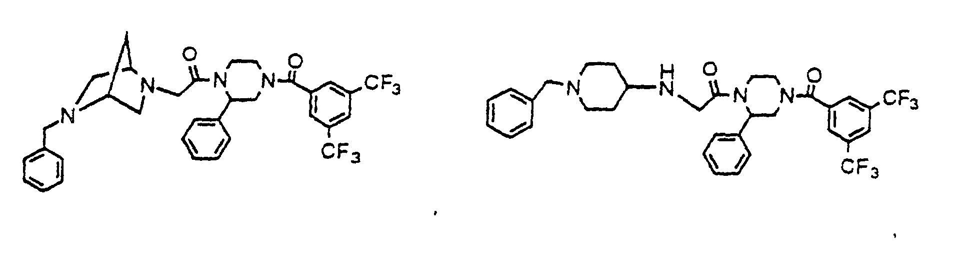 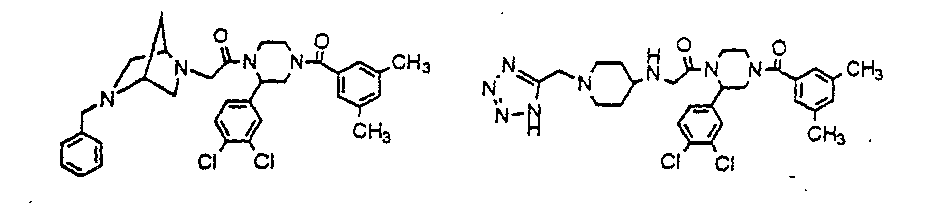 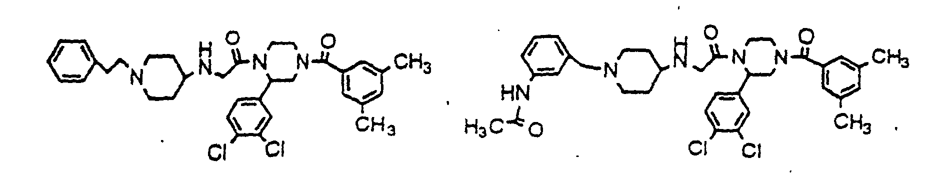 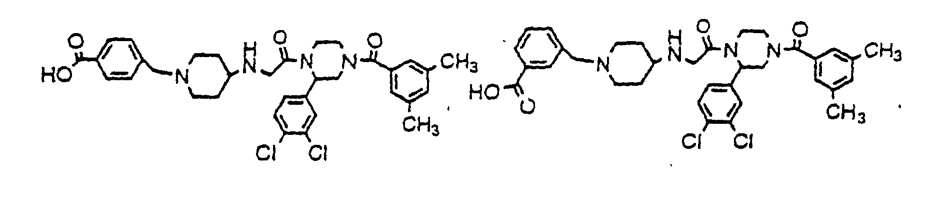 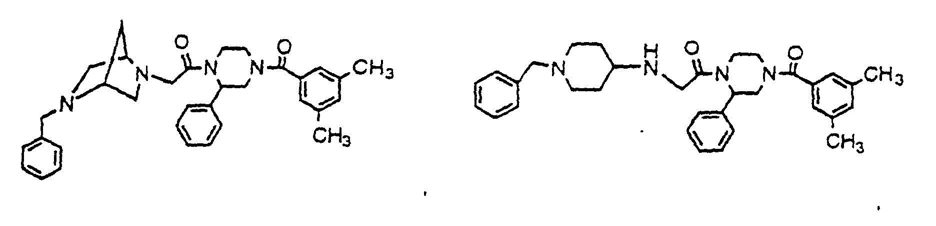 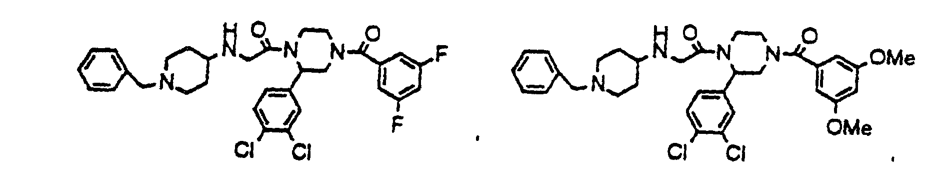 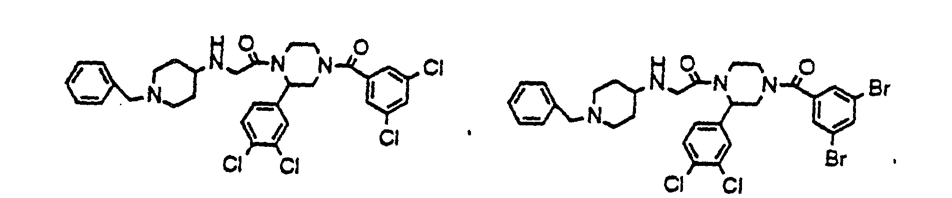 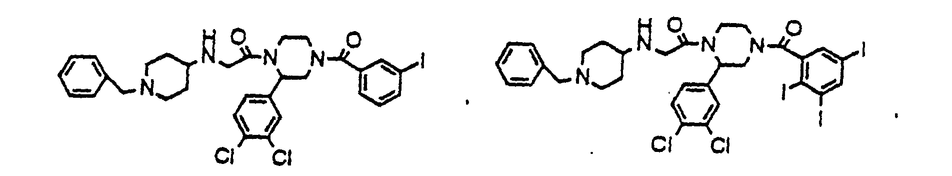 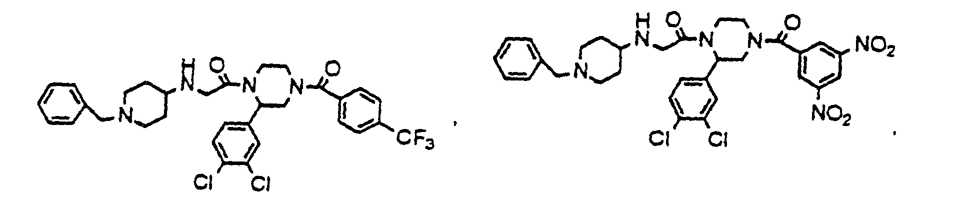 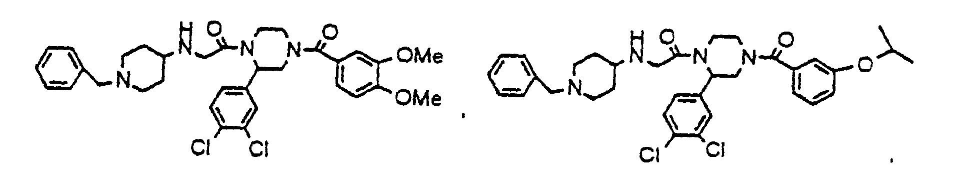 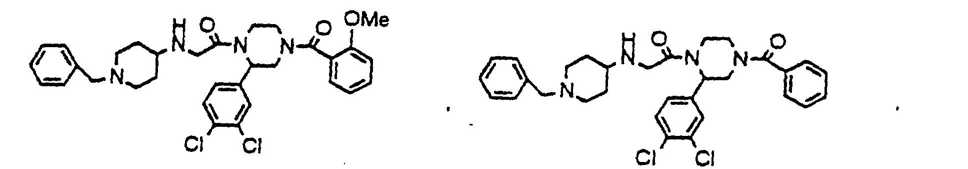 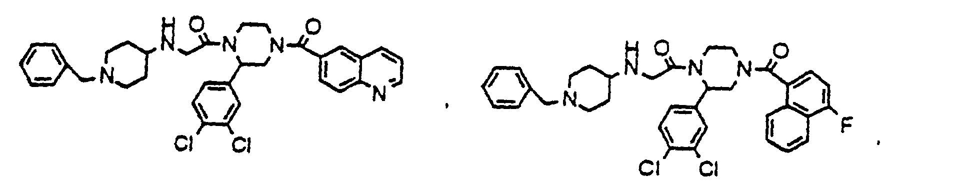 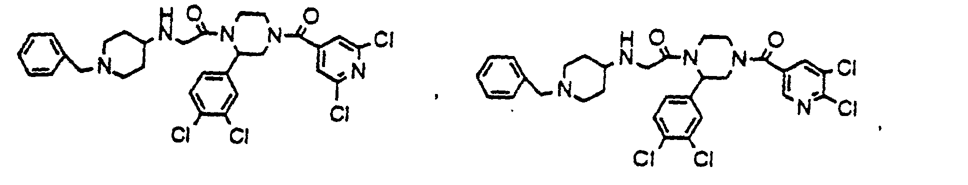 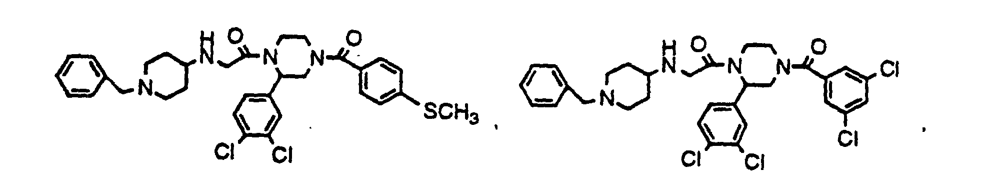 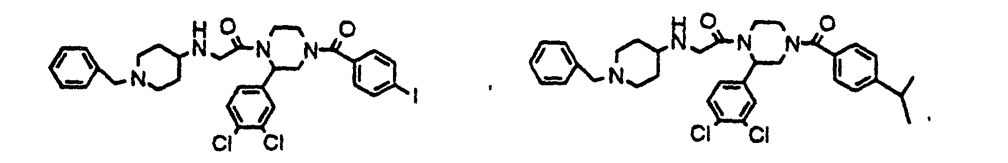 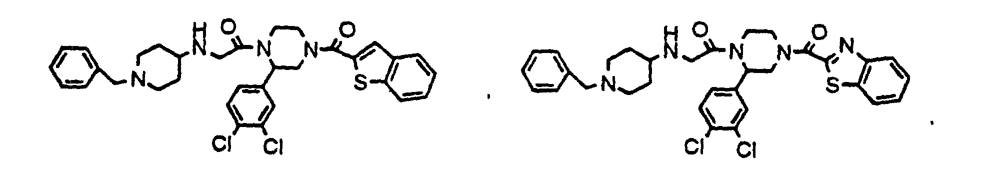 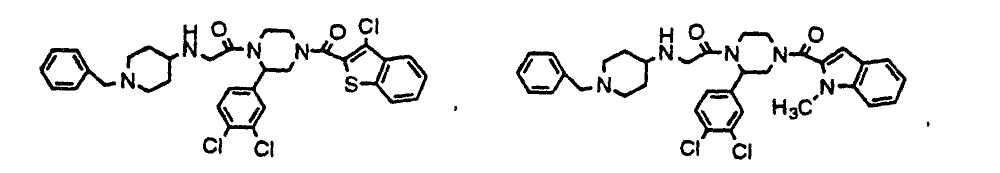 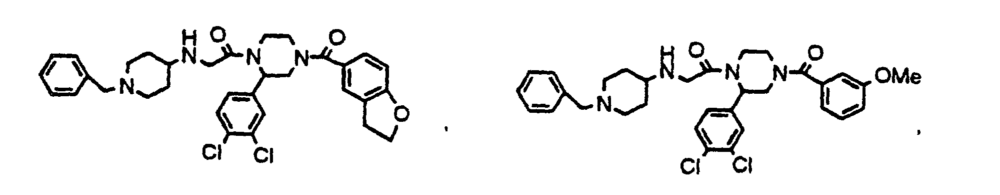 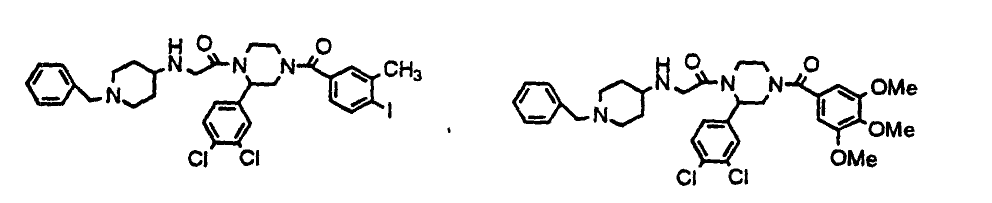 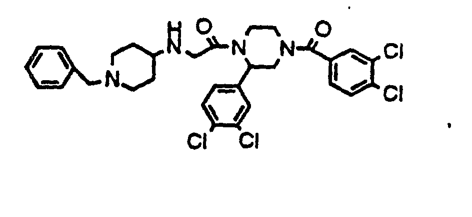 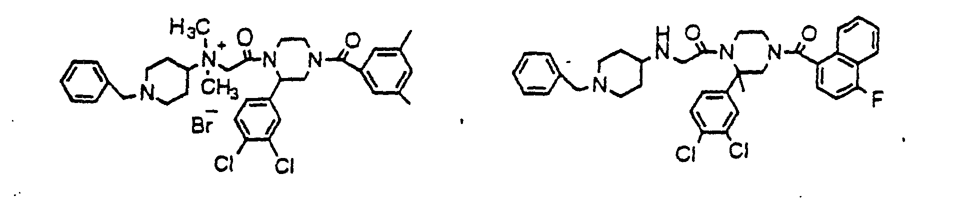 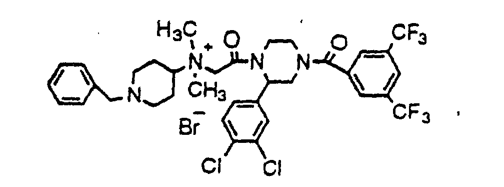 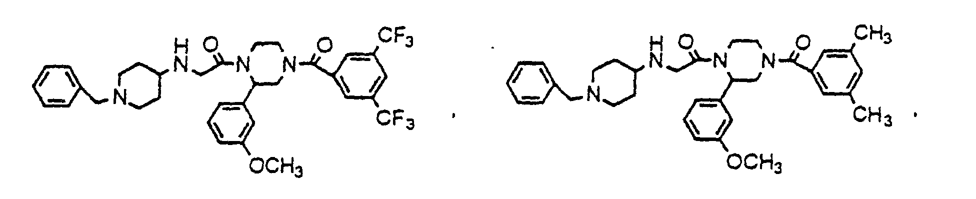 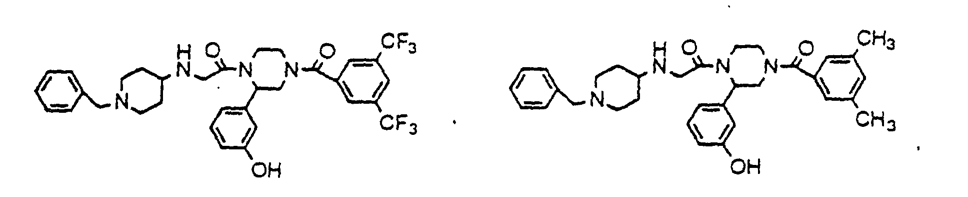 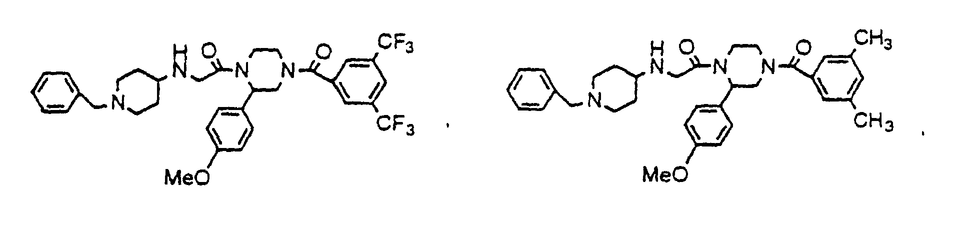 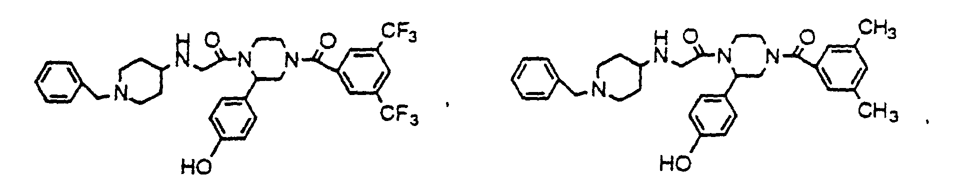 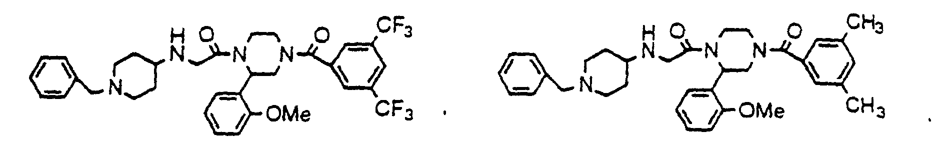 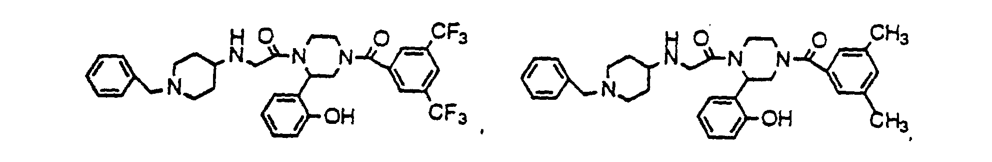 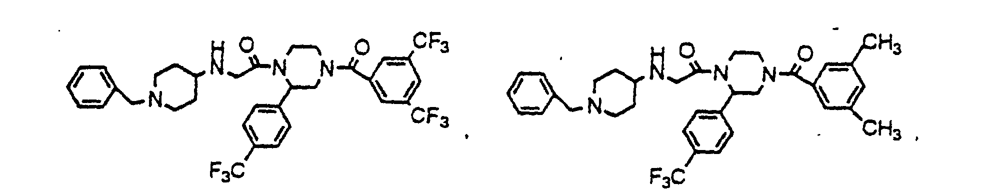 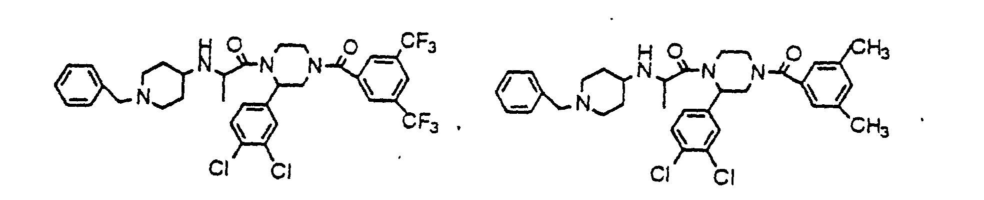 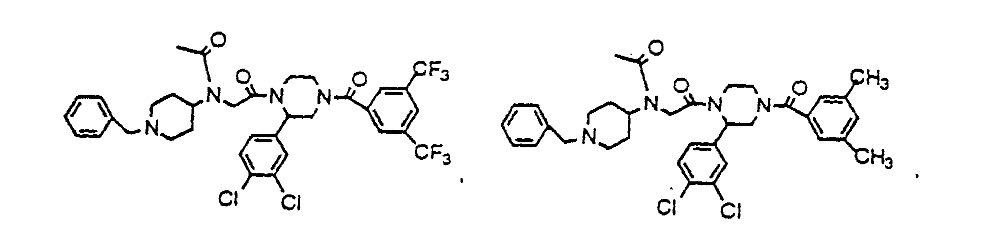 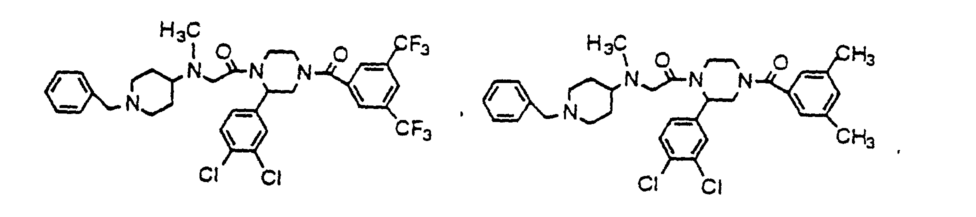 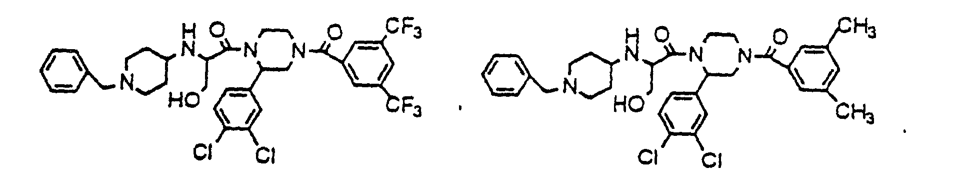 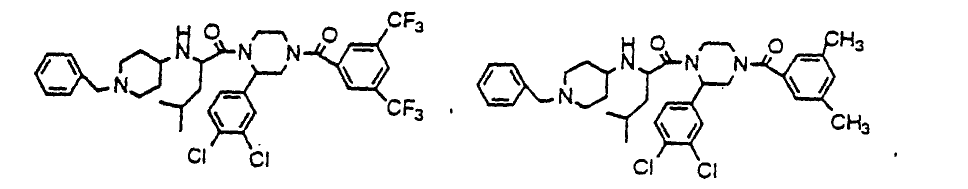 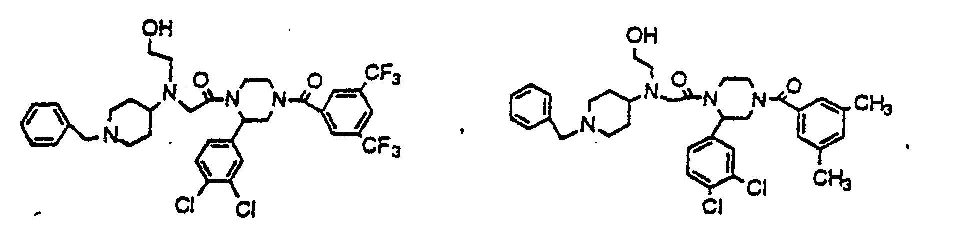 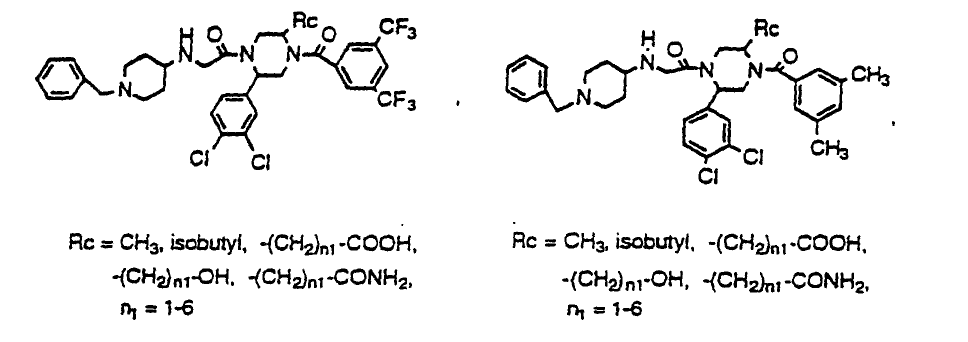 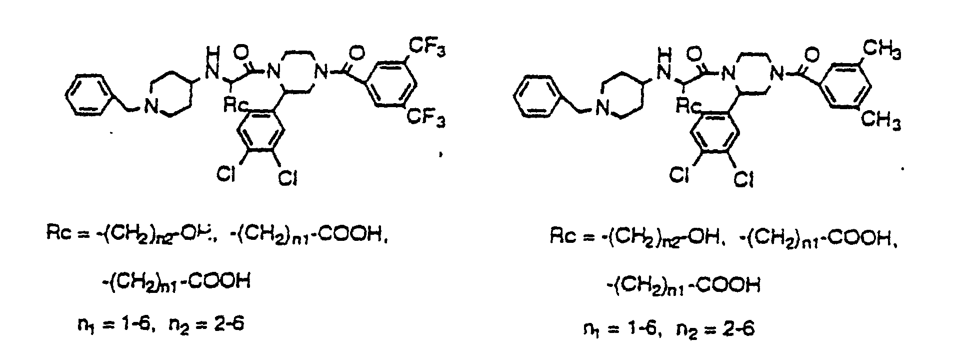 or a pharmaceutically acceptable salt thereof.

11. A composition comprising a neurokinin antagonist effective amount of a compound according to claim 1 and a pharmaceutically acceptable carrier material.

12. Use of a compound according to claim 1 in the manufacture of a medicament for inducing neurokinin antagonism.

13. Use according to claim 12 for treating chronic airway diseases; inflammatory diseases; migraine; central nervous system disorders; Down's syndrome; neuropathy; multiple sclerosis; ophthalmic disorders; auto immune disorders; Gl disorders and disorders of bladder function; circulatory disorders; Raynaud's disease; coughing and pain.

14. Use according to claim 13 for treating asthma, allergies, inflammatory bowel disease, psoriasis, fibrositis, osteoarthritis, rheumatoid arthritis, depression, psychosis, dementia, Alzheimer's disease, conjunctivitis, graft rejection, systemic lupus erythematosus, Crohn's disease, ulcerative colitis and angina.

## Patentansprüche

1. Verbindung der Formel: 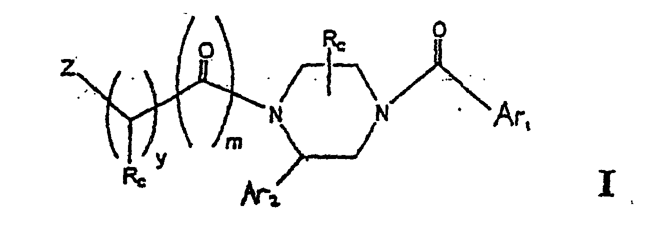
m ist 1, und y ist 1 bis 3; oder m ist 2, und y ist 0; und mit der weiteren Maßgabe, dass in der 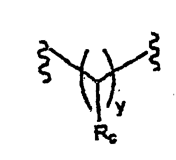 -Einheit nicht mehr als ein R_{c} von H verschieden ist,
jedes R_{c} ist unabhängig H, C₁-C₆ alkyl, -(CH₂)ₙ₁-R4, wobei n₁ 1 bis 6 ist;
R₄ -ORₐ, 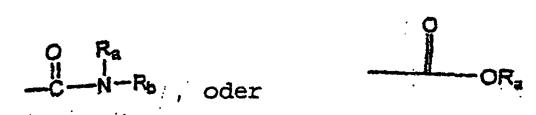 ist;
jedes Rₐ und R_{b} ist unabhängig ausgewählt aus der Gruppe bestehend aus H, C₁ bis C₆ Alkyl, Phenyl, Phenyl substituiert mit R¹, R² und/oder R³; Benzyl, Benzyl substituiert mit R¹, R², und/oder R³; Allyl; oder, wenn Rₐ und R_{b} an dasselbe Stickstoffatom gebanden sind, Rₐ und R_{b} zusammen mit dem Stickstoff, an welchen sie gebunden sind, einen 4 bis 7 gliedrigen Ring bilden;
wobei jedes R₁ und R₂ unabhängig H, C₁ bis C₆ Alkyl, CF₃, C₂F₅, Cl, Br, I, F, NO₂, ORₐ, CN, NRₐR_{b}, 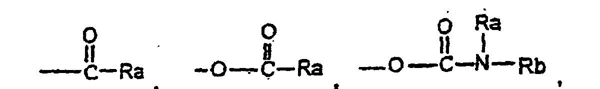 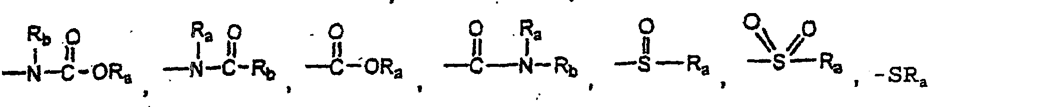 und 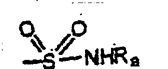 ist; und wobei Rₐ nicht H ist in 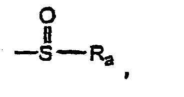 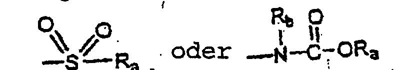 oder, wenn R₁ und R₂ sich an benachbarten Kohlenstoffatomen eines Ringes befinden, können sie 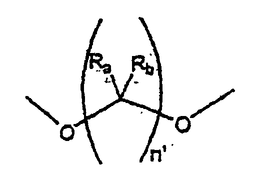 bilden, wobei n' 1 oder 2 ist;
und jedes R₃ unabhängig H, C₁ bis C₆-Alkyl, CF₃, C₂F₅, 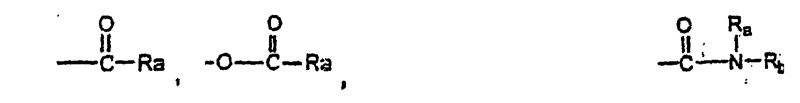 Cl, Br, I, oder F, ORₐ, OCF₃, oder Phenyl ist;
Ar₁ ist R₁-, R₂-, R₃-substituiertes Heteroaryl, wobei Heteroaryl ausgewählt ist aus Pyridyl, Benzothienyl, Benzofuranyl, Benzothiazolyl, Indolyl oder Quinolyl, 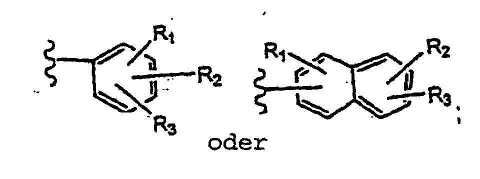
Ar₂ ist 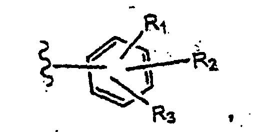
Z ist 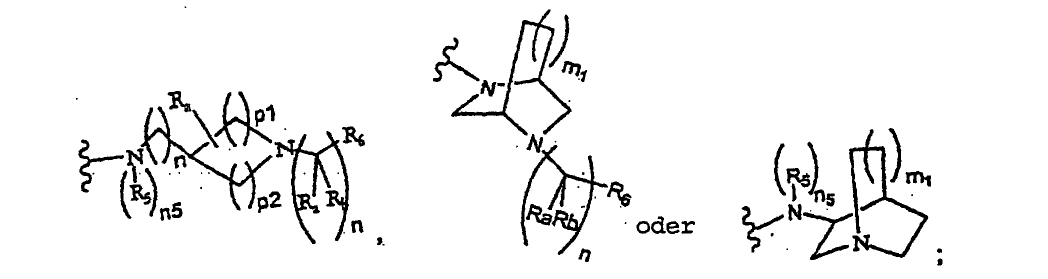 wobei jedes R₅ unabhängig ausgewählt ist aus der Gruppe bestehend aus 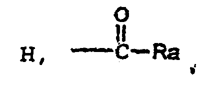 C₁ bis C₆ Alkyl; mit der Maßgabe, dass, wenn R₅ C₁ bis C₆ Alkyl ist, zwei R₅ an den Stickstoff gebunden sein können, um ein quartäres Salz zu bilden; n₅ ist 1, oder n₅ ist 2 mit der Maßgabe 1-, dass jedes R₅ unabhängig C₁ bis C₆ Alkyl ist; und m₁ ist 0 oder 1;
p₁ und p₂ sind jeweils unabhängig 1 bis 4 mit der Maßgabe, dass p₁ und p₂ zusammen addiert 2 bis 6 sind;
R₆ ist Thienyl, Furanyl, Pyridyl, Tetrazolyl, Imidazolyl, 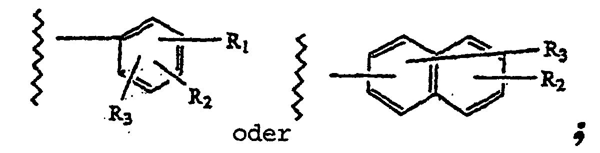
n ist 0 bis 2;
oder ein pharmazeutisch akzeptables Salz von der Verbindung.

2. Verbindung nach Anspruch 1, wobei m 1 ist und y 1 ist.

3. Verbindung nach Anspruch 1, wobei Z 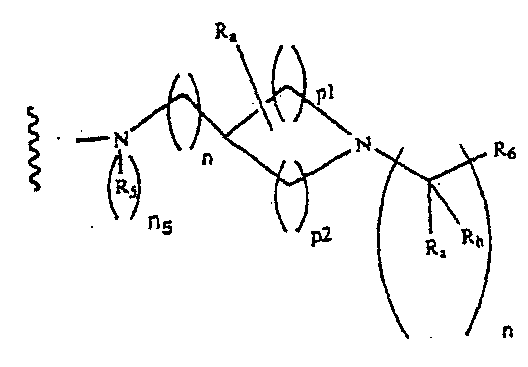 ist.

4. Verbindung nach Anspruch 1, wobei Z 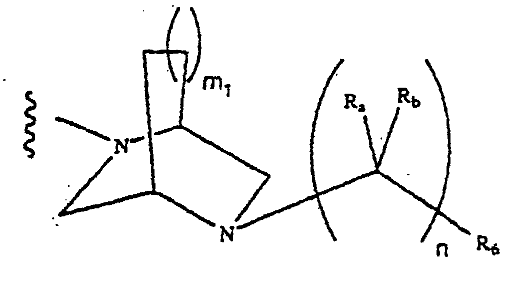 ist.

5. Verbindung nach Anspruch 1, wobei Z 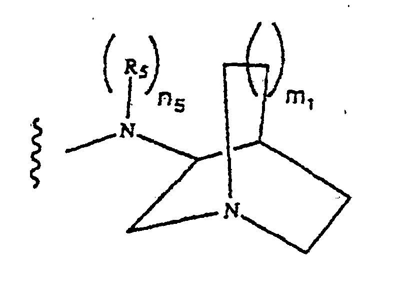 ist.

6. Verbindung nach Anspruch 1 der Formel

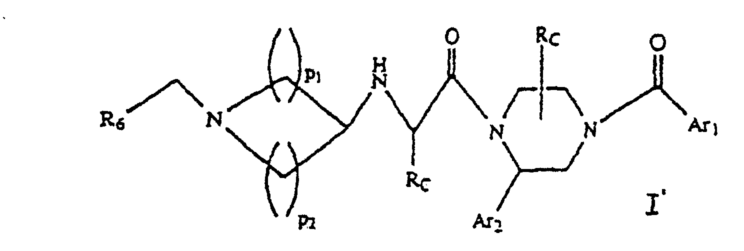

7. Verbindung nach Anspruch 6, wobei R_{c} H ist, p₁ und p₂ 2 sind, Ar₁ und Ar₂ beide 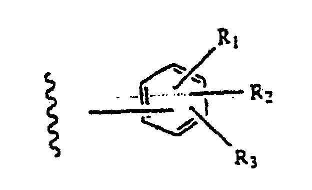 sind.

8. Verbindung nach Anspruch 6, wobei R₆ Thieny, Furanyl, Pyridyl, Tetrazolyl, Imidazolyl, 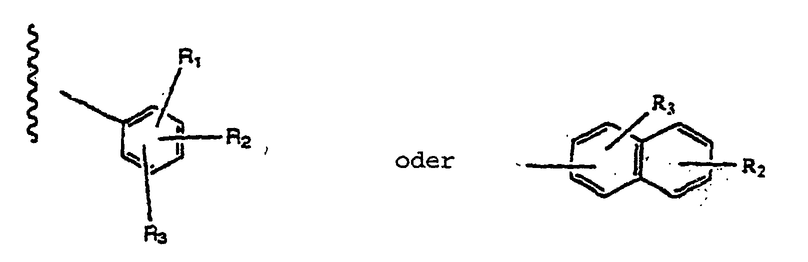 ist.

9. Verbindung nach Anspruch 4, wobei R₆ 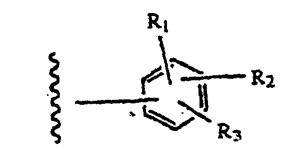 ist.

10. Verbindung nach Anspruch 1, ausgewählt aus der Gruppe bestehend aus 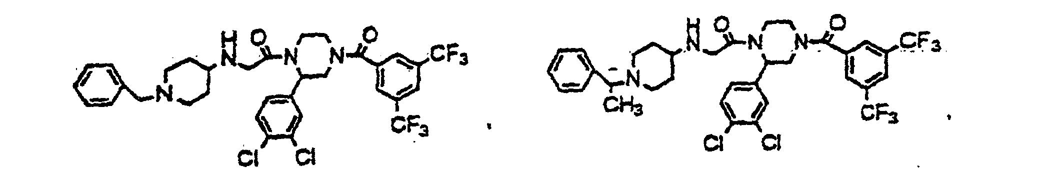 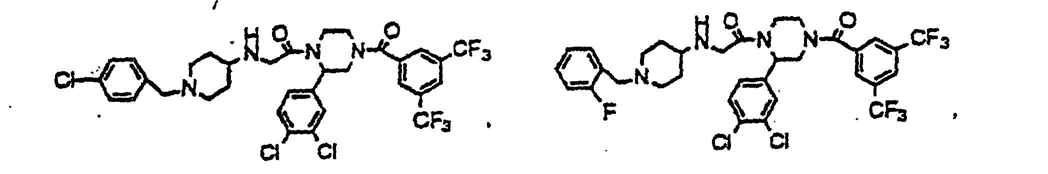 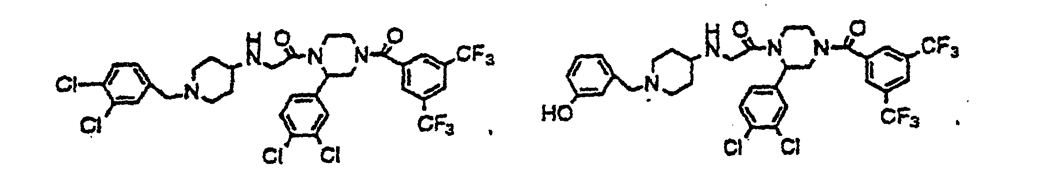 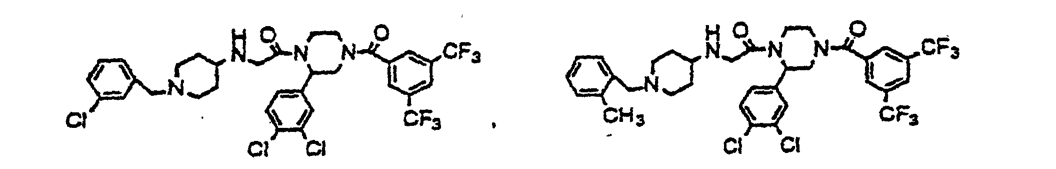 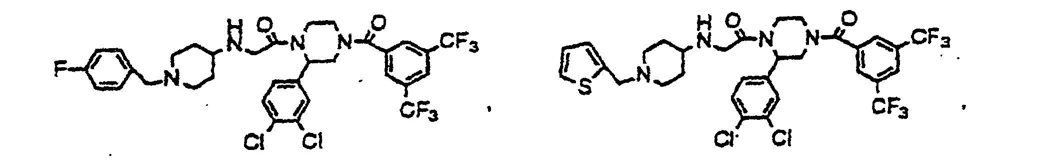 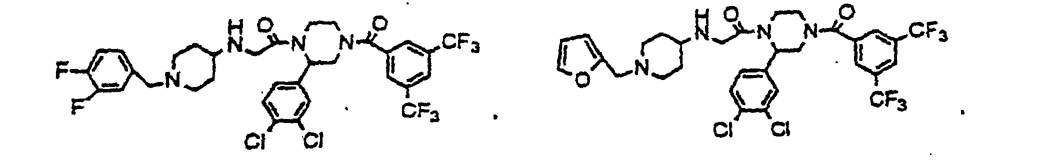 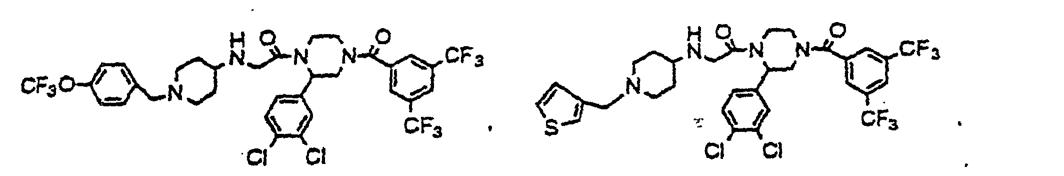 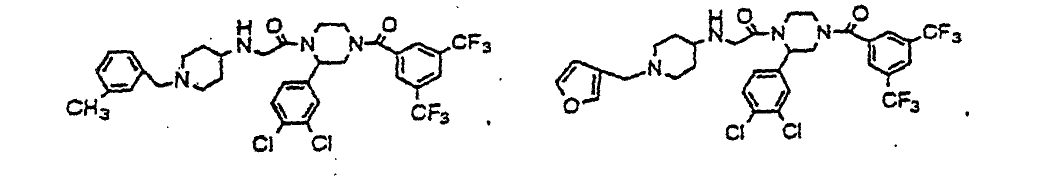 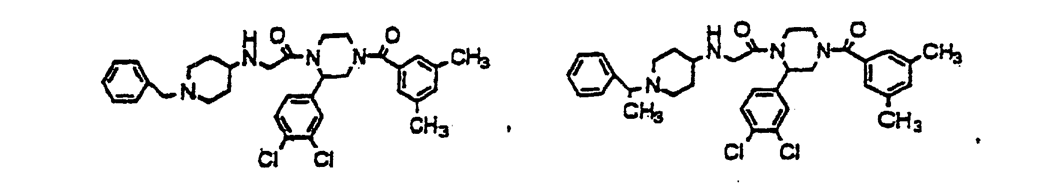 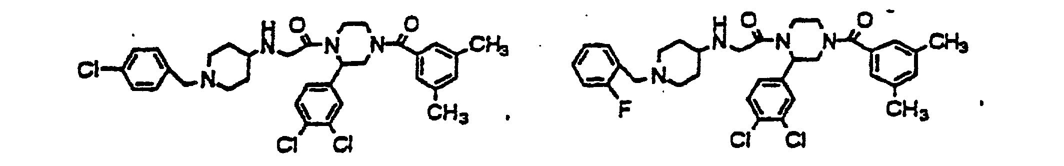 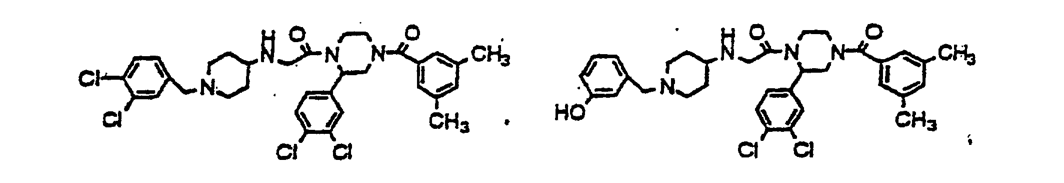 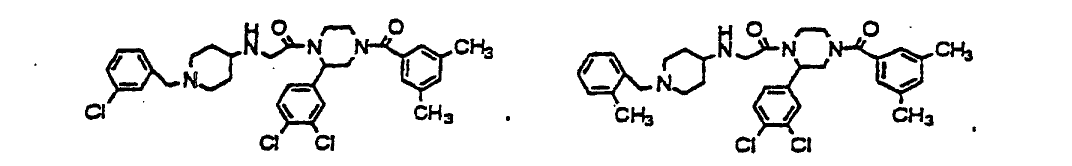 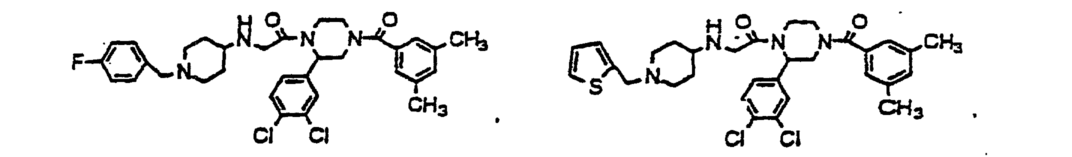 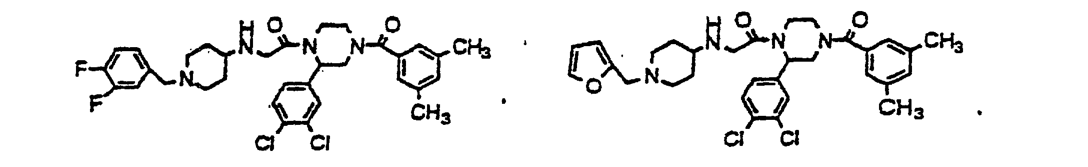 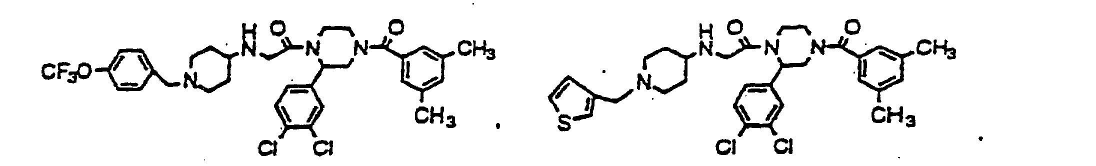 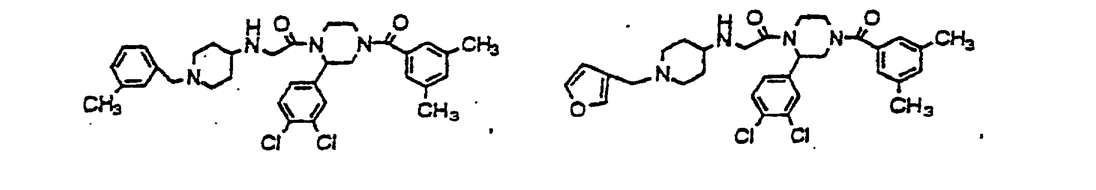 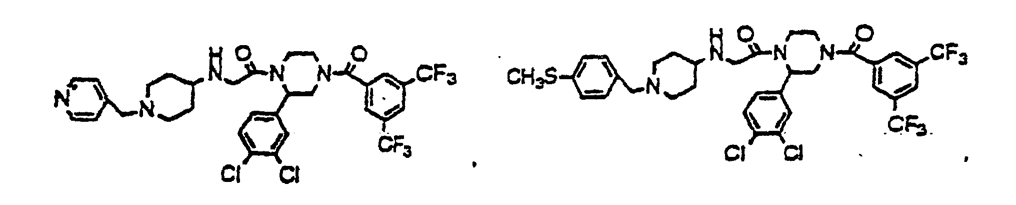 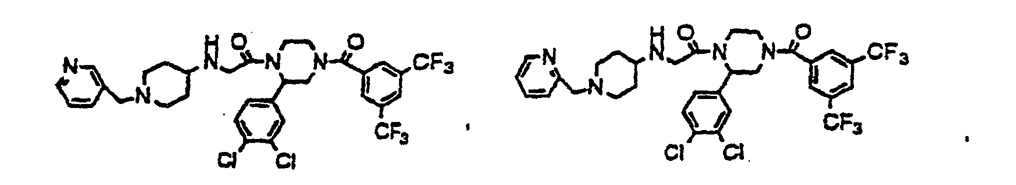 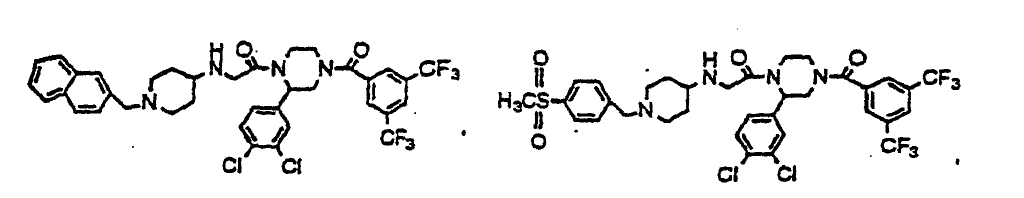 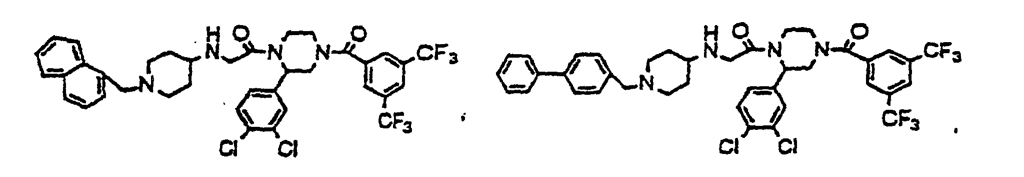 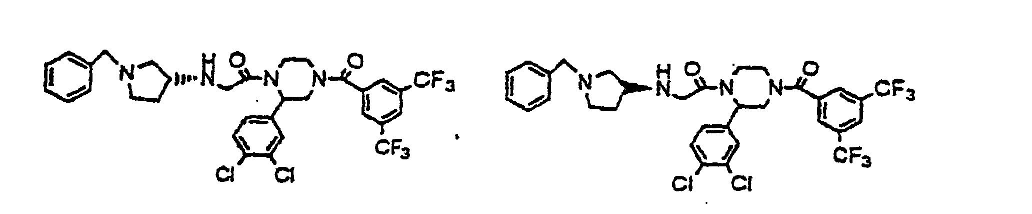 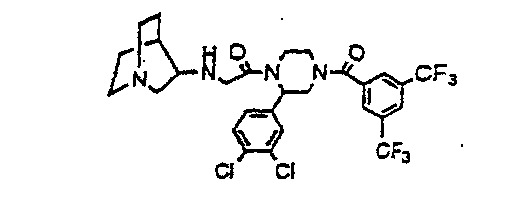 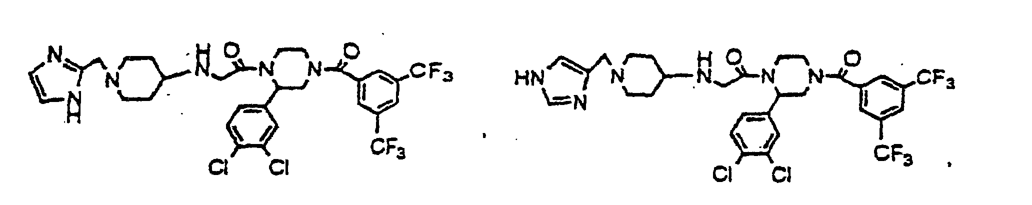 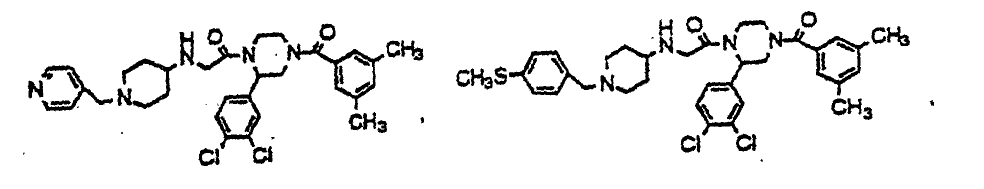 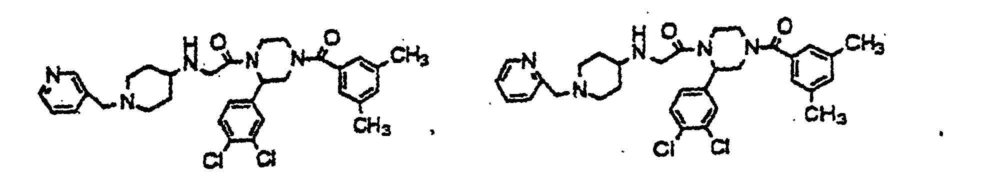 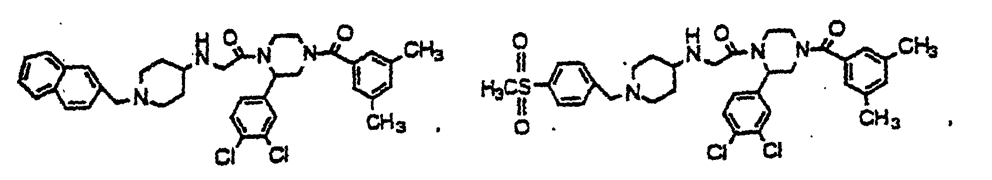 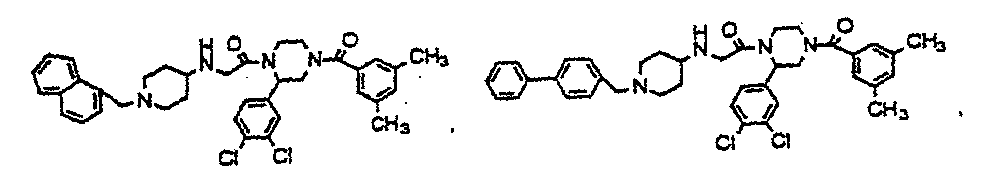 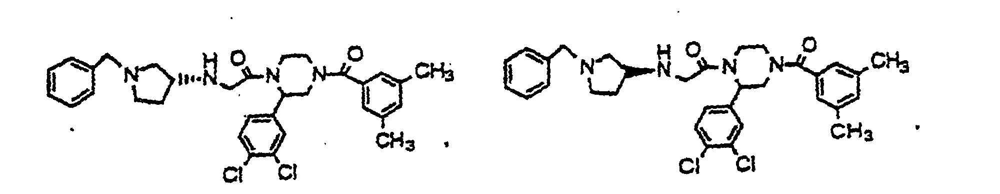 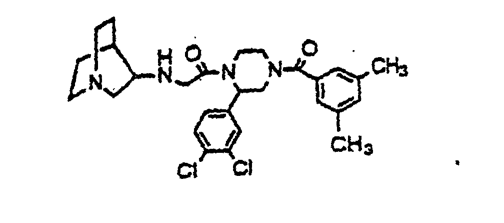 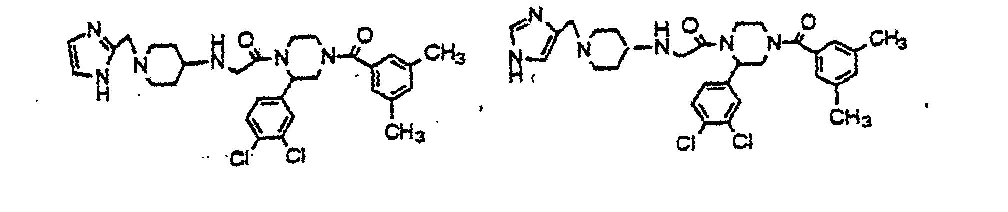 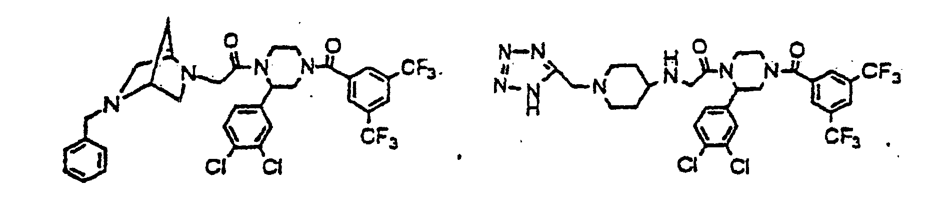 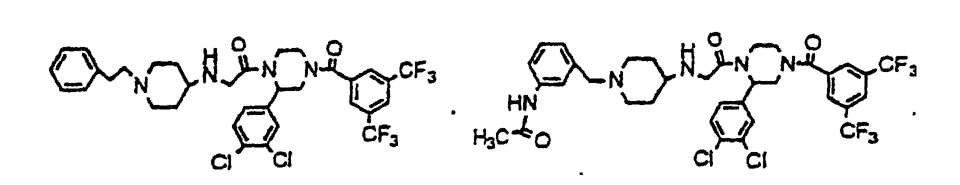 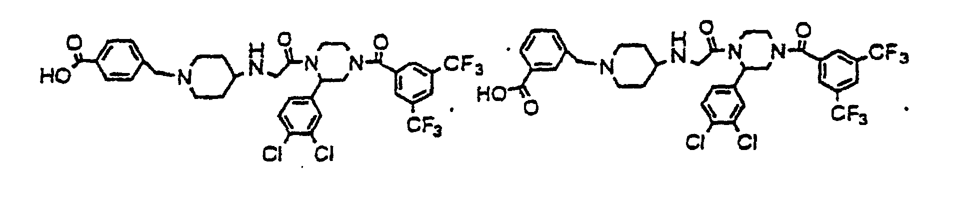 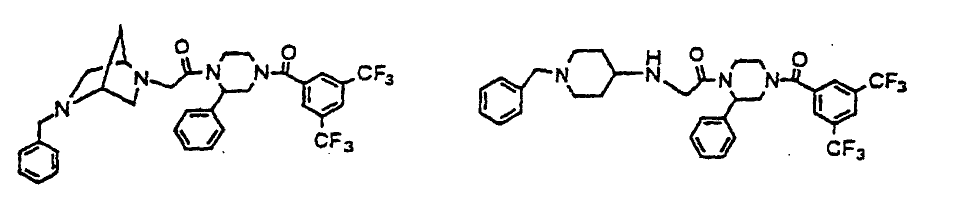 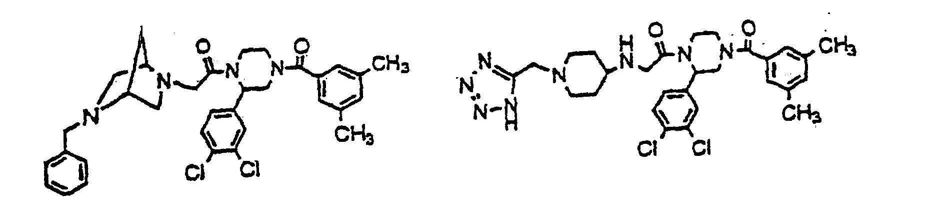 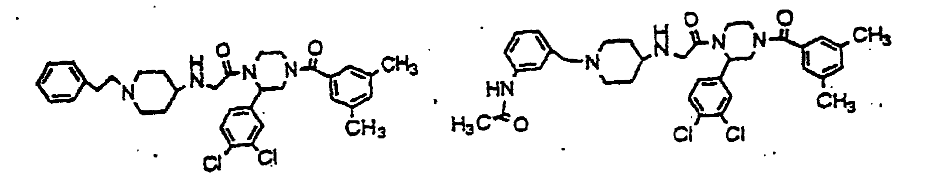 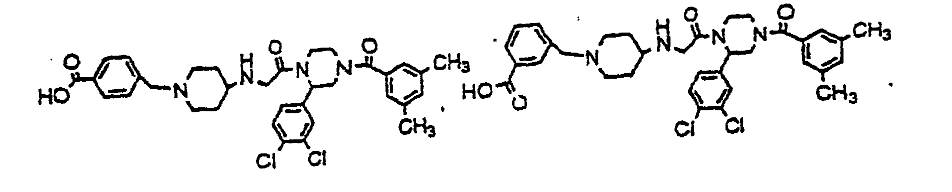 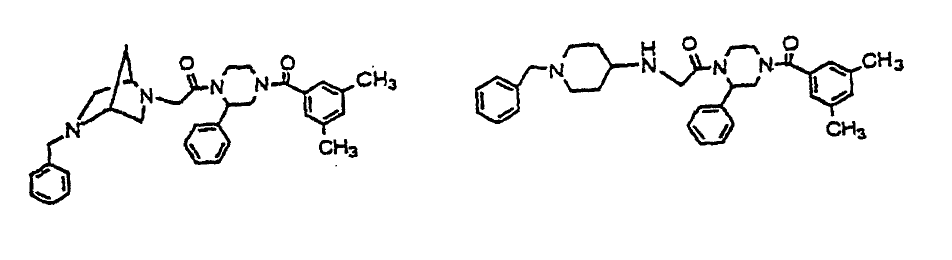 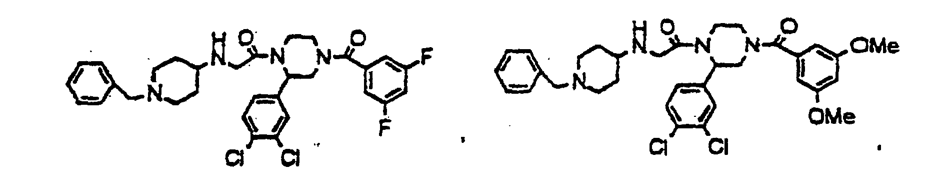 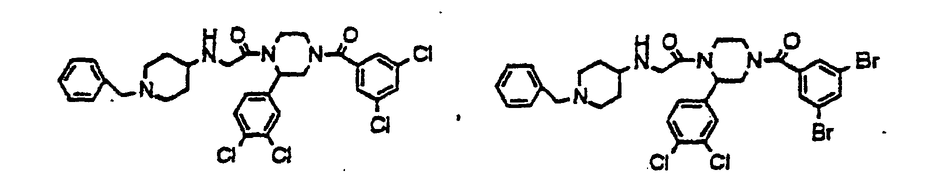 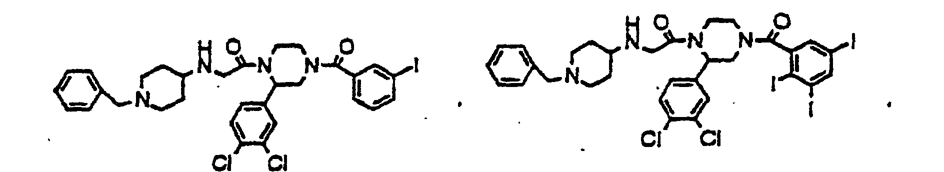 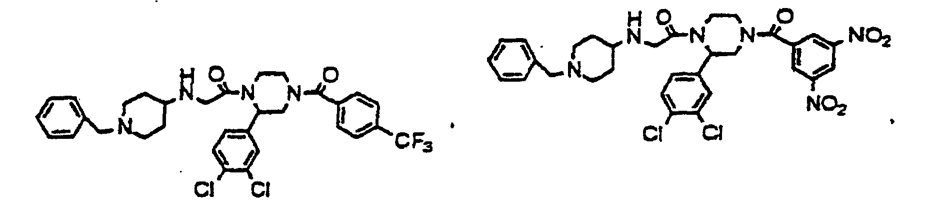 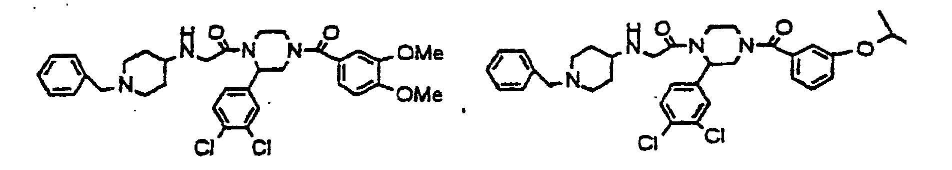 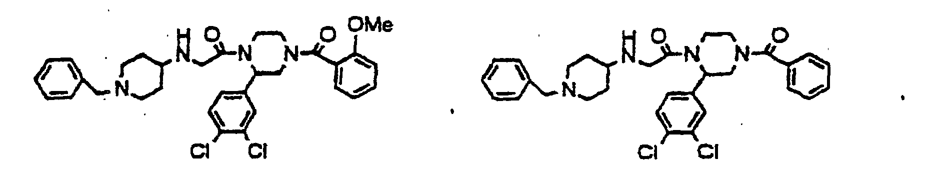 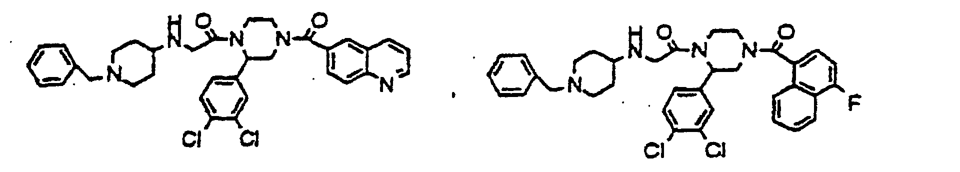 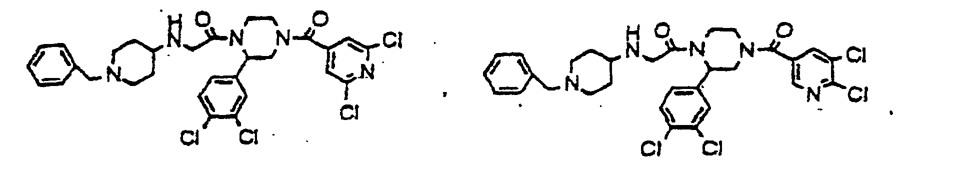 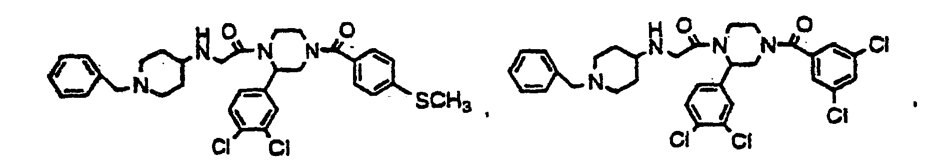 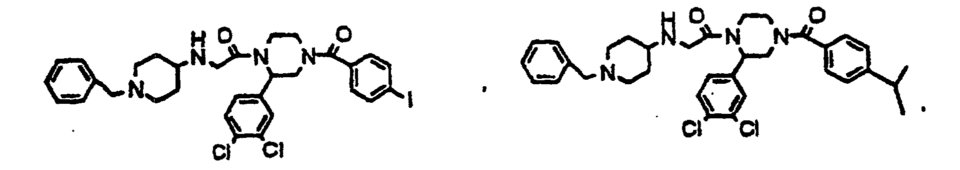 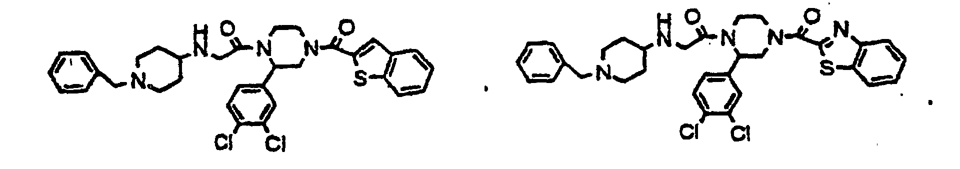 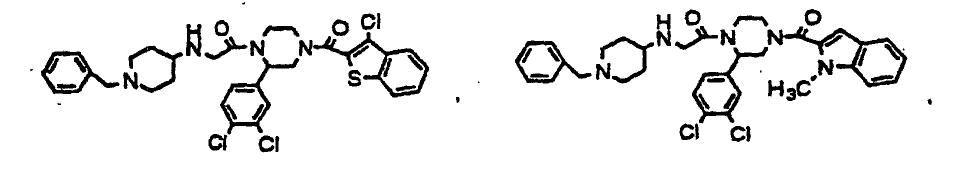 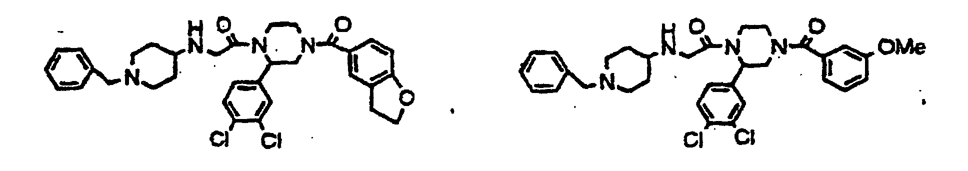 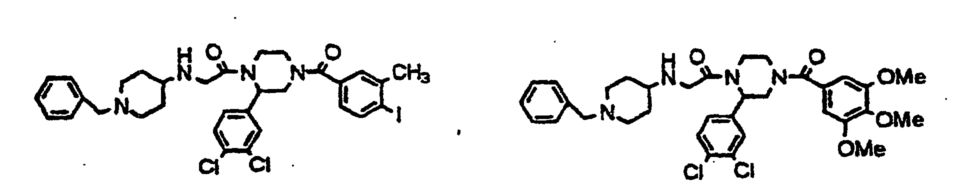 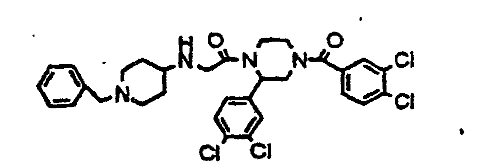 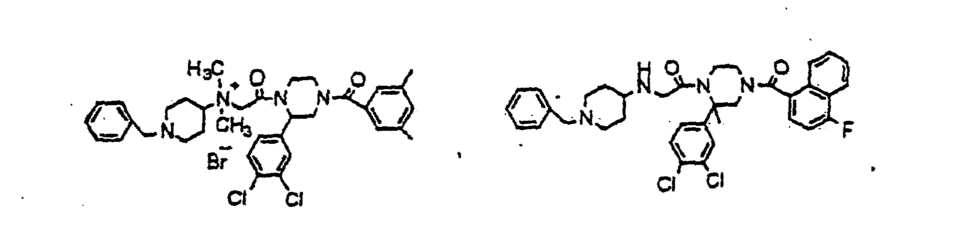 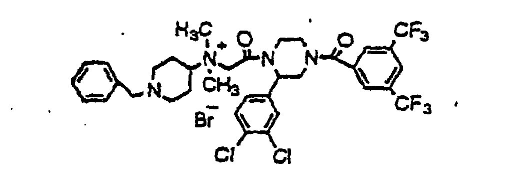 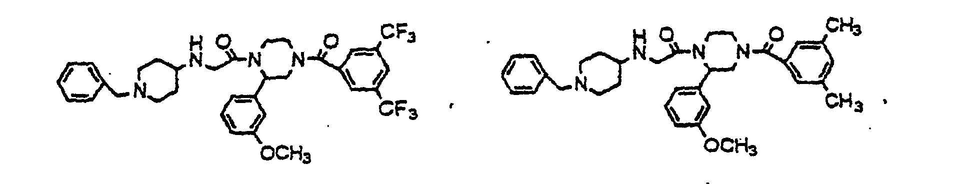 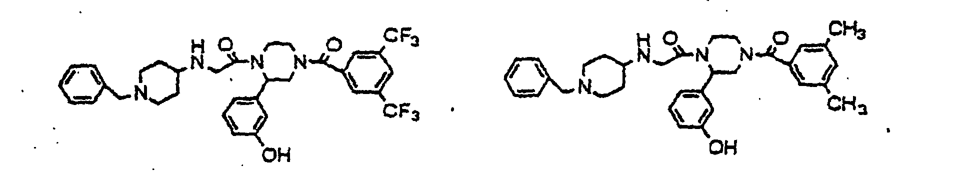 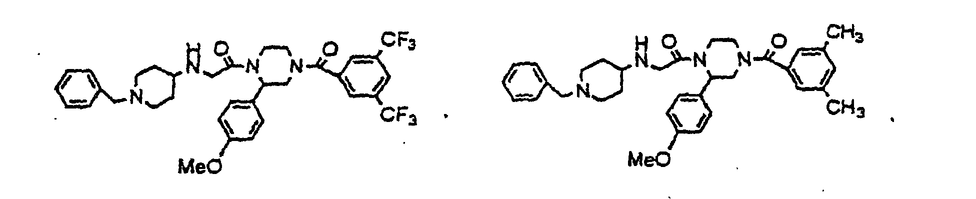 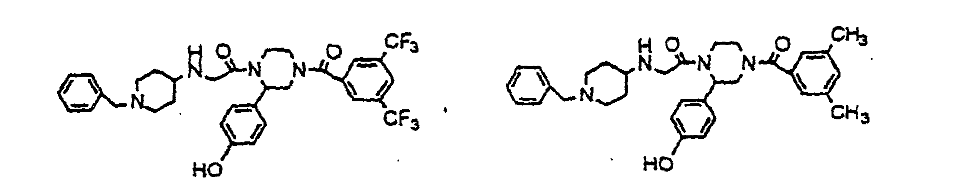 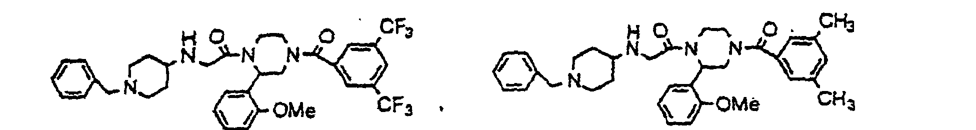 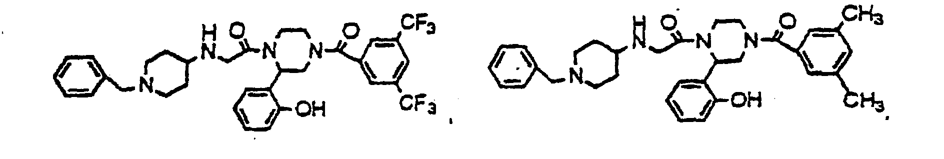 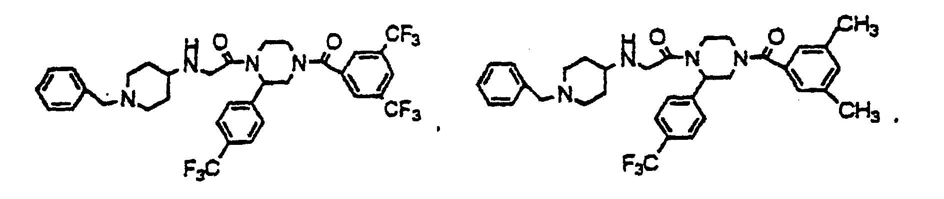 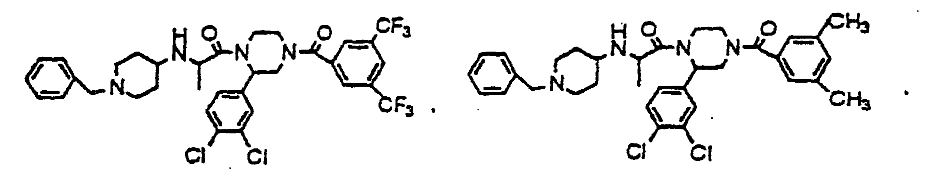 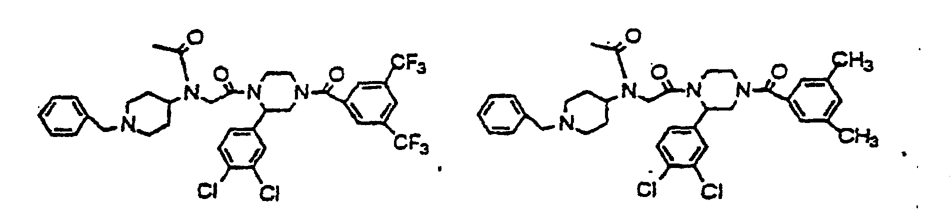 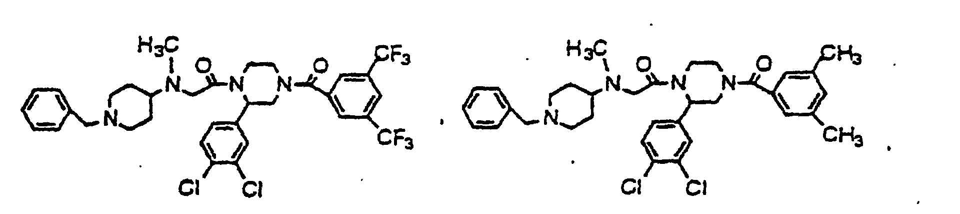 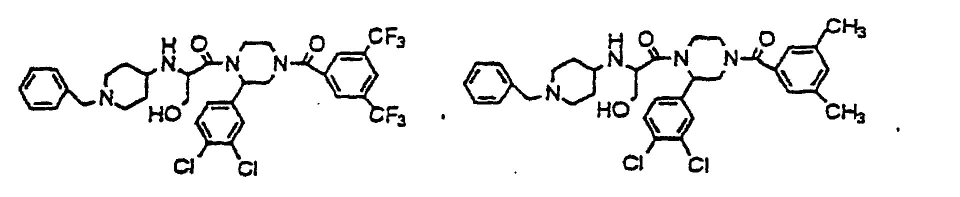 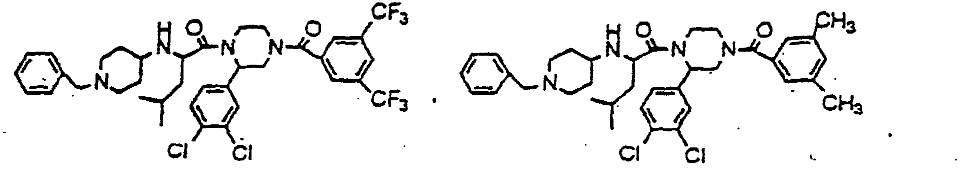 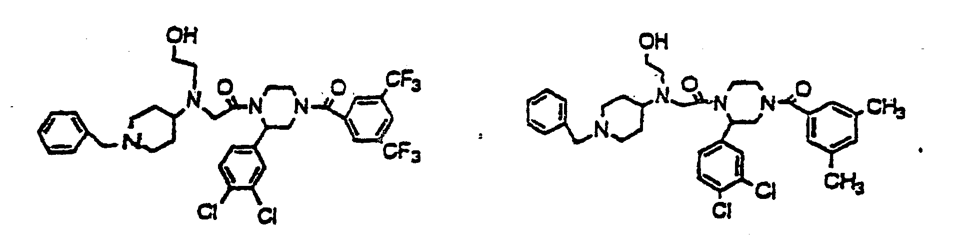 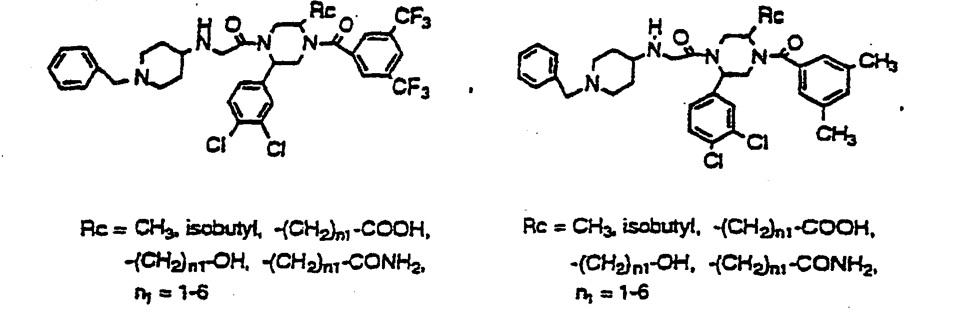 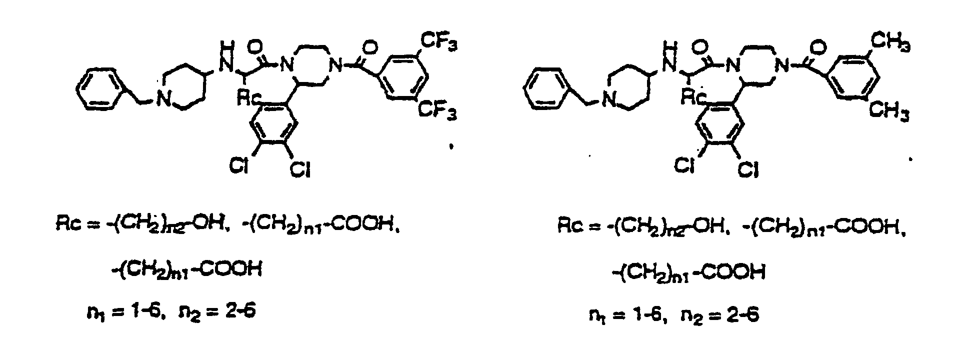 oder ein pharmazeutisch akzeptables Salz davon.

11. Zusammensetzung, die eine Neurokinin-antagonistisch wirksame Menge einer Verbindung gemäß Anspruch 1 und ein pharmazeutisch akzeptables Trägermaterial enthält.

12. Verwendung einer Verbindung gemäß Anspruch 1 bei der Herstellung eines Medikaments zur Induzierung von Neurokinin-Antagonismus.

13. Verwendung nach Anspruch 12 zur Behandlung von chronischen Atemwegskrankheiten; Entzündungskrankheiten; Migräne; Erkrankungen des zentralen Nervensystems; Down's Syndrom, Neurophatie; multipler Sklerose; Augenkrankheiten; Auto-Immun-Erkrankungen; Gastro-Intestinal-Erkrankungen und Fehlfunktionen der Blasenfunktion; Kreislauferkrankungen; Raynaud's-Krankheit; Husten und Schmerzen.

14. Verwendung nach Anspruch 13 zur Behandlung von Asthma, Allergien, entzündlichen Darmerkrankungen, Schuppenflechte, Fibrositis, Osteoarthritis, rheumatischer Arthritis, Depressionen, Psychose, Demenz, Alzheimer-Krankheit, Bindehautentzündung, Transplantatabstossung, systemischer Lupus Erythematosus, Crohn-Krankheit, ulcerativer Colitis und Angina.

## Revendications

1. Composé de formule 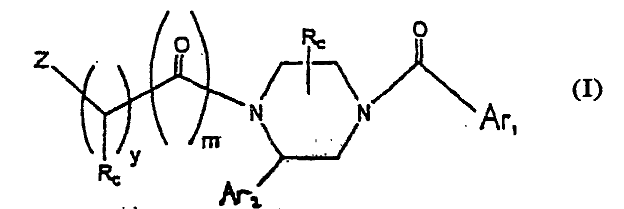 dans laquelle
m vaut 1 et y vaut de 1 à 3, ou bien m vaut 2 et y vaut 0 ;
sous réserve qu'au plus un seul R_{c} représente autre chose que H dans le fragment 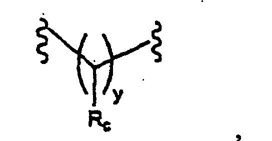 chaque R_{c} représente indépendamment H, allyle en C₁₋₆ ou -(CH₂)ₙ₁-R₄ où n1 vaut de 1 à 6 et R₄ représente -ORₐ, 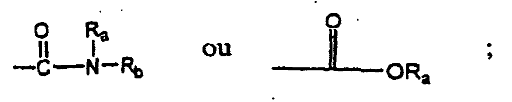
Rₐ et R_{b} sont choisis, chacun indépendamment, dans l'ensemble formé par H, alkyle en C₁₋₆, phényle, phényle à substituant(s) R₁, R₂ et/ou R₃, benzyle, benzyle à substituant(s) R₁, R₂ et/ou R₃, et allyle, ou bien, si Rₐ et R_{b} sont liés au même atome d'azote, ils constituent, conjointement avec cet atome d'azote auquel ils sont liés, un cycle comportant 4 à 7 chaînons ;
chaque R₁ et R₂ représénte indépendamment H, alkyle en C₁₋₆, CF₃, C₂F₅, Cl, Br, I, F, NO₂, -ORₐ, CN, -NRₐR_{b}, 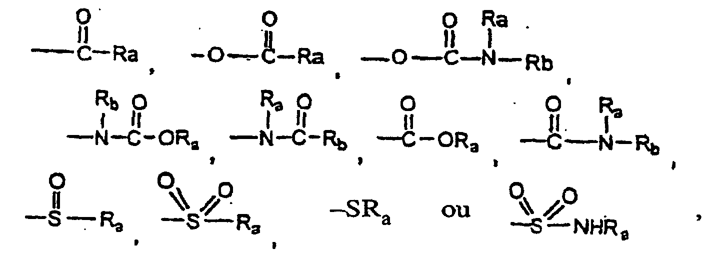
Rₐ ne représentant pas H dans 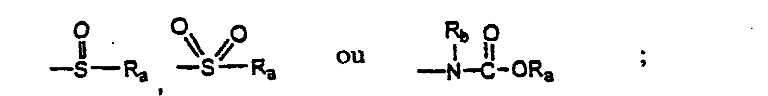 ou bien, si R₁ et R₂ sont liés à des atomes de carbone adjacents d'un cycle, ils peuvent former 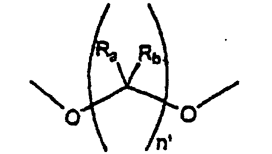 où n' vaut 1 ou 2 ;
et chaque R₃ représente indépendamment H, alkyle en C₁₋₆, CF₃, C₂F₅, Cl, Br, I, F, OCF₃, phényle, ORₐ, 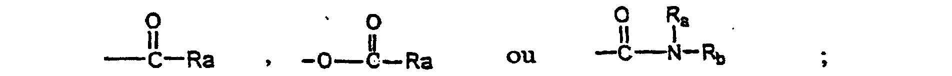
Ar₁ représente un groupe hétéroaryle à substituant(s) R₁, R₂ et R₃, ce groupe hétéroaryle étant choisi parmi les groupes pyridyle, benzothiényle, benzofuranyle, benzothiazolyle, indolyle et quinolyle,
ainsi que 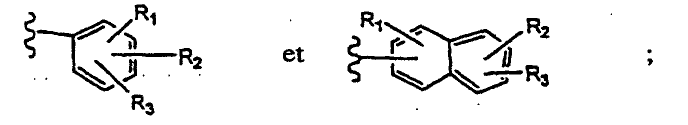
Ar₂ représente 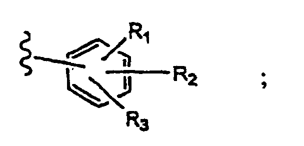
et Z représente 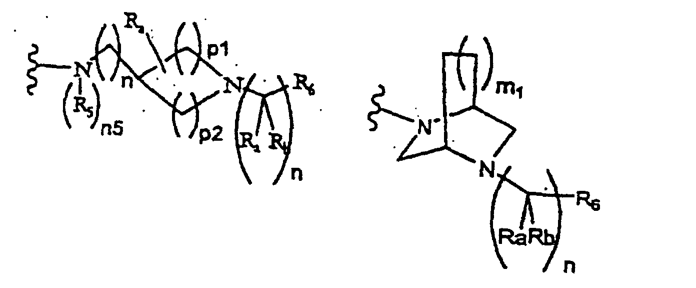 ou 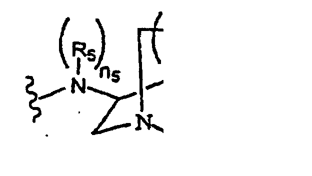 où chaque R₅ est indépendamment choisi parmi H, alkyle en C₁₋₆ et 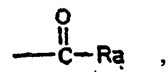 sous réserve que, quand R₅ représente un groupe alkyle en C₁₋₆, deux substituants R₅ peuvent être liés à l'atome d'azote pour former un sel quaternaire ;
n₅ vaut 1, mais n₅ peut valoir 2 à condition que chaque R₅ représente indépendamment un groupe allyle en C₁₋₆ ;
m₁ vaut 0 ou 1 ;
p₁ et p₂ valent chacun indépendamment de 1 à 4, sous réserve que la somme p₁ + p₂ vaille de 2 à 6 ;
R₆ représente un groupe thiényle, furanyle, pyridyle, tétrazolyle, imidazolyle, 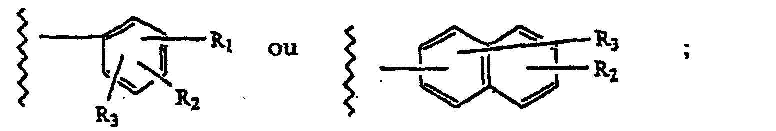
et n vaut de 0 à 2 ;
ou sel d'un tel composé, admissible en pharmacie.

2. Composé conforme à la revendication 1, dans lequel m vaut 1 et y vaut 1.

3. Composé conforme à la revendication 1, dans lequel Z représente

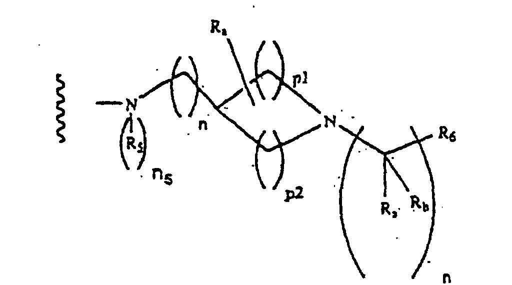

4. Composé conforme à la revendication 1, dans lequel Z représente

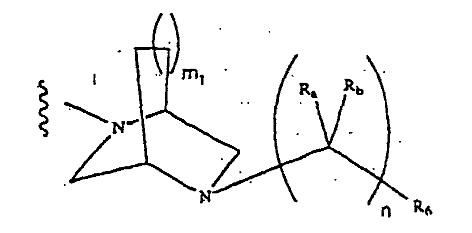

5. Composé conforme à la revendication 1, dans lequel Z représente

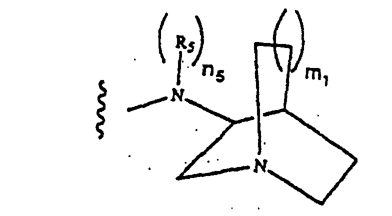

6. Composé conforme à la revendication 1, de formule

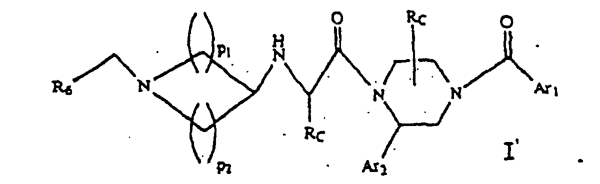

7. Composé conforme à la revendication 6, dans lequel R_{c} représente H, p₁ et p₂ valent 2 et Ar₁ et Ar₂ représentent tous les deux

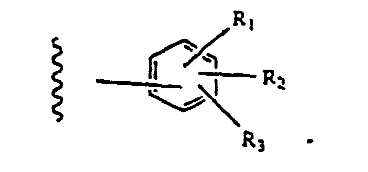

8. Composé conforme à la revendication 6, dans lequel R₆ représente un groupe thiényle, furanyle, pyridyle, tétrazolyle, imidazolyle,

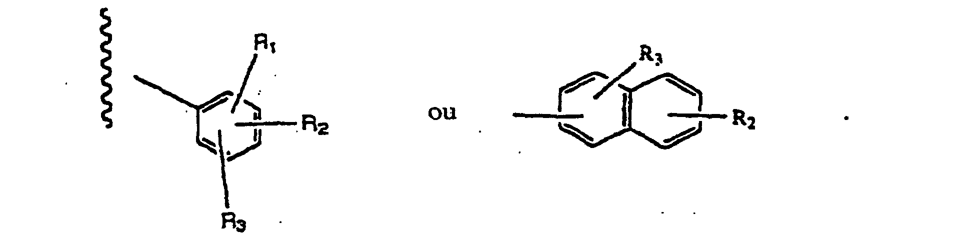

9. Composé conforme à la revendication 4, dans lequel R₆ représente

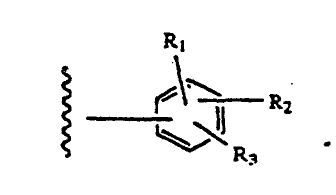

10. Composé conforme à la revendication 1, choisi dans l'ensemble que constituent 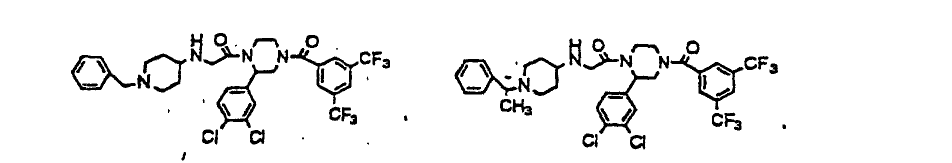 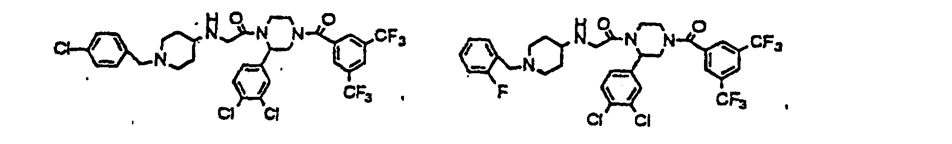 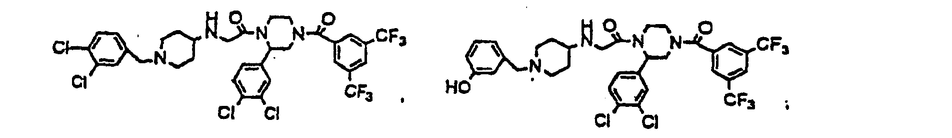 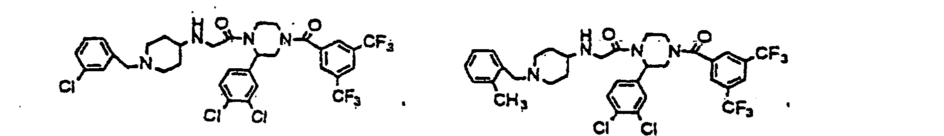 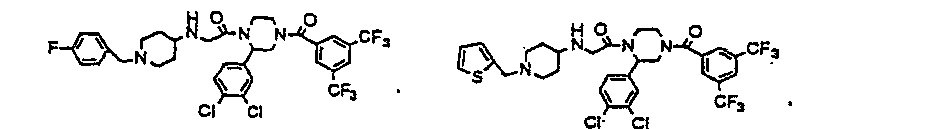 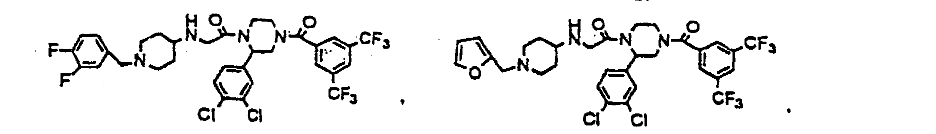 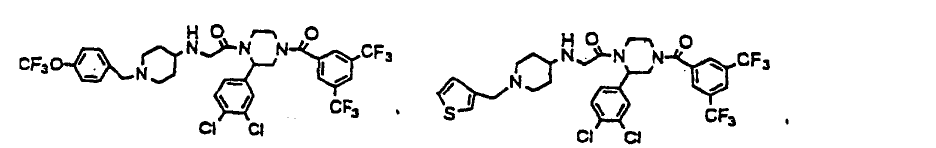 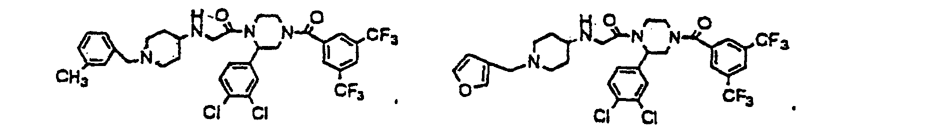 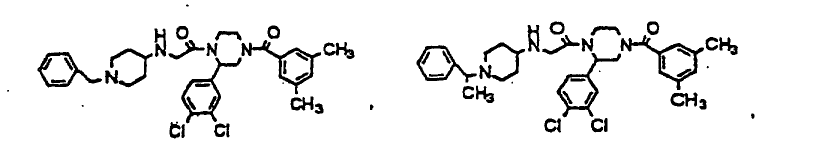 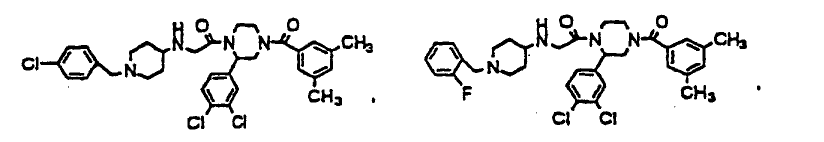 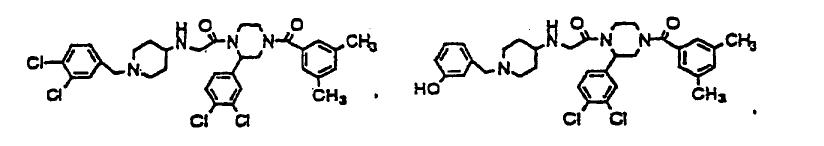 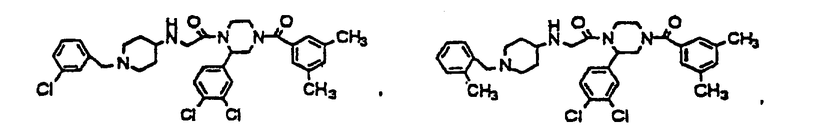 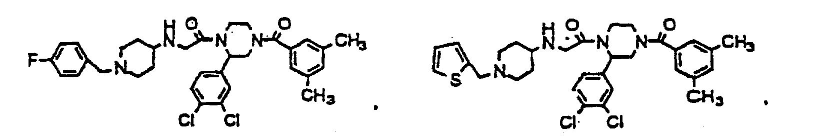 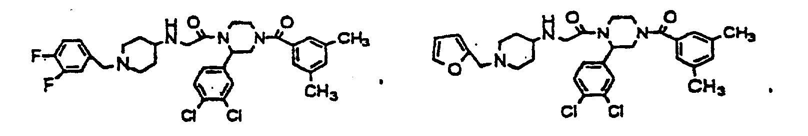 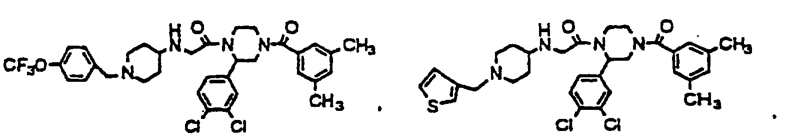 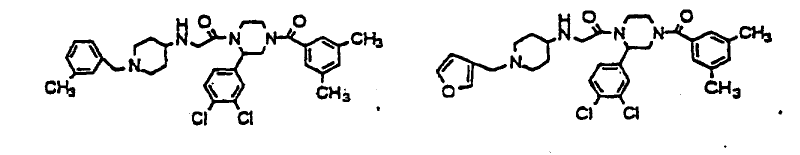 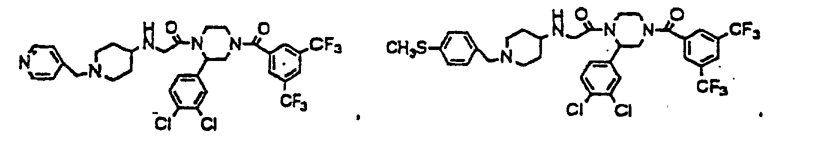 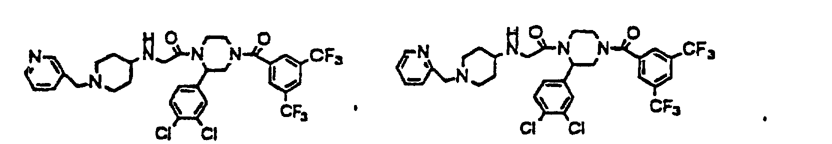 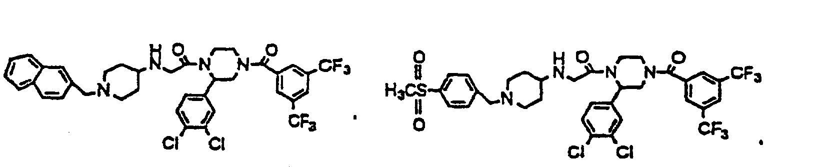 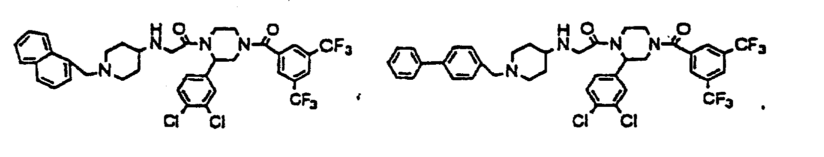 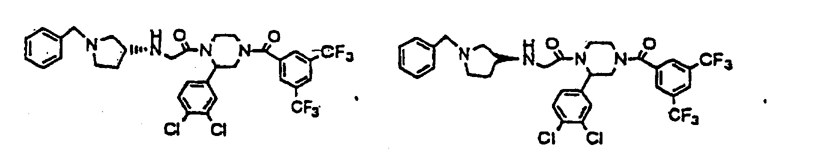 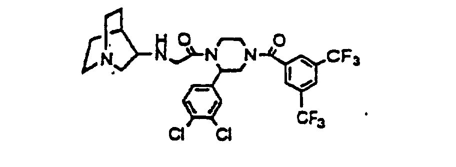 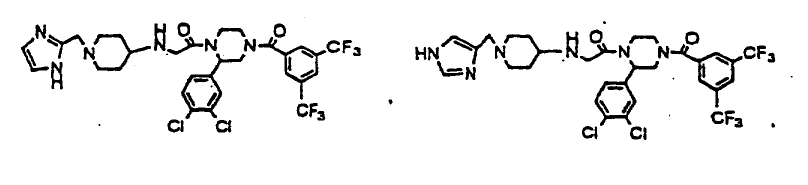 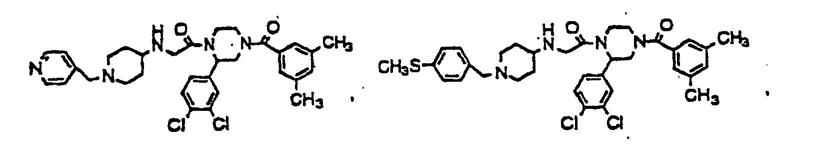 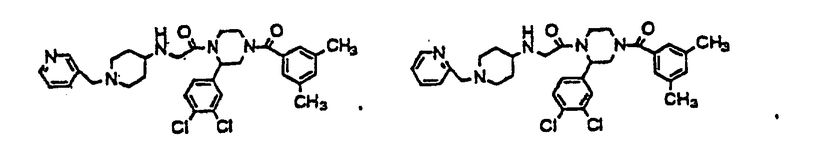 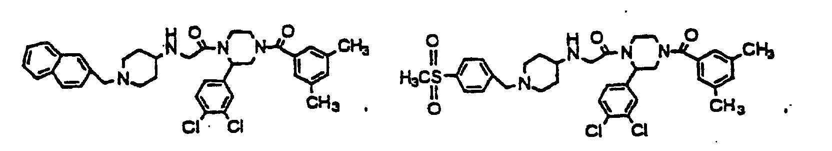 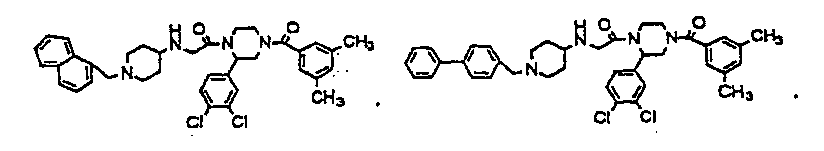 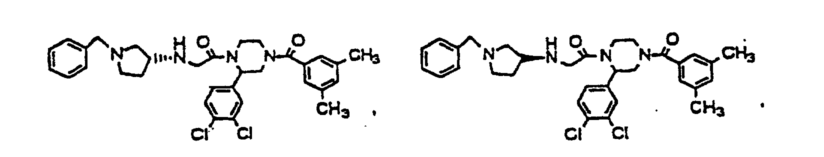 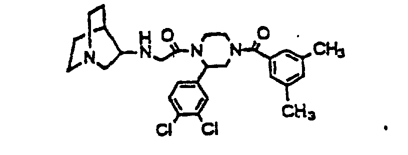 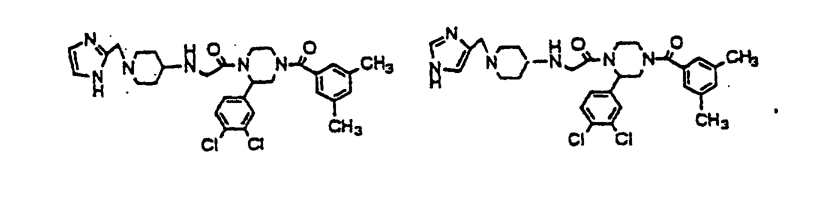 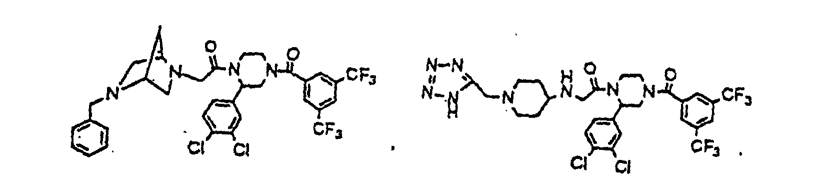 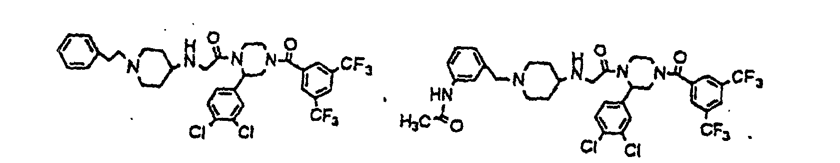 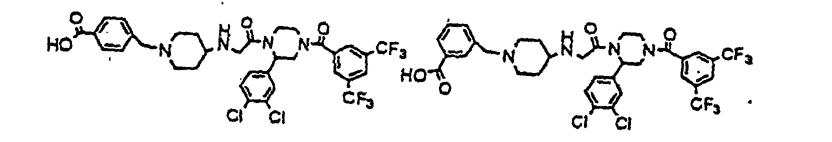 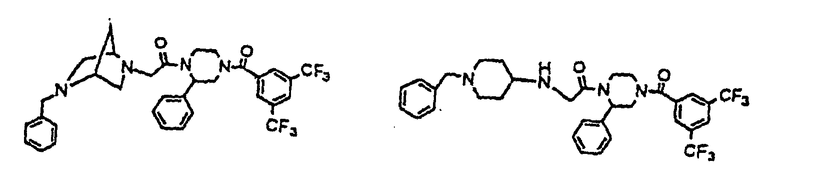 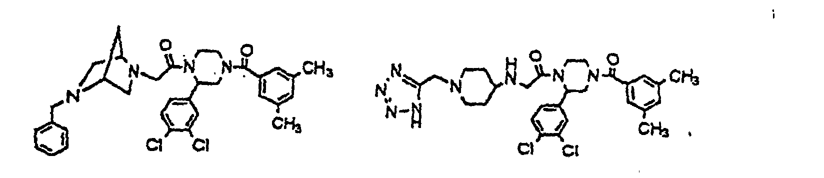 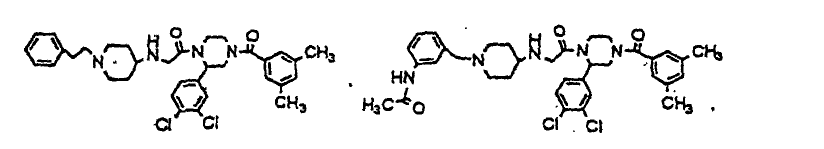 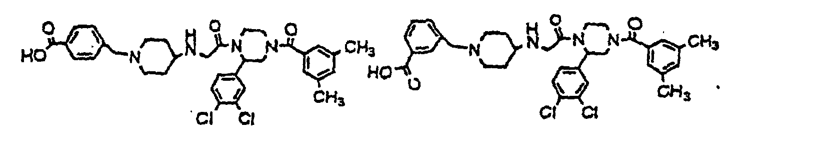 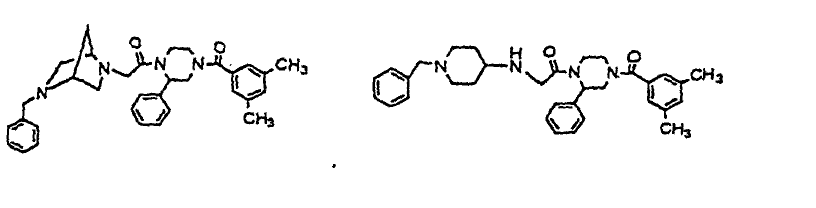 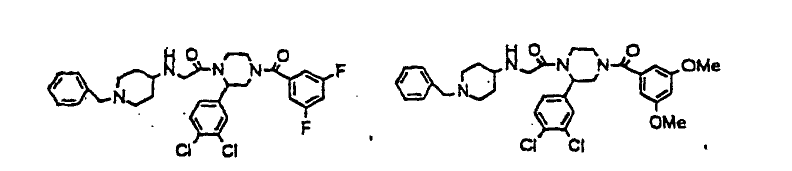 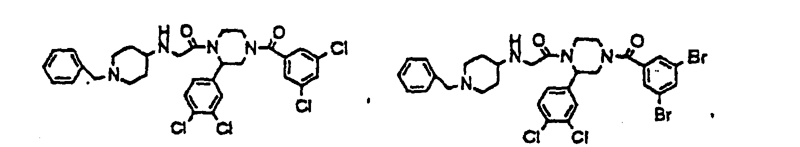 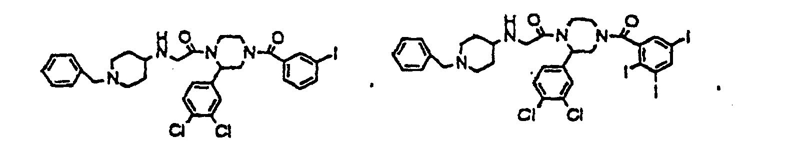 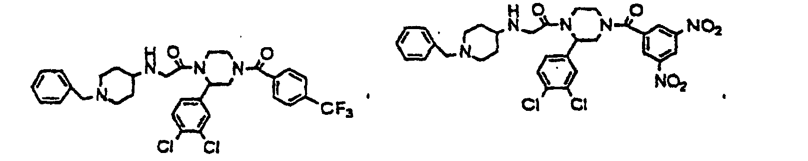 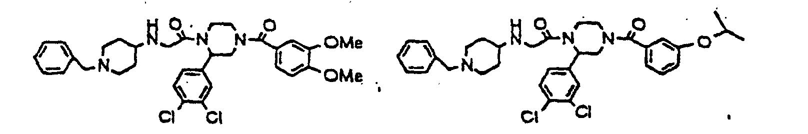 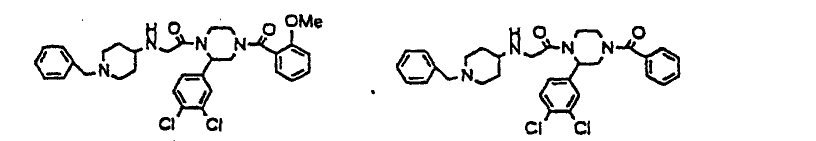 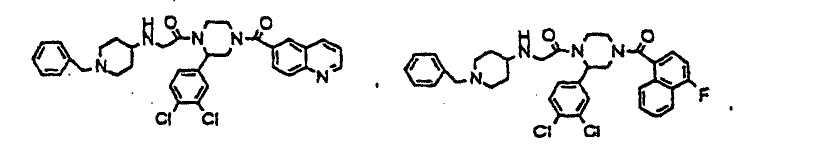 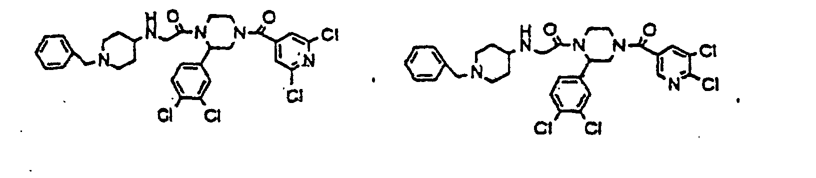 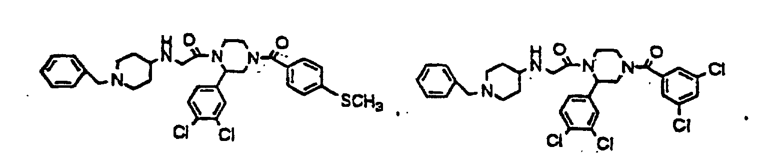 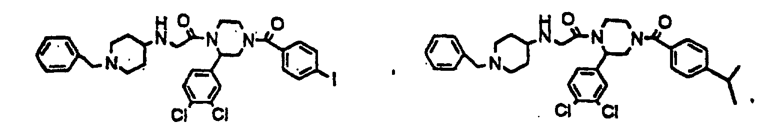 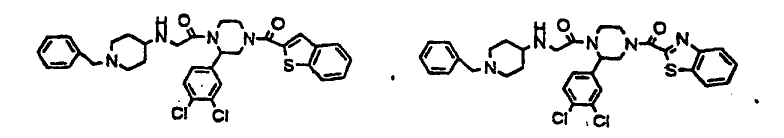 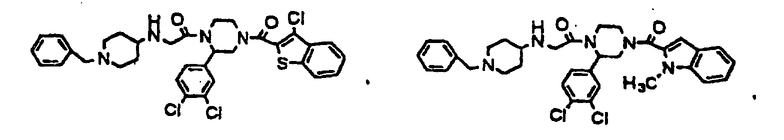 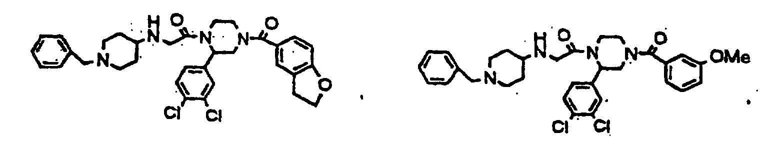 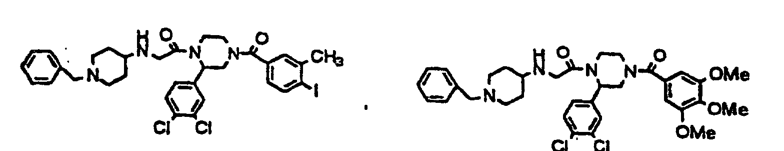 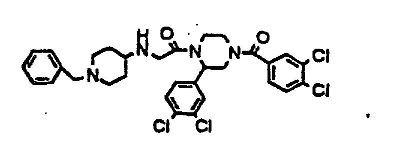 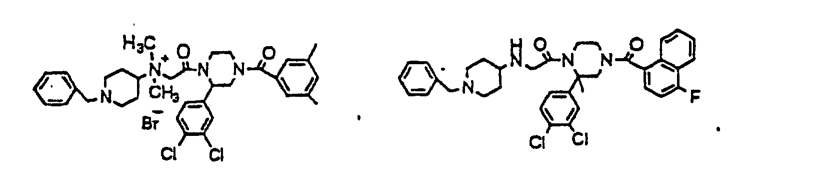 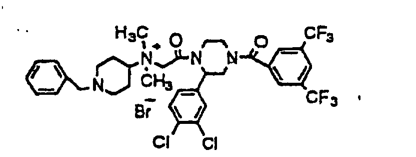 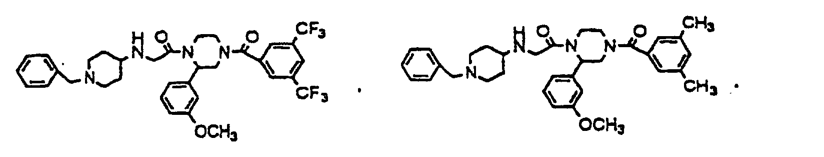 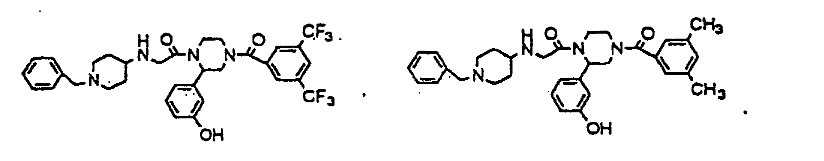 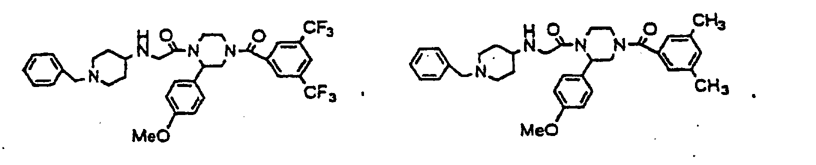 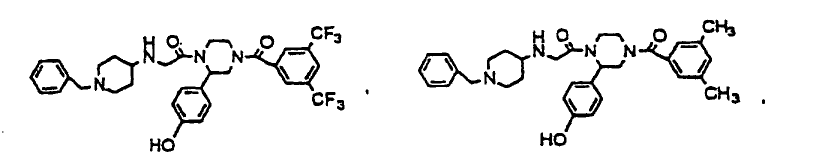 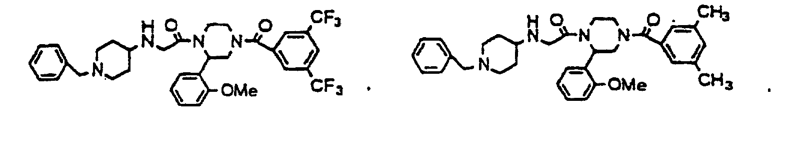 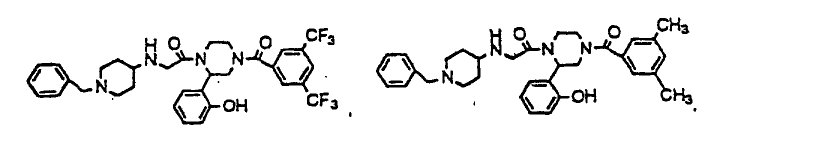 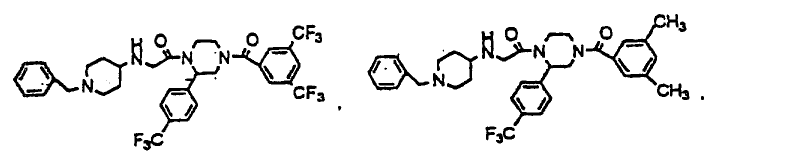 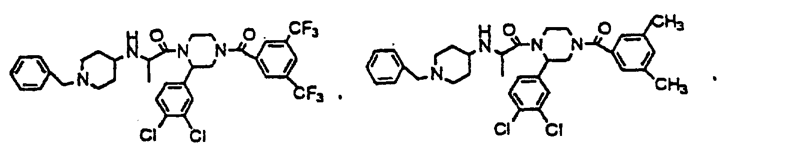 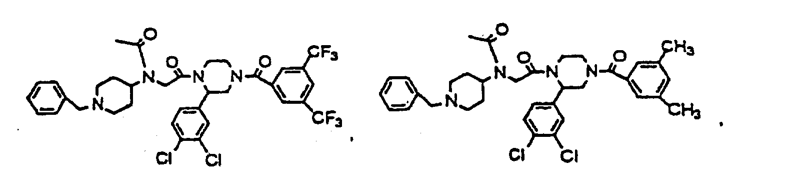 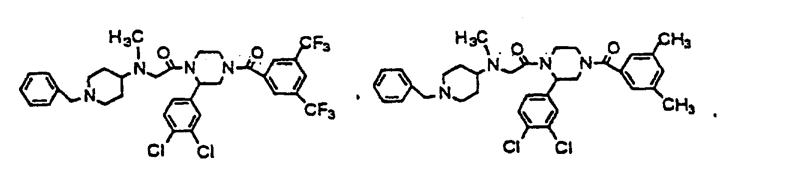 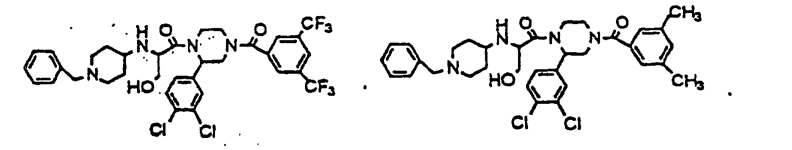 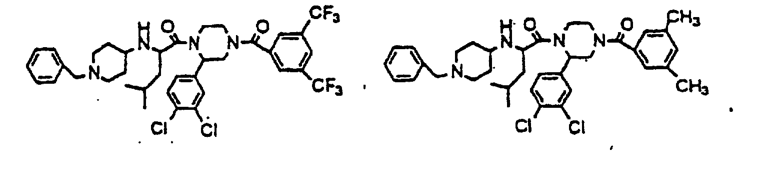 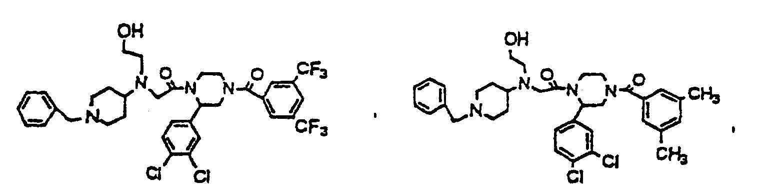 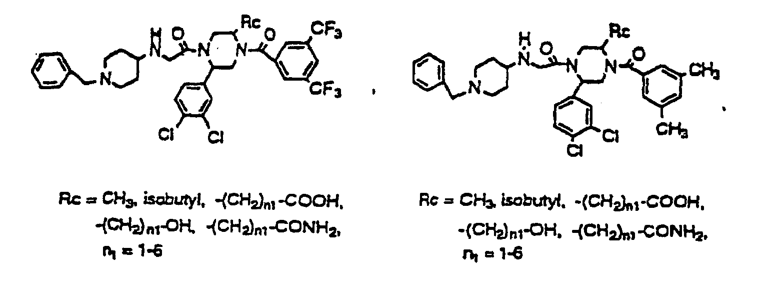 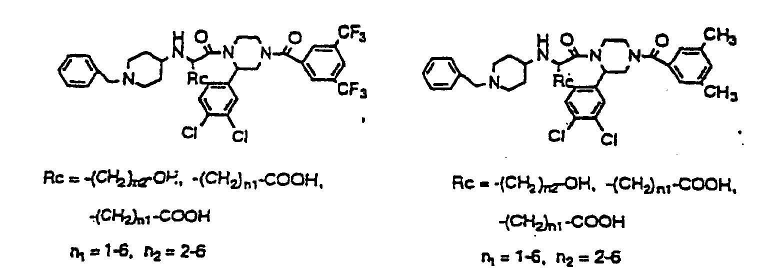 ou sel d'un tel composé, admissible en pharmacie.

11. Composition contenant un composé conforme à la revendication 1, en une quantité suffisante pour qu'il agisse efficacement comme antagoniste de neurokinine, ainsi qu'un véhicule admissible en pharmacie.

12. Emploi d'un composé conforme à la revendication 1 dans la fabrication d'un médicament destiné à induire un antagonisme vis-à-vis d'une neurokinine.

13. Emploi conforme à la revendication 12, en vue de traiter des maladies chroniques des voies aériennes, des maladies inflammatoires, la migraine, des désordres du système nerveux central, le syndrome de Down, des neuropathies, la sclérose en plaques, des maladies ophtalmiques, des maladies auto-immunes, des maladies gastro-intestinales, des désordres des fonctions de la vessie, des maladies de la circulation, la maladie de Raynaud, la toux ou la douleur.

14. Emploi conforme à la revendication 13, en vue de traiter l'asthme, les allergies, les affections intestinales inflammatoires, le psoriasis, la fibrosite, l'arthrose, la polyarthrite rhumatoïde, la dépression, les psychoses, les démences, la maladie d'Alzheimer, la conjonctivite, les rejets de greffons, le lupus érythémateux aigu disséminé, la maladie de Crohn, la colite ulcéreuse ou l'angine.
